(19) Europäisches Patentamt / European Patent Office / Office européen des brevets

(11) **EP 4 310 079 B1**

(12) # EUROPEAN PATENT SPECIFICATION

(45) Date of publication and mention of the grant of the patent:
**17.06.2026 Bulletin 2026/25**

(21) Application number: **22770451.7**

(22) Date of filing: **14.03.2022**

(51) International Patent Classification (IPC):
***C07D 498/04*** *(2006.01)* ***C07D 519/00*** *(2006.01)*
***A61P 35/00*** *(2006.01)* ***A61K 31/4738*** *(2006.01)*

(52) Cooperative Patent Classification (CPC):
**C07D 498/04; A61P 35/00; C07D 519/00**

(86) International application number:
**PCT/CN2022/080680**

(87) International publication number:
**WO 2022/194096 (22.09.2022 Gazette 2022/38)**

(54) **ESTROGEN RECEPTOR ANTAGONISTS**

ÖSTROGENREZEPTOR-ANTAGONISTEN

ANTAGONISTES DU RÉCEPTEUR DES OESTROGÈNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2021 CN 202110277849**

(43) Date of publication of application:
**24.01.2024 Bulletin 2024/04**

(73) Proprietor: **Shenzhen Forward Pharmaceuticals Co., Limited**
**Shenzhen, Guangdong 518067 (CN)**

(72) Inventors:
- **YANG, Xuan**
  **Shenzhen, Guangdong 518067 (CN)**
- **ZHU, Chenggang**
  **Shenzhen, Guangdong 518067 (CN)**
- **ZHANG, Chaochun**
  **Shenzhen, Guangdong 518067 (CN)**
- **TALLEY, John Jeffery**
  **Shenzhen, Guangdong 518067 (CN)**
- **CHEN, Chaole**
  **Shenzhen, Guangdong 518067 (CN)**
- **BAO, Liming**
  **Shenzhen, Guangdong 518067 (CN)**
- **MIN, Xiangyan**
  **Shenzhen, Guangdong 518067 (CN)**
- **XU, Liangliang**
  **Shenzhen, Guangdong 518067 (CN)**

(74) Representative: **Synergy IP Group AG**
**Unterer Rheinweg 50**
**Postfach**
**4001 Basel (CH)**

(56) References cited:
**EP-A1- 3 312 184**
**WO-A1-2016/202161**
**WO-A1-2017/174757**
**WO-A1-2019/192533**
**WO-A1-2019/223715**
**CN-A- 109 219 604**
**US-A1- 2017 305 909**

- **SCOTT, J.S. ET AL.: "Selective estrogen receptor degraders (SERDs) and covalent antagonists (SERCAs): a patent review (July 2021-December 2023)", vol. 34, no. 5, 12 June 2024 (2024-06-12), pages 333 - 350, XP093203853, ISSN: 1354-3776, Retrieved from the Internet <URL:https://dx.doi.org/10.1080/13543776.2024.2364803> DOI: 10.1080/13543776.2024.2364803**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Technical Field

The present invention relates to some novel compounds or pharmaceutical salts thereof, which have anticancer activity and are thus potentially useful for methods for treating human or animal bodies. The present invention further relates to a method for manufacturing such compounds, pharmaceutical compositions containing the compounds, and the medical use thereof. For example, they are used for manufacturing medicaments for preventing or treating cancers in warm-blooded animals (such as humans), including estrogen receptor-dependent or -mediated diseases.

The present invention further relates to compounds as selective estrogen receptors down-regulation modulators.

### Background Art

Estrogen receptors (ERs) are steroid hormone receptors and include two subtypes, i.e., ER$\alpha$ and ER$\beta$. ERs are involved in the regulation and development of female reproductive tract. In cancers such as breast cancer, tumor growth is related to the functions of estrogen and ER receptors. For example, ER expression in most patients with breast cancer is increased.

It is known that some compounds can bind to ERs to competitively inhibit the binding of ERs to endogenous estrogen ligands, such as the female sex steroid hormone 17b estradiol (E2), and are thus used as ER inhibitors. For example, selective estrogen receptor modulators (SERMs) such as Tamoxifen, AZD9496 and AZD9833 are used as ER inhibitors to treat breast cancer, etc. WO 2017/182493 A1, WO 2017/174757 A1, EP 3 312 184 A1, WO 2019/192533 A1 and WO 2019/223715 A1 also disclose estrogen receptor modulators.

Although existing SERM therapies have certain effects on the treatment of breast cancer, there is still a need for ER inhibitors with stronger inhibitory activity and improved metabolic stability.

### Summary of the Invention

The inventors of the present invention have found that compounds as represented by Formula (I) have a basic structure of tetrahydrooxazolisoquinolinone, which has significant ER degradation activity and thus unexpectedly has significantly better ER inhibition activity than compounds known in the prior art; in addition, such compounds further have excellent metabolic stability. Therefore, the compound of the present invention can be used for treating an estrogen receptor-dependent or -mediated disease, thereby completing the present invention.

Specifically, the present invention relates to the following content.

In one aspect of the present invention, there is provided a compound of Formula (I) or a stereoisomer or pharmaceutically acceptable salt thereof:

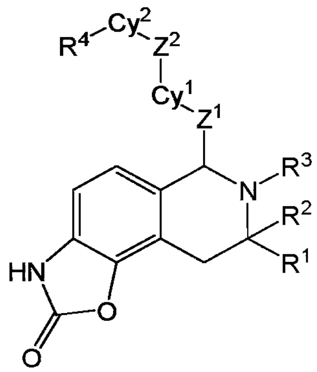

(I)

wherein:

- $Z^1$ is a bond;
- $Z^2$ is selected from $-O-CH_2-CH_2-$, $-O-CH_2-$, $-NH-CH_2-CH_2-$, $-NH-CH_2-$, -NH-, or -O-;
- $Cy^1$ is phenyl or pyridinyl, each optionally substituted with a halogen atom or $C_{1-6}$alkoxy;
- $Cy^2$ is selected from a bond, azetidinylidene, pyrrolidinylidene, piperazinylidene,

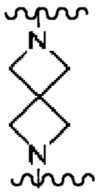 , and 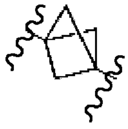 ;

$R^1$ is $C_{1-6}$alkyl;
$R^2$ is H;
$R^3$ is $-CH_2-CR^{31}R^{32}R^{33}$;
$R^{31}$, $R^{32}$ and $R^{33}$ are independently selected from H, a halogen atom, cyano, and $C_{1-6}$alkyl optionally substituted with hydroxyl or a halogen atom, and $R^{31}$ and $R^{32}$ can jointly form $C_{3-8}$cycloalkylene optionally substituted with hydroxyl or a halogen atom;
$R^4$ is $C_{1-6}$alkyl, $C_{1-6}$alkylamino, amino$C_{1-6}$alkyl, $C_{2-6}$alkenyl, or $C_{2-6}$alkenylamino, optionally substituted with a group selected from a halogen atom, oxo, $C_{1-6}$alkylamino, $(C_{1-6}alkyl)_2$amino, and

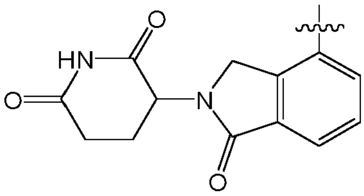
.

Preferably, $Cy^2$ is selected from a bond, azetidinylidene, and pyrrolidinylidene, and $R^4$ is $C_{1-6}$alkyl, $C_{1-6}$alkylamino, $C_{2-6}$alkenyl, or $C_{2-6}$alkenylamino, optionally substituted with a group selected from a halogen atom, oxo, $C_{1-6}$alkylamino, and $(C_{1-6}alkyl)_2$amino.

In a particularly preferred embodiment of the compound of Formula (I):

$Z^1$ is a bond;
$Z^2$ is selected from -NH- or -O-;
$Cy^1$ is phenyl or pyridinyl, optionally substituted with F or methoxy;
$Cy^2$ is selected from azetidinylidene, pyrrolidinylidene, and piperazinylidene;
$R^1$ is methyl;
$R^2$ is H;
$R^3$ is $-CH_2-CR^{31}R^{32}R^{33}$;
$R^{31}$ and $R^{32}$ are each F, or $R^{31}$ and $R^{32}$ jointly form cyclopropylidene;
$R^{33}$ is selected from F, hydroxymethyl, and fluoromethyl;
$R^4$ is $F-(CH_2)_3-$ or

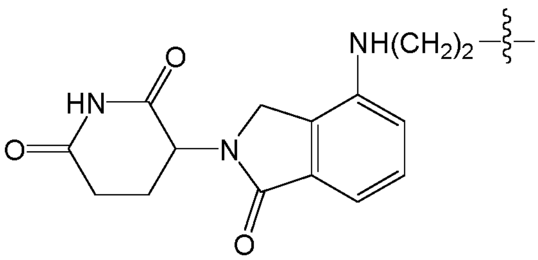
.

Further preferably, $Cy^2$ is selected from azetidinylidene and pyrrolidinylidene, and $R^4$ is $F-(CH_2)_3-$.

In some embodiments, the present invention relates to the following compounds or stereoisomers or pharmaceutically acceptable salts thereof:

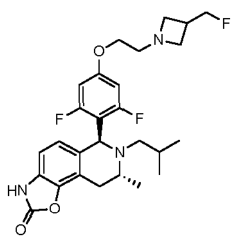 1, 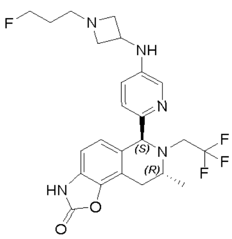 5, 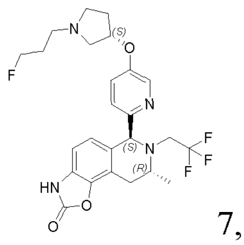 7, 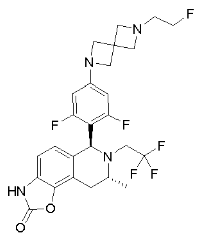 8,

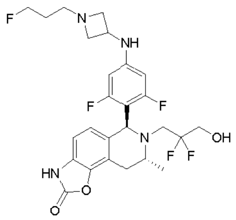 9, 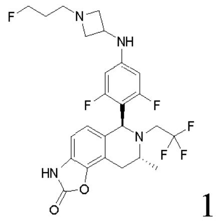 10, 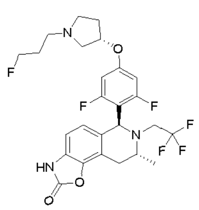 11, 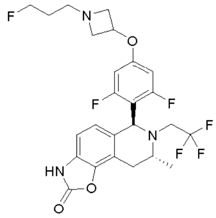 12,

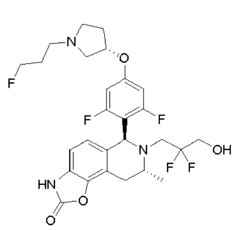 13, 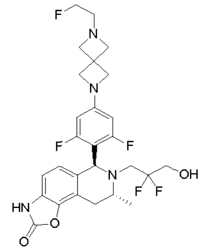 14, 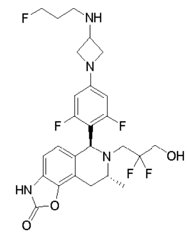 15, 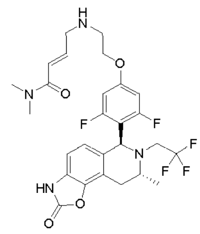 16,

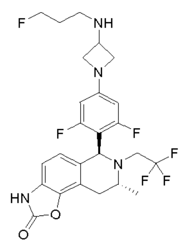 18, 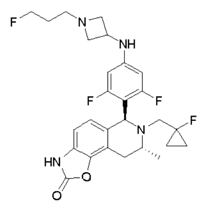 20, 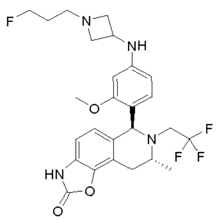 25, 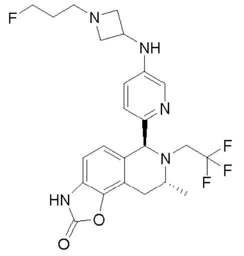 26,

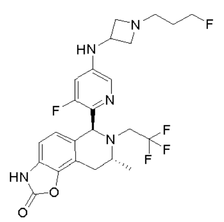 27, 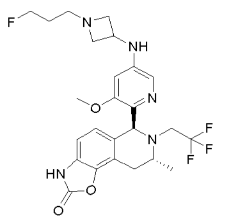 28, 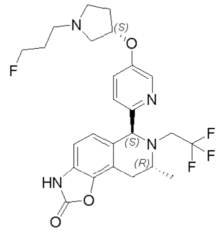 29, 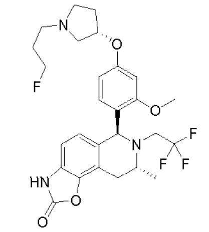 30,

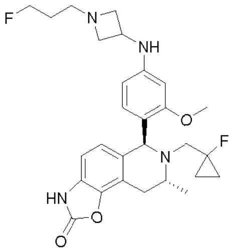 31, 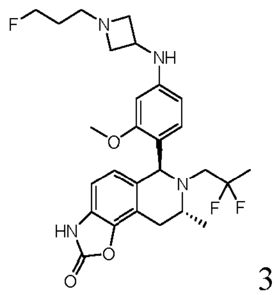 32, 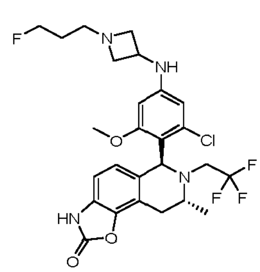 33, 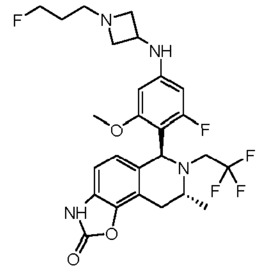 34,

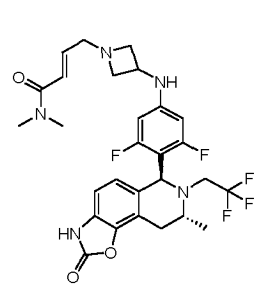 35, 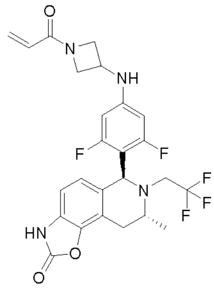 36, 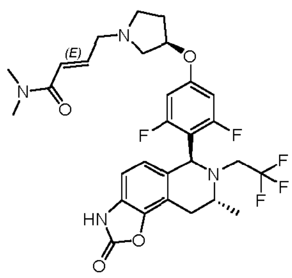 37, 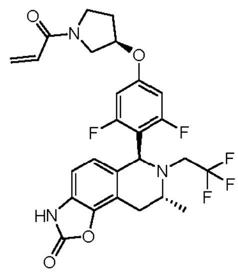 38,

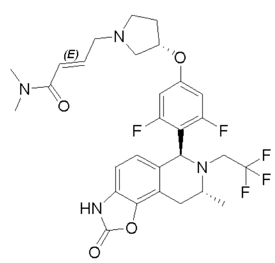 39, 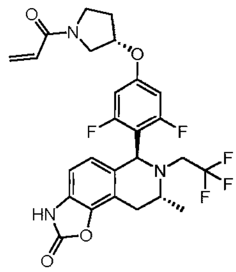 40, 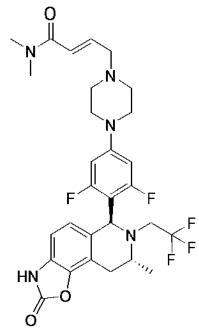 41, 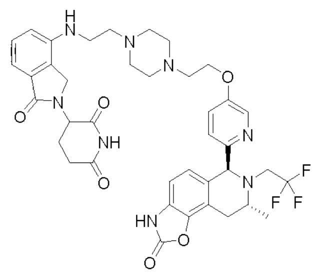 42

and

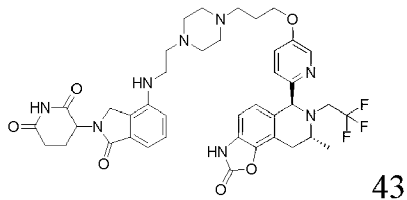 43.

In another aspect, the present invention relates to a pharmaceutical composition comprising the compound of Formula (I) or stereoisomer or pharmaceutically acceptable salts thereof, and a pharmaceutically acceptable carrier.

In another aspect, the present invention relates to a compound of Formula (I) or a stereoisomer or pharmaceutically acceptable salt thereof for use in treating an estrogen receptor-dependent or -mediated disease in a mammal.

**Detailed Description of Embodiments**

The present invention will be further illustrated by specific preparation examples and test examples. It should be recognized that the examples and test examples are not intended to limit the scope of the present invention. The raw materials, reagents, etc. used in the examples are all well known to those skilled in the art and can be commercially available substances or obtained by literature methods; the test or characterization methods used are also well known to those skilled in the art.

**Example 1: (6S,8R)-6-(2,6-difluorophenyl-4-(2-(3-(fluoromethyl)-azetidin-1-yl-ethoxy-phenyl)-7-isobutyl-8-methyl-6,7,8,9-tetrahydrooxazolo[5,4-f]isoquinolin-2(3H)-one (Compound 1)**

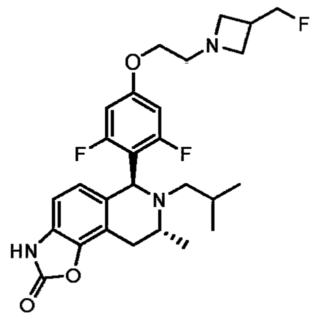

Step I: Synthesis of 7-bromobenzo[d]oxazol-2(3H)-one

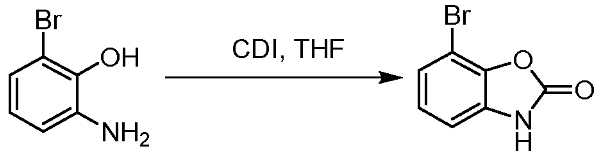


2-Amino-6-bromophenol (5 g, 26.6 mmol, 1 eq) was dissolved in 120 ml of tetrahydrofuran, dicarbonyl imidazole (5.17 g, 31.9 mmol, 1.2 eq) was added at 20°C, and the reaction solution was reacted at 80°C for 2 h. TLC detected the completion of the reaction. The reaction solution was poured into ice water, adjusted to pH = 2 with hydrochloric acid, extracted with ethyl acetate, dried and concentrated, and the crude product was separated by column chromatography to obtain 7-bromobenzo[d]oxazol-2(3H)-one as a red solid (5.1 g, 23.8 mmol, 89.6% yield).

**$^1$H NMR** (400 MHz, DMSO-d6): $\delta$ = 12.0 (s, 1H), 7.30 - 7.27 (m, 1H), 7.11 - 7.09 (m, 2H) ppm.

Step II: Synthesis of 7-bromo-3-triphenylmethylbenzo[d]oxazol-2(3H)-one

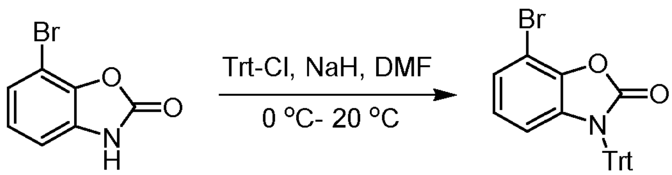


7-Bromobenzo[d]oxazol-2(3H)-one (5.1 g, 23.8 mmol, 1 eq) was dissolved in 50 mL N,N-dimethylformamide, sodium hydride (1.43 g, 35.7 mmol, 60% purity, 1.5 eq) was added at 0°C, the reaction solution was stirred at 0°C for 30 min, triphenylmethyl chloride (6.64 g, 23.8 mmol, 1 eq) was then added, and the reaction solution was reacted at 20°C for 2 h. TLC detected the completion of the reaction. The reaction solution was poured into ice water, extracted with ethyl acetate, dried and concentrated, and the crude product was separated by column chromatography to obtain 7-bromo-3-triphenylmethylbenzo[d]oxazol-2(3H)-one as a white solid (9.5 g, 20.8 mmol, 87.4% yield).

**$^1$H NMR** (400 MHz, $CDCl_3$): $\delta$ = 7.40 - 7.37 (m, 6H), 7.25 - 7.20 (m, 9H), 7.12 (d, *J*= 8.0 Hz, 1H), 6.62 (t, *J*=8.4 Hz, 1H), 5.93 (d, *J*= 8.0 Hz, 1H) ppm.

Step III: Synthesis of tert-butyl (R)-(1-(2-oxo-3-triphenylmethyl-2,3-dihydrobenzo[d]oxazol-7-yl)prop-2-yl)carbamate

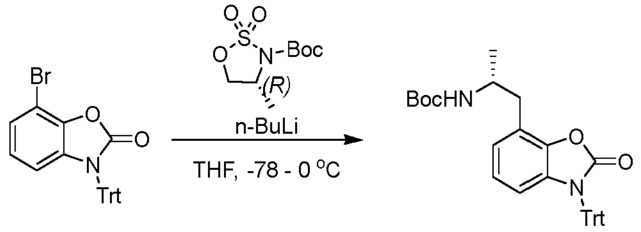


7-Bromo-3-triphenylmethylbenzo[d]oxazol-2(3H)-one (4.0 g, 8.77 mmol, 1 eq) was dissolved in tetrahydrofuran (20 mL) was added to n-butyl lithium (2.5 M, 4.03 mL, 1.15 eq) under nitrogen protection at -78°C and stirred for 30 min. A solution of (R)-tert-butyl 4-methyl-1,2,3-oxazolidine-3-carboxylic acid 2,2-dioxide (2.50 g, 10.5 mmol, 1.2 eq) in tetrahydrofuran (10 mL) was added to the system at -78°C and then naturally recovered to 0°C for 2 h. TLC detected the completion of the reaction. The reaction solution was poured into ice water and quenched, extracted with ethyl acetate, dried and concentrated. The crude product was separated by column chromatography to obtain tert-butyl (R)-(1-(2-oxo-3-triphenylmethyl-2,3-dihydrobenzo[d]oxazol-7-yl)prop-2-yl)carbamate as a white solid (4.5 g, 8.42 mmol, 76.8% yield).

**$^1$H NMR** (400 MHz, $CDCl_3$): δ = 7.50 - 7.48 (m, 6H), 7.33 - 7.28 (m, 9H), 6.88 -6.78 (m, 1H), 6.76 - 6.74 (m, 1H), 5.92 (br d, *J*=8.0 Hz, 1H), 4.22 - 4.11 (m, 1H), 2.84 (br d, *J*=6.4 Hz, 2H), 1.58 (s, 9H), 1.16 (d, *J*=6.4 Hz, 3H) ppm.

Step IV: Synthesis of (R)-7-(2-aminopropyl)-3-triphenylmethylbenzo[d]oxazol-2(3*H*)-one

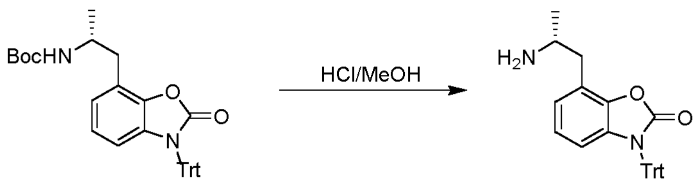


At 20°C, tert-butyl (R)-(1-(2-oxo-3-triphenylmethyl-2,3-dihydrobenzo[d]oxazol-7-yl)prop-2-yl)carbamate (4.3 g, 8.04 mmol, 1 eq) was added to a methanolic hydrochloride solution (4 M, 30 mL, 14.9 eq). After 2 hours of reaction, TLC and LCMS detected the completion of the reaction. The reaction solution was concentrated, neutralized by adding saturated sodium bicarbonate, extracted with ethyl acetate and concentrated to obtain a crude product, which was slurried with 30 ml of a mixed solvent of petroleum ether : ethyl acetate = 3 : 1 and filtered to obtain (R)-7-(2-aminopropyl)-3-triphenylmethylbenzo[d]oxazol-2(3*H*)-one as a white solid (1.9 g, 4.37 mmol, 54.4% yield).

**$^1$H NMR** (400 MHz, DMSO-d6): δ = 7.50 - 7.48 (m, 6H), 7.35 - 7.25 (m, 9H), 6.92 - 6.87 (m, 1H), 6.86 - 6.78 (m, 1H), 5.92 (d, *J*=7.2 Hz, 1H), 3.11 - 3.06 (m, 1H), 2.60 - 2.57 (m, 2H), 0.97 (d, *J*=6.4 Hz, 3H) ppm.

**LCMS:** m/z $(M+H)^+$ = 435.1.

Step V: Synthesis of (R)-7-(2-(isobutylamine)propyl)-3-triphenylmethylbenzo[d]oxazol-2(3*H*)-one

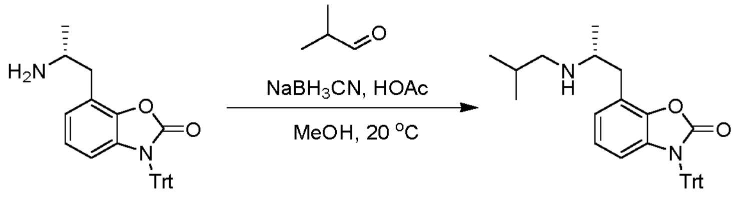


(R)-7-(2-aminopropyl)-3-triphenylmethylbenzo[d]oxazol-2(3*H*)-one (1.65 g, 3.38 mmol, 1 eq) and 2-methylpropionaldehyde (292 mg, 4.06 mmol, 370 uL, 1.2 eq) were dissolved in 30 ml of methanol, and acetic acid (406 mg, 6.76 mmol, 387 uL, 2 eq) and sodium cyanoborohydride (425 mg, 6.76 mmol, 2 eq) were added to the reaction solution at 20°C. After 2 hours of reaction, TLC and LCMS monitored the completion of the reaction. The reaction solution was concentrated, then neutralized by adding saturated sodium bicarbonate, extracted with ethyl acetate, dried, concentrated and separated by column chromatography to obtain (R)-7-(2-(isobutylamine)propyl)-3-triphenylmethylbenzo[d]oxazol-2(3*H*)-one as a yellow solid (1.70 g, 100% yield).

**$^1$H NMR** (400 MHz, $CDCl_3$): δ = 7.46 - 7.48 (m, 6H), 7.30 - 7.28 (m, 9H), 6.93 (d, *J*=7.2 Hz, 1H), 6.82 (t, *J*=8.0 Hz, 1H), 5.99 (d, *J*= 8.0 Hz, 1H), 4.13 (q, *J*=7.2 Hz, 1H), 3.57 - 3.53 (m, 1H), 3.26 - 3.21 (m, 1H), 2.99 - 2.94 (m, 1H), 2.79 - 2.73 (m, 2H), 1.31 - 1.29 (m, 3H), 0.97 - 0.94 (m, 6H) ppm.
**LCMS:** m/z $(M+H)^+$ = 491.4.

Step VI: Synthesis of (R)-7-(2-(isobutylamine)propyl)benzo[d]oxazol-2(3*H*)-one

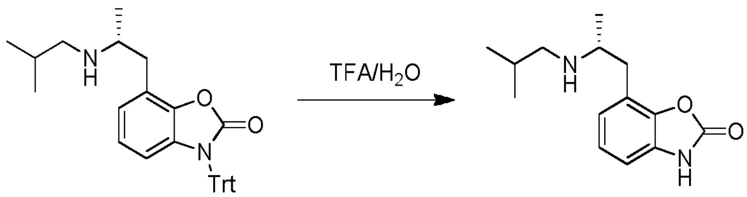


(R)-7-(2-(isobutylamine)propyl)-3-triphenylmethylbenzo[d]oxazol-2(3*H*)-one (1.7 g, 3.46 mmol, 1 eq) was dissolved in a mixed solvent of 20 ml of trifluoroacetic acid and 2 ml of water and reacted at 20°C for 3 h. TLC and LCMS monitored the completion of the reaction. The reaction solution was concentrated at 50°C, then adjusted to neutral pH by adding saturated sodium bicarbonate, and extracted with ethyl acetate, the organic phase was dried and concentrated, and the crude product was separated by column chromatography to obtain (R)-7-(2-(isobutylamine)propyl)benzo[d]oxazol-2(3*H*)-one as a yellow solid (650 mg, 2.62 mmol, 75.5% yield).
**$^1$H NMR** (400 MHz, $CDCl_3$): δ = 7.08 - 7.06 (m, 1H), 6.96 - 6.93 (m, 2H), 3.25 (br d, *J*=5.6 Hz, 1H), 3.04 - 2.99 (m, 1H), 2.83-2.80 (m, 1H), 2.61 - 2.58 (m, 2H), 1.84 - 1.77 (m, 1H), 1.18 (d, *J*=6.4 Hz, 3H), 0.90 (t, *J*=6.4 Hz, 6H) ppm.
**LCMS:**m/z $(M+H)^+$ = 249.2.

Step VII: Synthesis of (6S,8R)-6-(4-bromo-2,6-difluorophenyl)-7-isobutyl-8-methyl-6,7,8,9-tetrahydrooxazolo [5,4-f] isoquinolin-2(3*H*)-one

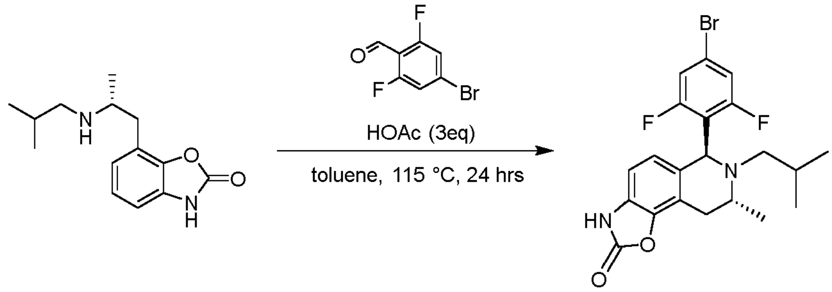


(R)-7-(2-(isobutylamine)propyl)benzo[d]oxazol-2(3*H*)-one (600 mg, 1 eq) and 4-bromo-2,6-difluorobenzaldehyde (800 mg, 1.5 eq) were dissolved in 30 ml of toluene, and acetic acid (450 mg, 3 eq) was added. The reaction solution was reacted at 115°C for 24 h. TLC and LCMS detected that 25% of raw material remained and 50% of products were produced. The reaction solution was concentrated at 60°C, and the crude product was separated by column chromatography to obtain (6S,8R)-6-(4-bromo-2,6-difluorophenyl)-7-isobutyl-8-methyl-6,7,8,9-tetrahydrooxazolo [5,4-f]isoquinolin-2(3*H*)-one as a yellow solid (460 mg, 42.2% yield).
**$^1$H NMR** (400 MHz, $CDCl_3$): δ = 7.59 (br s, 1H), 6.94 (br d, *J*=8.4 Hz, 2H), 6.67 (d, *J*=8.4 Hz, 1H), 6.49 (d, *J*=8.4 Hz, 1H), 5.04 (s, 1H), 3.45 - 3.39 (m, 1H), 3.09 - 3.04 (m, 1H), 2.77 - 2.72 (m, 1H), 2.38 - 2.34 (m, 1H), 1.89 - 1.84 (m, 1H), 1.60 - 1.58 (m, 1H), 0.91 (br d, *J*=6.4 Hz, 3H), 0.74 (br d, *J*=6.4 Hz, 3H), 0.60 (br d, *J*=6.4 Hz, 3H) ppm.
**LCMS:** m/z $(M+H)^+$ = 450.9

Step VIII: Synthesis of (6S,8R)-6-(4-bromo-2,6-difluorophenyl)-7-isobutyl-8-methyl-3-triphenylmethyl-6,7,8,9 -tetrahydrooxazolo[5,4-f]isoquinolin-2(3*H*)-one

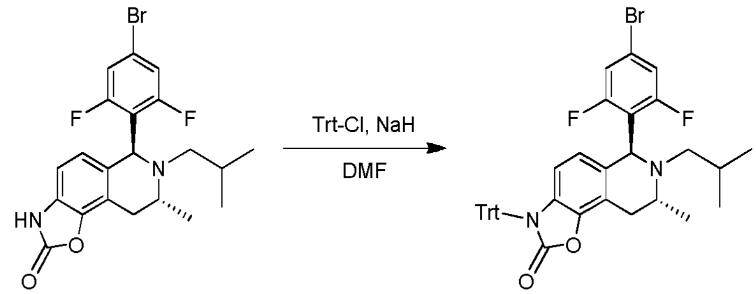


(6S,8R)-6-(4-bromo-2,6-difluorophenyl)-7-isobutyl-8-methyl-6,7,8,9-tetrahydroox azolo[5,4-f]isoquinolin-2(3*H*)-one (240 mg, 532 μmol, 1 eq) was dissolved in 10 ml of N,N-dimethylformamide, and sodium hydride (42.5 mg, 1.06 mmol, 60% purity, 2 eq) was added at 0°C. Triphenylmethyl chloride (163 mg, 585 μmol, 1.1 eq) was then added to the reaction solution at 0°C, and the reaction was slowly heated to 20°C for 2 hours of reaction. TLC and LCMS detected the completion of the reaction. The reaction solution was poured into water and quenched, and extracted with ethyl acetate, the organic phase was dried and concentrated and the crude product was separated by column chromatography to obtain (6S,8R)-6-(4-bromo-2,6-difluorophenyl)-7-isobutyl-8-methyl-3-triphenylmethyl-6,7,8,9 -tetrahydrooxazolo[5,4-f]isoquinolin-2(3*H*)-one as a yellow solid (420 mg, 460 μmol, 86.5% yield).

**$^1$H NMR**(400 MHz, $CDCl_3$): δ = 7.48 - 7.46 (m, 6H), 7.31 - 7.29 (m, 9H), 6.99 (d, *J*=8.4 Hz, 2H), 6.24 - 6.16 (m, 1H), 5.66 - 5.62 (m, 1H), 5.00 (s, 1H), 3.48 - 3.41 (m, 1H), 3.09 - 3.04 (m, 1H), 2.79 - 2.75 (m, 1H), 2.42 - 2.37 (m, 1H), 1.90 - 1.85 (m, 1H), 1.64 - 1.60 (m, 1H), 0.95 - 0.92 (m, 3H), 0.78 (d, *J*=6.4 Hz, 3H), 0.66 (d, *J*=6.4 Hz, 3H) ppm.

**LCMS**:m/z $(M+H)^+$ = 695.0.

Step IX: Synthesis of 2-(3-(fluoromethyl)azetidin-1-yl)ethanol

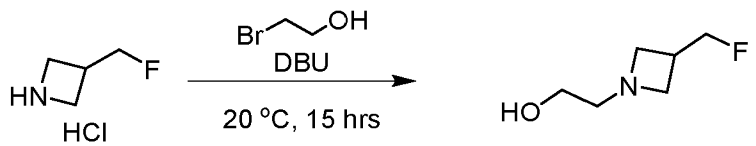


3-(Fluoromethyl)azetidine hydrochloride (3 g, 23.9 mmol, 1 eq) was dissolved in tetrahydrofuran (50 mL), and 1,8-diazacycloundecene (10.9 g, 71.7 mmol, 10.8 mL, 3 eq) was added at 20°C. After stirring for 15 minutes, 2-bromoethanol (5.97 g, 47.8 mmol, 2 eq) was added and reacted for 12 h. TLC detected the completion of the reaction. The reaction solution was adjusted to pH > 9 and extracted with ethyl acetate, the organic phase was dried and concentrated, and the crude product was separated by column chromatography to obtain 2-(3-(fluoromethyl)azetidin-1-yl)ethanol as a yellow oily product (950 mg, 7.13 mmol, 29.8% yield).

**$^1$H NMR** (400 MHz, $CDCl_3$): δ = 4.57 - 4.42 (m, 1H), 4.41 - 4.38 (m, 1H), 3.53 - 3.50 (m, 2H), 3.43 - 3.40 (m, 2H), 3.10 - 3.08 (m, 2H) 2.85 - 2.80 (m, 1H), 2.61 - 2.59 (m, 2H).

Step X: Synthesis of (6S,8R)-6-(2,6-difluorophenyl-4-(2-(3-(fluoromethyl)-azetidin-1-yl-ethoxy-phenyl)-7-is obutyl-8-methyl-3-triphenylmethyl-6,7,8,9-tetrahydrooxazolo[5,4-f]isoquinolin-2(3*H*)-one

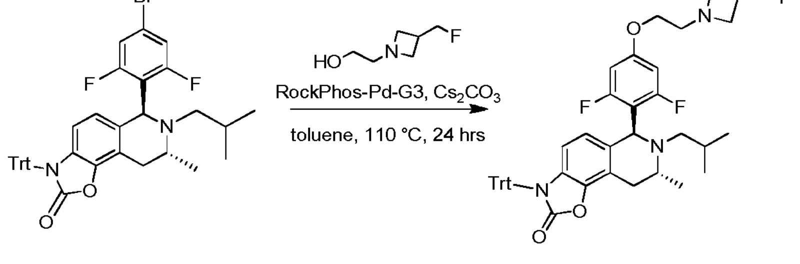


(6S,8R)-6-(4-bromo-2,6-difluorophenyl)-7-isobutyl-8-methyl-3-triphenylmethyl-6, 7,8,9-tetrahydrooxazolo[5,4-f]isoquinolin-2(3*H*)-one (300 mg, 329 μmol, 1 eq) and 2-(3-(fluoromethyl)azetidin-1-yl)ethanol (109 mg, 822 μmol, 2.5 eq) were dissolved in toluene (9 ml), [(2-2-tert-butylphosphine-3-methoxy-6-methyl-2,4,6-triisopropyl-1,1-biphenyl)-2-(2-a minobiphenyl)]palladium(II) methanesulfonate (27.6 mg, 32.8 μmol, 0.1 eq) and cesium carbonate (321 mg, 986 μmol, 3 eq) were added, and the reaction solution was reacted at 110°C for 24 h. TLC and LCMS monitored the completion of the reaction. The reaction solution was concentrated and then separated by column chromatography to obtain (6S,8R)-6-(2,6-difluorophenyl-4-(2-(3-(fluoromethyl)-azetidin-1-yl-ethoxy-phenyl)-7-is obutyl-8-methyl-3-triphenylmethyl-6,7,8,9-tetrahydrooxazolo[5,4-f]isoquinolin-2(3*H*)-one as a yellow solid (90 mg, 121 μmol, 36.7% yield).

**$^1$H NMR** (400 MHz, $CDCl_3$): δ = 7.47 - 7.46 (m, 6H), 7.26 - 7.23 (m, 9H), 6.34 (d, *J*=10.4 Hz, 2H), 6.23 (d, *J*=8.4 Hz, 1H), 5.61 (d, *J*=8.4 Hz, 1H), 4.95 - 4.93 (m, 1H), 4.57 (d, *J*=5.6 Hz, 1H), 4.45 (d, *J*=5.6 Hz, 1H), 3.91 (q, *J*=5.6 Hz, 2H), 3.50 - 3.42 (m, 5H), 3.17 - 3.16 (m, 2H), 3.06 - 3.04 (m, 1H), 2.84 - 2.77 (m, 2H), 2.76 - 2.72 (m, 1H), 2.37 - 2.32 (m, 1H), 1.92 (m, 1H), 0.95 - 0.92 (m, 3H), 0.78 (d, *J*=6.4 Hz, 3H), 0.69 - 0.65 (m, 3H) ppm.

**LCMS:** m/z $(M+H)^+$ = 742.2.

<u>Step XI: Synthesis of (6S,8R)-6-(2,6-difluorophenyl-4-(2-(3-(fluoromethyl)-azetidin-1-yl-ethoxy-phenyl)-7-is obutyl-8-methyl-6,7,8,9-tetrahydrooxazolo[5,4-f]isoquinolin-2(3*H*)-one (Compound 1)</u>

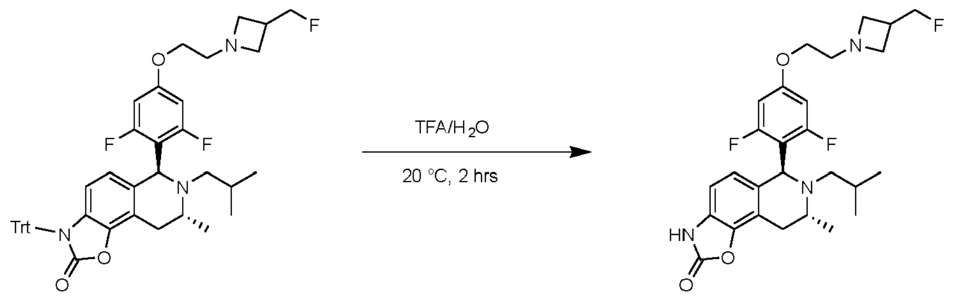


A mixed solution of (6S,8R)-6-(2,6-difluorophenyl-4-(2-(3-(fluoromethyl)-azetidin-1-yl-ethoxy-phenyl)-7-is obutyl-8-methyl-3-triphenylmethyl-6,7,8,9-tetrahydrooxazolo[5,4-f]isoquinolin-2(3*H*)-one (90 mg, 120.66 μmol, 1 eq) was dissolved in trifluoroacetic acid (9 mL) and water (1 mL) was reacted at 20°C for 2 h. LCMS and TLC monitored the completion of the reaction. The reaction solution was concentrated at 40°C, then neutralized by adding saturated sodium bicarbonate, and extracted with ethyl acetate, the organic phase was dried and concentrated, and the crude product was separated by thin layer chromatography on a silica gel plate to obtain (6S,8R)-6-(2,6-difluorophenyl-4-(2-(3-(fluoromethyl)-azetidin-1-yl-ethoxy-phenyl)-7-is obutyl-8-methyl-6,7,8,9-tetrahydrooxazolo[5,4-f]isoquinolin-2(3*H*)-one as a yellow solid (35 mg, 67.4 μmol, 55.8% yield, 97% purity, 89% ee).

**$^1$H NMR** (400 MHz, DMSO-d6): δ = 6.70 (d, *J*=8.0 Hz, 1H), 6.57 (d, *J*=8.0 Hz, 1H), 6.31 (br d, *J*=10.4 Hz, 2H), 5.06 (s, 1H), 4.56 (d, *J*=5.4 Hz, 1H), 4.44 (d, *J*=5.4 Hz, 1H), 3.93 - 3.92 (m, 2H), 3.56 - 3.54 (m, 2H), 3.45 - 3.42 (m, 1H), 3.20 - 3.10 (m, 3H), 2.89 - 2.86 (m, 2H), 2.82 - 2.77 (m, 1H), 2.40 - 2.32 (m, 1H), 2.01 - 1.96 (m, 1H), 1.26 (s, 2H), 0.99 (d, *J*=6.4 Hz, 3H), 0.81 (d, *J*=6.4 Hz, 3H), 0.68 (d, *J*=6.4 Hz, 3H) ppm.

**LCMS:m/z** $(M+H)^+$ = 504.4.

**Example 5: (6S,8R)-6-(5-((1-(3-fluoropropane)azetidin-3-yl)amino)pyridin-2-yl)-8-methyl-7-(2, 2,2-trifluoroethyl)-6,7,8,9-tetrahydrooxazolo[5,4-f]isoquinolin-2(3H)-one (Compound 5)**

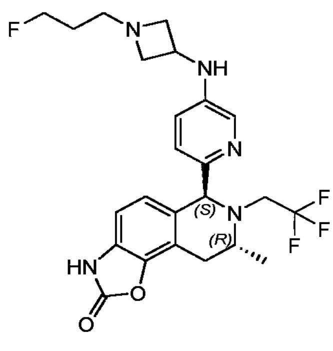

Step I: Synthesis of tert-butyl (R)-(1-(2-oxo-3-triphenylmethyl-2,3-dihydrobenzo[d]oxazol-7-yl)prop-2-yl)carbamate

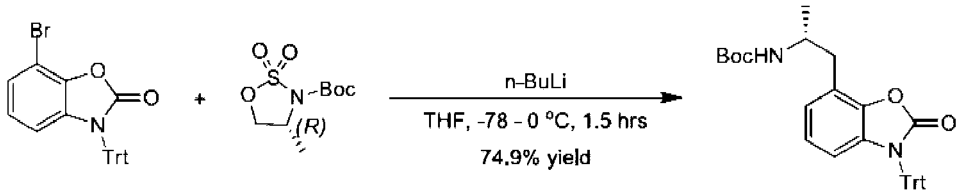


7-Bromo-3-triphenylmethyl-benzoxazol-2-one (100 g, 219 mmol, 1 eq) was dissolved in tetrahydrofuran (1000 mL), and n-butyl lithium (2.5 M, 87.6 mL, 1 eq) was added under nitrogen protection at -78°C and stirred for 30 min. A solution of (R)-tert-butyl 4-methyl-1,2,3-oxazolidine-3-carboxylic acid 2,2-dioxide (52.0 g, 219 mmol, 1 eq) in tetrahydrofuran (100 mL) was added to the system at -78°C and then naturally recovered to 0°C for 2 h. TLC detected the completion of the reaction. The reaction solution was poured into a saturated ammonium chloride aqueous solution and quenched, extracted with ethyl acetate, dried and concentrated. The crude product was separated by column chromatography to obtain tert-butyl (R)-(1-(2-oxo-3-triphenylmethyl-2,3-dihydrobenzo[d]oxazol-7-yl)prop-2-yl)carbamate as a yellow solid (360 g, 673 mmol, 74.9% yield).

**$^1$H NMR** (400MHz, $CDCl_3$): $\delta$ = 7.52 - 7.47 (m, 6H), 7.35 - 7.27 (m, 9H), 6.92 - 6.85 (m, 1H), 6.76 (t, *J* = 8.0 Hz, 1H), 5.93 (br d, *J* = 8.0 Hz, 1H), 4.00(s, 1H), 3.81 - 3.72 (m, 1H), 2.84 (br d, *J* = 6.0 Hz, 2H), 1.40 (s, 9H), 1.16 (d, *J* = 6.4 Hz, 3H) ppm.

Step II: Synthesis of (R)-7-(2-aminopropyl)-3-triphenylmethylbenzo[d]oxazol-2(3H)-one

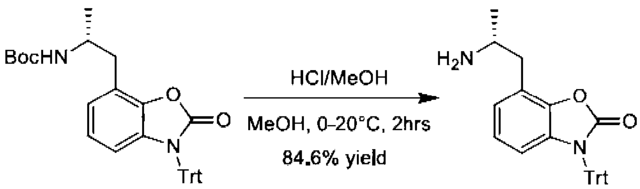


Tert-butyl (R)-(1-(2-oxo-3-triphenylmethyl-2,3-dihydrobenzo[d]oxazol-7-yl)prop-2-yl)carbamate (240 g, 448 mmol, 1 eq) was dissolved in methanol (400 ml) under nitrogen protection at 0°C, and the solution was then added to a methanolic hydrochloride solution (6 M, 800 mL, 10.6 eq). The reaction was carried out at 0°C for 2 h. TLC detected the completion of the reaction. The reaction solution was concentrated, neutralized by adding saturated sodium bicarbonate, extracted with ethyl acetate and concentrated to obtain a crude product, which was slurried with petroleum ether : ethyl acetate = 1 : 1 and filtered to obtain (R)-7-(2-aminopropyl)-3-triphenylmethylbenzo[d]oxazol-2(3*H*)-one as a yellow solid (198 g, 455 mmol, 84.6% yield).

**$^1$H NMR** (400MHz, $CDCl_3$): $\delta$ = 7.48 - 7.34 (m, 6H), 7.23 - 7.14 (m, 9H), 6.81 - 6.73 (m, 1H), 6.66 (t, *J* = 8.0 Hz, 1H), 5.88 (m, 1H), 3.33 (s, *J* = 6.4 Hz, 1H), 2.82 - 2.65 (m, 2H), 1.12 (d, *J* = 6.4 Hz, 3H) ppm.

Step III: Synthesis of (R)-7-2-aminopropyl)(2,2,2-trifluoroethyl-3-triphenylmethylbenzo[d]oxazol-2(3H)-one

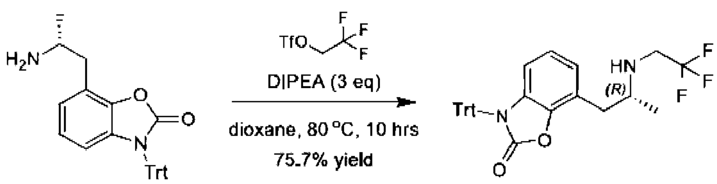


(R)-7-(2-aminopropyl)-3-triphenylmethylbenzo[d]oxazol-2(3H)-one (10.0 g, 23.0 mmol, 1 eq), 2,2,2-trifluoroethyl trifluoromethanesulfonate (6.14 g, 26.5 mmol, 1.15 eq), and diisopropyl ethylenediamine (8.92 g, 69.0 mmol, 12.0 mL, 3 eq) were dissolved in dioxane (100 mL) under nitrogen protection at 20°C and then reacted under nitrogen

protection at 80°C for 10 h. TLC detected the completion of the reaction. The reaction solution was concentrated, and the crude product was separated by column chromatography to obtain (R)-7-2-aminopropyl)(2,2,2-trifluoroethyl-3-triphenylmethylbenzo[d]oxazol-2(3H)-one as a white solid (9.00 g, 17.4 mmol, 75.7% yield).

**$^1$H NMR** (400MHz, $CDCl_3$): δ = 7.48 (br d, *J* = 7.2 Hz, 6H), 7.33 - 7.27 (m, 9H), 6.86 (br d, *J* = 7.6 Hz, 1H), 6.79 - 6.73 (m, 1H), 5.95 (br d, *J* = 8.0 Hz, 1H), 3.50 (s, 1H), 3.26 (br d, *J* = 9.3 Hz, 3H), 2.98 - 2.85 (m, 1H), 2.82 - 2.71 (m, 1H), 1.15 (br d, *J* = 5.6 Hz, 3H) ppm.

Step IV: Synthesis of (6S,8R)-6-(5-bromopyridin-2-yl)-8-methyl-7-(2,2,2-trifluoroethyl)-6,7,8,9-tetrahydrois oquinolino [5,4-floxazol-2(3H)-one

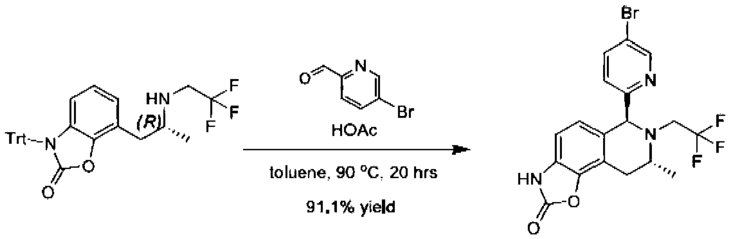


(R)-7-2-aminopropyl)(2,2,2-trifluoroethyl-3-triphenylmethylbenzo[d]oxazol-2(3H) -one (1.00 g, 1.94 mmol, 1 eq) and 5-bromo-2-pyridinecarboxaldehyde (540 mg, 2.90 mmol, 1.5 eq) was dissolved in toluene (50 mL), trifluoroacetic acid (662 mg, 5.81 mmol, 430 uL, 3 eq) was added thereto, and the mixture was reacted at 90°C for 20 h. TLC detected the completion of the reaction. The reaction solution was concentrated, the crude product was purified by TLC (petroleum ether : ethyl acetate = 10 : 1) to obtain (6S,8R)-6-(5-bromopyridin-2-yl)-8-methyl-7-(2,2,2-trifluoroethyl)-6,7,8,9-tetrahydrois oquinolino[5,4-f]oxazol-2(3H)-one as a yellow solid (780 mg, 1.76 mmol, 91.1% yield).

**$^1$H NMR** (400MHz, $CDCl_3$): δ = 8.89 (s, 1H), 8.54 (d, *J* = 2.4 Hz, 1H), 7.84 - 7.80 (m, 1H), 7.48 (d, *J* = 8.4 Hz, 1H), 6.79 - 6.74 (m, 2H), 5.09 (s, 1H), 4.13 (q, *J* = 7.2 Hz, 1H), 3.50 - 3.39 (m, 1H), 3.35 - 3.22 (m, 1H), 3.09 - 3.03 (m, 1H), 3.00 - 2.89 (m, 1H), 2.70 - 2.64 (m, 1H), 1.13 (d, *J* = 6.4 Hz, 3H) ppm.

Step V: Synthesis of (6S,8R)-6-(5-bromopyridin-2-yl)-8-methyl-7-(2,2,2-trifluoroethyl)-3-triphenylmethyl-6, 7,8,9-tetrahydrooxazolo[5,4-f]isoquinolin-2(3H)-one

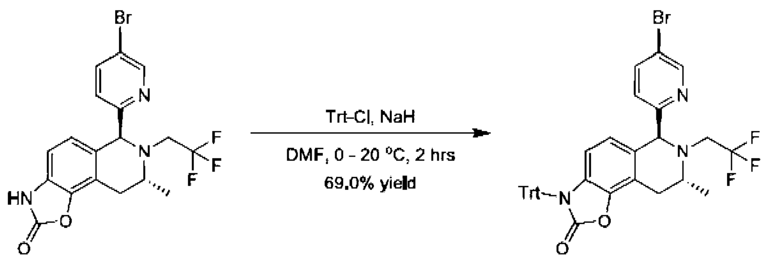


(6S,8R)-6-(5-bromopyridin-2-yl)-8-methyl-7-(2,2,2-trifluoroethyl)-6,7,8,9-tetrahy droisoquinoline[5,4-f]oxazol-2(3H)-one (1.00 g, 2.26 mmol, 1 eq) was dissolved in N,N-dimethylformamide (20 mL), sodium hydride (181 mg, 4.52 mmol, 60% purity, 2 eq) was added at 0°C, triphenylmethyl chloride (693 mg, 2.49 mmol, 1.1 eq) was then added at 20°C, and the mixture was reacted for 2 h. TLC detected the completion of the reaction. The reaction solution was quenched by adding water, extracted with ethyl acetate, dried and concentrated. The crude product was separated by column chromatography to obtain (6S,8R)-6-(5-bromopyridin-2-yl)-8-methyl-7-(2,2,2-trifluoroethyl)-3-triphenylmethyl-6, 7,8,9-tetrahydrooxazolo[5,4-f]isoquinolin-2(3H)-one as a white solid (1.20 g, 1.56 mmol, 69.0% yield).

**$^1$H NMR** (400MHz, $CDCl_3$): δ = 8.52 (s, 1H), 7.75 (dd, $J_1$ = 2.4 Hz, $J_2$ =8.4 Hz, 1H), 7.49 - 7.45 (m, 6H), 7.36 (d, *J* = 8.4 Hz, 1H), 7.32 - 7.27 (m, 9H), 6.39 (d, *J*= 8.4 Hz, 1H), 5.75 (d, *J* = 8.4 Hz, 1H), 4.94 (s, 1H), 3.53 - 3.42 (m, 1H), 3.29 - 3.18 (m, 1H), 3.01 (dd, $J_1$ = 4.8 Hz, $J_2$ =16.8 Hz, 1H), 2.90 (br dd, $J_1$ = 9.2 Hz, $J_2$ =15.6 Hz, 1H), 2.68 (dd, $J_1$ = 6.8 Hz, $J_2$ =16.8 Hz, 1H), 1.10 (d, *J* = 6.4 Hz, 3H) ppm.

Step VI: Synthesis of tert-butyl 3-((6-((6S,8R)-8-methyl-2-oxo-7-(2,2,2-trifluoroethyl)-3-triphenylmethyl-2,3,6,7,8,9-he xahydrooxazolo[5,4-f]isoquinolin-6-yl)pyridin-3-yl)amino)azetidine-1-carboxylate

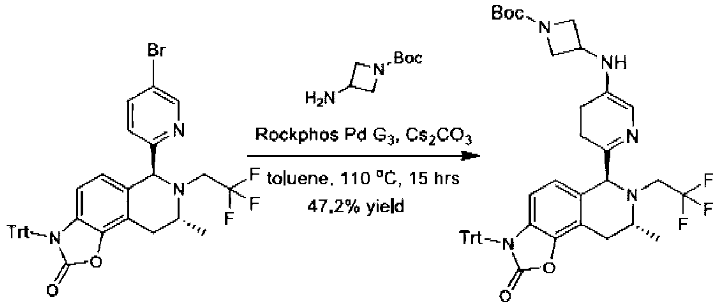


(6S,8R)-6-(5-bromopyridin-2-yl)-8-methyl-7-(2,2,2-trifluoroethyl)-3-triphenylmet hyl-6,7,8,9-tetrahydroisoquinolino[5,4-f]oxazol-2(3H)-one (200 mg, 292 μmol, 1 eq) and tert-butyl 3-aminoazetidine-1-carboxylate (126 mg, 730 μmol, 2.5 eq) were dissolved in 8 ml of toluene, [(2-2-tert-butylphosphine-3-methoxy-6-methyl-2,4,6-triisopropyl-1,1-biphenyl)-2-(2-a minobiphenyl)]palladium(II) methanesulfonate (24.5 mg, 29.2 μmol, 0.1 eq) and cesium carbonate (286 mg, 876 μmol, 3 eq) were added, and the reaction solution was reacted at 110°C for 15 h. TLC detected the completion of the reaction. The reaction solution was quenched by adding water, extracted with ethyl acetate, dried and concentrated. The crude product was separated by column chromatography to obtain tert-butyl 3-((6-((6S,8R)-8-methyl-2-oxo-7-(2,2,2-trifluoroethyl)-3-triphenylmethyl-2,3,6,7,8,9-he xahydrooxazolo[5,4-f]isoquinolin-6-yl)pyridin-3-yl)amino)azetidine-1-carboxylate as a yellow solid (120 mg, 137 μmol, 47.2% yield).

**$^1$H** NMR (400MHz, $CDCl_3$): δ = 7.77 (d, *J* = 2.4 Hz, 1H), 7.52 - 7.42 (m, 7H), 7.32 - 7.28 (m, 4H), 7.26 - 7.17 (m, 9H), 6.89 - 6.69 (m, 1H), 6.37 (d, *J* = 8.4 Hz, 1H), 5.80 - 5.64 (m, 1H), 5.39 - 5.20 (m, 1H), 4.98 - 4.77 (m, 1H), 4.33 - 4.27 (m, 2H), 4.23 - 4.11 (m, 1H), 4.08 - 3.98 (m, 1H), 3.74 (m, 2H), 3.53 - 3.43 (m, 2H), 3.26 - 3.11 (m, 1H), 3.04 - 2.85 (m, 2H), 2.72 - 2.59 (m, 1H), 1.45 (s, 9H), 1.09 (d, *J* = 6.4 Hz, 3H) ppm.

Step VII: Synthesis of (6S,8R)-6-(5-(azetidine-3-amino)pyridin-2-yl)-8-methyl-7-(2,2,2-trifluoroethyl)-6,7,8,9 -tetrahydrooxazolo[5,4-f]isoquinolin-2(3H)-one

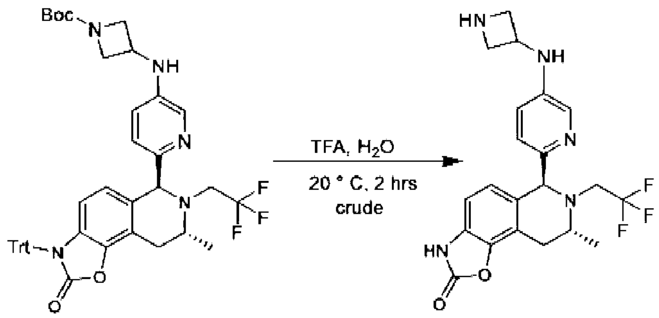


Trifluoroacetic acid (9.00 mL) and water (1.00 mL) were added to tert-butyl 3-((6-((6S,8R)-8-methyl-2-oxo-7-(2,2,2-trifluoroethyl)-3-triphenylmethyl-2,3,6,7,8,9-he xahydrooxazolo[5,4-f]isoquinolin-6-yl)pyridin-3-yl)amino) azetidine-1-carboxylate (120 mg, 154 μmol, 1 eq) at 0°C, and the mixture was reacted at 20°C for 2 h. TLC monitored the completion of the reaction. The reaction solution was quenched by adding water, extracted with ethyl acetate, dried and concentrated to obtain (6S,8R)-6-(5-(azetidine-3-amino)pyridin-2-yl)-8-methyl-7-(2,2,2-trifluoroethyl)-6,7,8,9 -tetrahydrooxazolo[5,4-f]isoquinolin-2(3H)-one as a yellow solid (120 mg, crude product).

**$^1$H NMR** (400MHz, $CDCl_3$): δ = 8.21 - 7.93 (m, 2H), 7.60 - 7.35 (m, 2H), 6.95 - 6.62 (m, 1H), 5.55 - 5.17 (m, 1H), 4.39 - 4.13 (m, 1H), 4.06 - 3.90 (m, 1H), 3.63 - 3.42 (m, 1H), 2.33 - 2.13 (m, 2H), 2.07 - 1.96 (m, 2H), 1.14 - 1.08 (m, 2H), 0.88 (br d, *J* = 4.8 Hz, 3H) ppm.

Step VIII: Synthesis of (6S,8R)-6-(5-((1-(3-fluoropropane)azetidin-3-yl)amino)pyridin-2-yl)-8-methyl-7-(2,2,2 -trifluoroethyl)-6,7,8,9-tetrahydrooxazolo[5,4-f]isoquinolin-2(3H)-one (Compound 5)

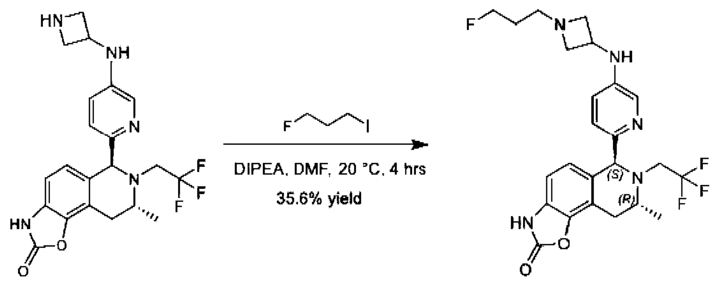


(6S,8R)-6-(5-(azetidine-3-amino)pyridin-2-yl)-8-methyl-7-(2,2,2-trifluoroethyl)-6, 7,8,9-tetrahydrooxazolo[5,4-f]isoquinolin-2(3H)-one (66.0 mg, 152 μmol, 1 eq) and 3-fluoro-1-iodopropane (28.6 mg, 152 μmol, 1 eq) were dissolved in N,N-dimethylformamide (1 mL) under nitrogen protection at 20°C, diisopropyl ethylenediamine (98.4 mg, 761 μmol, 132 uL, 5 eq) was added, and the mixture was reacted at 20°C for 4 h. LCMS monitored the completion of the reaction. The reaction solution was quenched by adding water, extracted with ethyl acetate, dried and concentrated. The crude product was separated by reversed-phase column and then separated by a chiral column to obtain (6S,8R)-6-(5-((1-(3-fluoropropane)azetidin-3-yl)amino)pyridin-2-yl)-8-methyl-7-(2,2,2 -trifluoroethyl)-6,7,8,9-tetrahydrooxazolo[5,4-f]isoquinolin-2(3H)-one as a yellow solid (27.0 mg, 54.2 μmol, 35.6% yield, 97.7% purity, 100% ee).

**$^1$H NMR** (400MHz, MeOH-$d_4$): δ = 7.76 - 7.71 (m, 1H), 7.13 (d, *J* = 8.4 Hz, 1H), 6.93 (m, 1H), 6.80 (d, *J* = 8.0 Hz, 1H), 6.56 (d, *J* = 8.0 Hz, 1H), 4.52 (t, *J* = 6.0 Hz, 1H), 4.40 (t, *J* = 6.0 Hz, 1H), 4.13 - 4.06 (m, 1H), 3.82 - 3.76 (m, 2H), 3.54 - 3.47 (m, 1H), 3.15 - 3.08 (m, 1H), 3.00 - 2.91 (m, 3H), 2.76 (m, 1H), 2.64 (t, *J* = 7.6 Hz, 2H), 1.83 - 1.69 (m, 2H), 1.06 (d, *J* = 6.6 Hz, 3H) ppm.

**LCMS**:m/z $(M+H)^+$ = 494.3.

**Example 7: (6S,8R)-6-(5-(((S)-1-(3-fluoropropane)pyrrolidin-3-yl)oxy)pyridin-2-yl)-8-methyl-7 -(2,2,2-trifluoroethyl)-6,7,8,9-tetrahydrooxazolo[5,4-f]isoquinolin-2(3H)-one (Compound 7)**

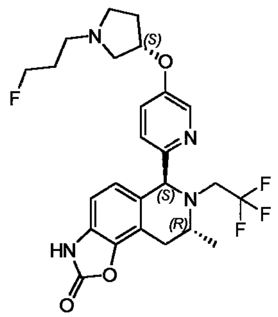

Step I: Synthesis of tert-butyl (S)-3-((6-((6S,8R)-8-methyl-2-oxo-7-(2,2,2-trifluoroethyl)-3-triphenylmethyl-2,3,6,7,8, 9-hexahydrooxazolo[5,4-f]isoquinolin-6-yl)pyridin-3-yl)oxy)pyrrolidine-1-carboxylate

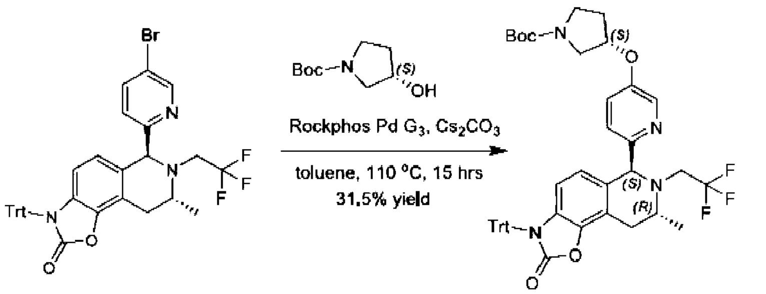


(6S,8R)-6-(5-bromopyridin-2-yl)-8-methyl-7-(2,2,2-trifluoroethyl)-3-triphenylmet hyl-6,7,8,9-tetrahydrooxazolo [5,4-f]isoquinolin-2(3H)-one (150 mg, 219 μmol, 1 eq) and tert-butyl (S)-3-hydroxypyrrolidine-1-carboxylate (102 mg, 547 μmol, 2.5 eq) were dissolved in toluene (20 mL), RockPhos Pd $G_3$ (18.3 mg, 21.9 μmol, 0.1 eq) and cesium carbonate (214 mg, 657 μmol, 3 eq) were then added, and the reaction solution was reacted at 115°C for 15 h. TLC monitored the completion of the reaction. The reaction solution was quenched by adding water, extracted with ethyl acetate, dried and concentrated. The crude product was separated by column chromatography to obtain tert-butyl (S)-3-((6-((6S,8R)-8-methyl-2-oxo-7-(2,2,2-trifluoroethyl)-3-triphenylmethyl-2,3,6,7,8, 9-hexahydrooxazolo[5,4-f]isoquinolin-6-yl)pyridin-3-yl)oxy)pyrrolidine-1-carboxylate as a yellow solid (120 mg, 138 μmol, 31.5% yield).

**$^1$H NMR** (400MHz, $CDCl_3$): δ = 8.02 (d, *J* = 2.8 Hz, 1H), 7.39 (d, *J* = 7.2 Hz, 6H), 7.24 - 7.20 (m, 5H), 7.18 - 7.14 (m, 4H), 7.08 - 7.04 (m, 1H), 6.37 - 6.25 (m, 1H), 5.66 (d, *J* = 8.4 Hz, 1H), 4.86 - 4.80 (m, 2H), 3.55 - 3.34 (m, 2H), 3.34 - 3.23 (m, 1H), 3.16 - 3.04 (m, 1H), 3.01 - 2.89 (m, 1H), 2.83 (br dd, $J_1$ = 8.8Hz, $J_2$ = 15.6 Hz, 1H), 2.59 (dd, $J_1$ = 6.4 Hz, $J_2$ =16.8 Hz, 1H), 2.15 - 2.01 (m, 2H), 1.95 - 1.81 (m, 2H), 1.40 (s, 9H), 1.02 (d, *J* = 6.4 Hz, 3H) ppm.

Step II: Synthesis of (6S,8R)-8-methyl-6-(5-((S)-pyrrolidin-3-oxy)pyridin-2-y1)-7-(2,2,2-trifluoroethyl)-6,7,8 ,9-tetrahydrooxazolo[5,4-f]isoquinolin-2(3H)-one

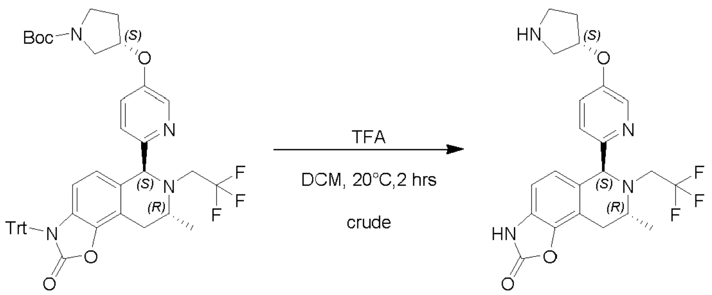


Trifluoroacetic acid (1.54 g, 13.4 mmol, 997 uL, 87.3 eq) was added and dissolved in tert-butyl (S)-3-((6-((6S,8R)-8-methyl-2-oxo-7-(2,2,2-trifluoroethyl)-3-triphenylmethyl-2,3,6,7,8, 9-hexahydrooxazolo[5,4-f]isoquinolin-6-yl)pyridin-3-yl)oxy)pyrrolidine-1-carboxylate (120 mg, 154 μmol, 1 eq) in dichloromethane (2 mL) at 0°C, and the mixture was reacted at 20°C for 2 h. TLC and LCMS monitored the completion of the reaction. The reaction solution was quenched by adding water, extracted with ethyl acetate, dried and concentrated. The crude product was separated by thin layer chromatography on a silica gel plate to obtain (6S,8R)-8-methyl-6-(5-((S)-pyrrolidin-3-oxy)pyridin-2-yl)-7-(2,2,2-trifluoroethyl)-6,7,8 ,9-tetrahydrooxazolo[5,4-f]isoquinolin-2(3H)-one as a yellow solid (105 mg, crude product).

**$^1$H NMR** (400 MHz, DMSO-d6): δ = 8.13 (d, *J* = 2.8 Hz, 1H), 7.45 - 7.34 (m, 1H), 7.32 - 7.26 (m, 1H), 6.87 - 6.82 (m, 1H), 6.74 - 6.67 (m, 1H), 5.14 - 5.02 (m, 2H), 3.00 - 2.92 (m, 2H), 2.90 - 2.78 (m, 2H), 2.72 - 2.71 (m, 1H), 2.74 - 2.58 (m, 1H), 2.21 - 2.07 (m, 2H), 2.06 - 1.93 (m, 1H), 1.23 (s, 1H), 1.06 (d, *J* = 6.4 Hz, 3H) ppm.

**LCMS:** m/z $(M+H)^+$ = 449.2.

Step III: Synthesis of (6S,8R)-6-(5-(((S)-1-(3-fluoropropane)pyrrolidin-3-yl)oxy)pyridin-2-yl)-8-methyl-7-(2, 2,2-trifluoroethyl)-6,7,8,9-tetrahydrooxazolo[5,4-f]isoquinolin-2(3H)-one (Compound 7)

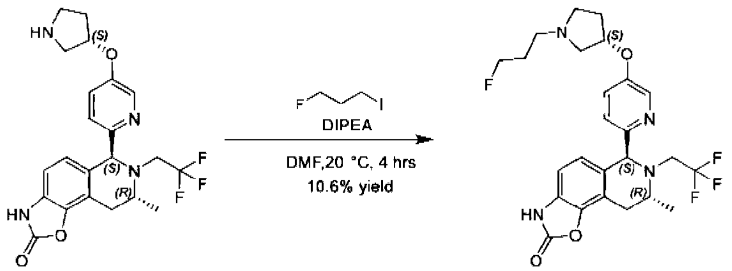


(6S,8R)-8-methyl-6-(5-((S)-pyrrolidin-3-oxy)pyridin-2-yl)-7-(2,2,2-trifluoroethyl)-6,7,8,9-tetrahydrooxazolo [5,4-f]isoquinolin-2(3H)-one (105 mg, 187 μmol, 1 eq) and 3-fluoro-1-iodopropane (35.2 mg, 187 μmol, 1 eq) were dissolved in N,N-dimethylformamide (2 mL) under nitrogen protection at 20°C, diisopropyl ethylenediamine (121 mg, 936 μmol, 163 uL, 5 eq) was added, and the mixture was reacted at 20°C for 4 h. LCMS detected the completion of the reaction. The reaction solution was quenched by adding water, extracted with ethyl acetate, dried and concentrated. The crude product was separated by a reversed-phase column to obtain (6S,8R)-6-(5-(((S)-1-(3-fluoropropane)pyrrolidin-3-yl)oxy) pyridin-2-yl)-8-methyl-7-(2, 2,2-trifluoroethyl)-6,7,8,9-tetrahydrooxazolo[5,4-f]isoquinolin-2(3H)-one as a white solid (10.5 mg, 19.8 μmol, 10.6% yield, 96 % purity, 100% ee).

**$^1$H NMR** (400MHz, MeOH-$d_4$): δ = 8.05 (d, *J* = 2.8 Hz, 1H), 7.40 - 7.30 (m, 2H), 6.83 (d, *J* = 8.0 Hz, 1H), 6.63 (d, *J* = 8.0 Hz, 1H), 4.54 (t, *J* = 6.0 Hz, 1H), 4.42 (t, *J* = 6.0 Hz, 1H), 3.53 - 3.48 (m, 1H), 3.40 (br d, *J* = 6.0 Hz, 1H), 3.10 (dd, $J_1$ = 4.8 Hz, $J_2$ =16.8 Hz, 1H), 3.02 - 2.87 (m, 4H), 2.78 (dd, $J_1$ = 6.2 Hz, $J_2$ =16.8 Hz, 1H), 2.68 - 2.58 (m, 2H), 2.56 - 2.49 (m, 1H), 2.38 (dt, $J_1$ = 7.6 Hz, $J_2$ =13.6 Hz, 1H), 1.99 - 1.84 (m, 3H), 1.29 (br s, 1H), 1.12 (d, *J* = 6.4 Hz, 3H) ppm.

**LCMS:** m/z $(M+H)^+$ = 509.4.

**Example 8: (6S,8R)-6-(2,6-difluoro-4-(6-(2-fluoroethyl)-2,6-diazspiro[3.3]hept-2-yl)phenyl)-8-methyl-7-(2,2,2-trifluoroethyl)-6,7,8,9-tetrahydrooxazolo[5,4-f]isoquinolin-2(3H)-o ne (Compound 8)**

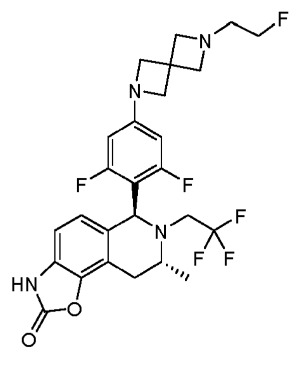

Step I: Synthesis of tert-butyl (6S,8R)-6-(3,5-difluoro-4-((6S,8R)-8-methyl-2-oxy-7-(2,2,2-trifluoroethyl)-3-triphenyl methyl-2,3,6,7,8,9-hexahydrooxazolo[5,4-f]isoquinolin-6-yl)phenyl)-2,6-diazspiro[3.3] heptane-2-carboxylate

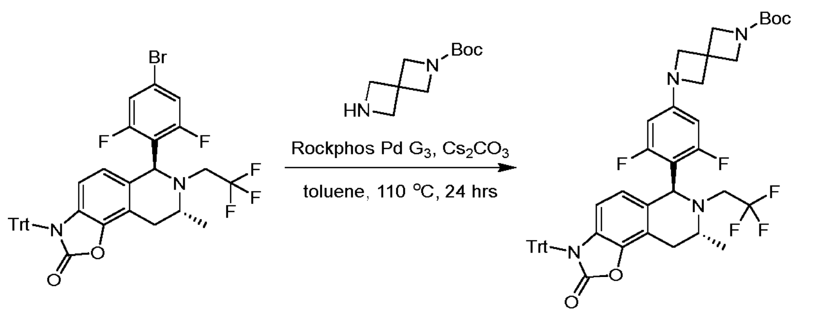


(6S,8R)-6-(4-bromo-2,6-difluorophenyl)-8-methyl-7-(2,2,2-trifluoroethyl)-3-triphe nylmethyl-6,7,8,9-tetrahydrooxazolo[5,4-f]isoquinolin-2(3H)-one (300 mg, 417 μmol) and tert-butyl 2,6-diazspiro[3.3]heptane-2-carboxylate (165.33 mg, 833.88 μmol) were dissolved in toluene (9 mL), cesium carbonate (272 mg, 834μmol) and [(2-bis-tert-butylphosphine-3-methoxy-6-methyl-2,4,6-triisopropyl-1,1-biphenyl)-2-(2-a minobiphenyl)]palladium(II) methanesulfonate (34.96 mg, 41.69 μmol) were added, and the mixture was reacted under nitrogen protection at 110°C for 24 h. LCMS detected the completion of the reaction. The reaction solution was concentrated and then purified by a silica gel column to obtain tert-butyl (6S,8R)-6-(3,5-difluoro-4-((6S,8R)-8-methyl-2-oxy-7-(2,2,2-trifluoroethyl)-3-triphenyl methyl-2,3,6,7,8,9-hexahydroisoquinolino[5,4-f]oxazol-6-yl)phenyl)-2,6-diazspiro[3.3] heptane-2-carboxylate (120 mg, 143 μmol, yield 34.4%).

**$^1$H NMR** (400MHz, $CDCl_3$): δ = 7.41 - 7.37 (m, 6H), 7.24 - 7.19 (m, 9H), 6.17 (d, *J= 8.5* Hz, 1H), 5.77 (d, *J* = 10.5 Hz, 2H), 5.56 (d, *J* = 8.5 Hz, 1H), 5.03 (s, 1H), 4.02 (s, 4H), 3.88 (s, 4H), 3.50 - 3.44 (m, 1H), 3.11 - 2.99 (m, 2H), 2.85 - 2.75 (m, 1H), 2.71 - 2.62 (m, 1H), 1.37 (s, 9H), 0.96 (d, *J* = 6.4 Hz, 3H) ppm.

**LCMS:** m/z 837.3 $[M+H]^+$.

Step II: Synthesis of (6S,8R)-6-(2,6-difluoro-4-(2,6-diazspiro[3.3]hept-2-yl)phenyl)-8-methyl-7-(2,2,2-triflu oroethyl)-6,7,8,9-tetrahydrooxazolo[5,4-f]isoquinolin-2(3H)-one

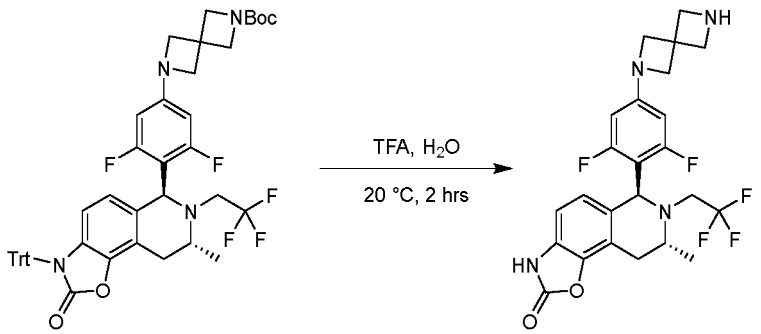


Tert-butyl 6-(3,5-difluoro-4-((6S,8R)-8-methyl-2-oxy-7-(2,2,2-trifluoroethyl)-3-triphenylmethyl-2 ,3,6,7,8,9-hexahydrooxazolo[5,4-f]isoquinolin-6-yl)phenyl)-2,6-diazspiro[3.3]heptane-2 -carboxylate (300 mg, 358.47μmol) was dissolved in trifluoroacetic acid (4.5 mL) and water (0.5 mL) at 0°C, and the mixture was heated to 20°C and stirred for 2 h. LCMS detected the completion of the reaction. The reaction product was concentrated, then adjusted to pH = 7-8, and extracted with water and ethyl acetate, and the organic phase was concentrated to obtain (6S,8R)-6-(2,6--difluoro-4-(2,6-diazspiro[3.3]hept-2-yl)phenyl)-8-methyl-7-(2,2,2-triflu oroethyl)-6,7,8,9-tetrahydrooxazolo[5,4-f]isoquinolin-2(3H)-one (350 mg, crude product).

**LCMS:** m/z 495.0 $[M+H]^+$.

Step III: Synthesis of (6S,8R)-6-(2,6-difluoro-4-(6-(2-fluoroethyl)-2,6-diazspiro[3.3]hept-2-yl)phenyl)-8-met hyl-7-(2,2,2-trifluoroethyl)-6,7,8,9-tetrahydrooxazolo[5,4-f]isoquinolin-2(3H)-one

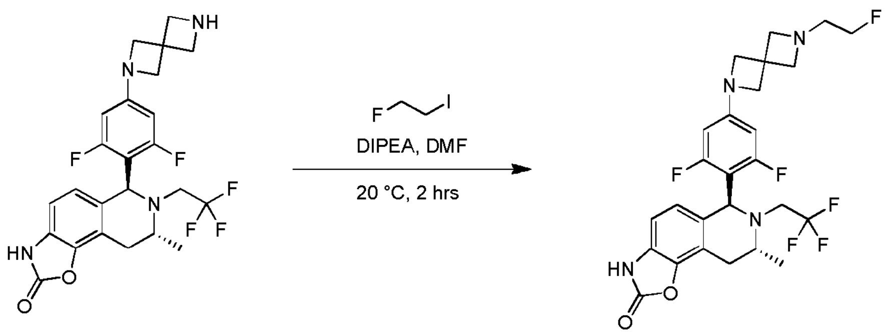


(6S,8R)-6-(2,6-difluoro-4-(2,6-diazspiro[3.3]hept-2-yl)phenyl)-8-methyl-7-(2,2,2-t rifluoroethyl)-6,7,8,9-tetrahydrooxazolo[5,4-f]isoquinolin-2(3H)-one (300 mg, 606.73 μmol), 1-iodo-3-fluoroethane (211.09 mg, 1.21 mmol) was dissolved in N,N-dimethylformamide (15 mL), N,N-diisopropylethylamine (313.66 mg, 2.43 mmol) was then added, and

the mixture was reacted at 20°C for 2 h. LCMS detected the completion of the reaction. The reaction product was extracted with water and ethyl acetate, and the organic phase was washed three times with brine, concentrated and then purified by a silica gel column to obtain (6S,8R)-6-(2,6-difluoro-4-(6-(2-fluoroethyl)-2,6-diazspiro[3.3]hept-2-yl)phenyl)-8-methyl-7-(2,2,2-trifluoroethyl)-6,7,8,9-tetrahydrooxazolo[5,4-f]isoquinolin-2(3H)-one (95 mg, 175.76 μmol, yield 28.9%).

**LCMS:** m/z 541.3 $[M+H]^+$.

Step IV: Resolution of (6S,8R)-6-(2,6-difluoro-4-(6-(2-fluoroethyl)-2,6-diazspiro[3.3]hept-2-yl)phenyl)-8-methyl-7-(2,2,2-trifluoroethyl)-6,7,8,9-tetrahydrooxazolo[5,4-f]isoquinolin-2(3H)-one (Compound 8)

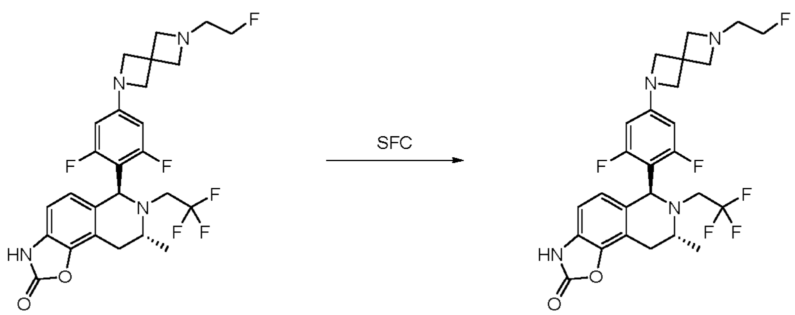


(6S,8R)-6-(2,6-difluoro-4-(6-(2-fluoroethyl)-2,6-diazspiro[3.3]hept-2-yl)phenyl)-8 -methyl-7-(2,2,2-trifluoroethyl)-6,7,8,9-tetrahydrooxazolo[5,4-f]isoquinolin-2(3H)-one was subjected to chiral resolution to obtain (6S,8R)-6-(2,6-difluoro-4-(6-(2-fluoroethyl)-2,6-diazspiro[3.3]hept-2-yl)phenyl)-8-met hyl-7-(2,2,2-trifluoroethyl)-6,7,8,9-tetrahydrooxazolo[5,4-f]isoquinolin-2(3H)-one (40 mg, 74.01 μmol, yield 44.4%).

**$^1$H NMR** (400MHz, MeCN-$d_3$) $\delta$ = 6.87 - 6.72 (m, 1H), 6.64 - 6.53 (m, 1H), 6.03 - 5.86 (m, 2H), 5.21 (s, 1H), 4.46 - 4.41 (m, 1H), 4.34 - 4.30 (m, 1H), 3.89 (s, 4H), 3.56 - 3.46 (m, 1H), 3.35 (s, 4H), 3.33 - 3.25 (m, 1H), 3.05 (dd, *J*=4.9, 16.4 Hz, 1H), 2.93 (qd, *J*=9.8, 15.9 Hz, 1H), 2.74 (dd, *J*=5.1, 16.5 Hz, 1H), 2.70 - 2.66 (m, 1H), 2.63 - 2.58 (m, 1H), 1.06 (d, J=6.5 Hz, 3H) ppm.

**Example 9: (6S,8R)-7-(2,2-difluoro-3-hydroxypropyl)-6-(2,6-difluoro-4-((1-(3-fluoropropyl)aze tidin-3-yl)amino) phenyl)-8-methyl-6,7,8,9-tetrahydrooxazolo[5,4-f]isoquinolin-2(3 H)-one (Compound 9)**

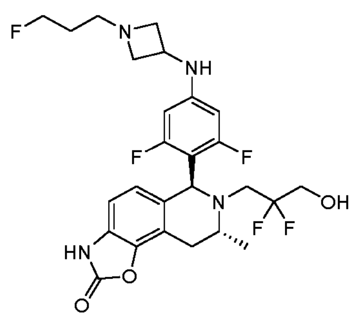

Step I: Synthesis of (R)-7-(2-((3-((tert-butylphenylsilyl)oxy)-2,2-difluoropropyl)amino)propyl)-3-triphenylb enzo[d]oxazol-2(3H)-one

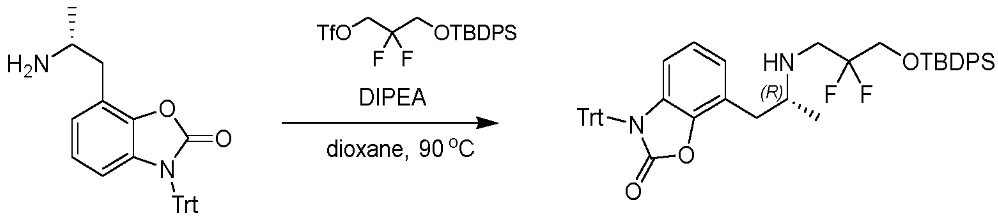


Subsequent steps: According to steps similar to those in Examples 1-8 and/or conventional reaction steps in the art, Compound 9 was synthesized starting from (R)-7-(2-((3-((tert-butylphenylsilyl)oxy)-2,2-difluoropropyl)amino)propyl)-3-triphenylb enzoldloxazol-2(3H)-one.

**$^1$H NMR:** (400 MHz, ACETONITRILE-d3)δ = 11. 6 (s, 1H), 9.16 (s, 1H), 6.81 (d, J = 8.0 Hz, 1H), 6.60 (br d, J = 8.0 Hz, 1H), 6.13 - 6.10 (m, 2H), 5.18 (s, 1H), 4.61 - 4.52 (m, 2H), 4.44 - 4.33 (m, 2H), 4.22 - 4.12 (m, 2H), 3.81 - 3.46 (m, 4H), 3.36 - 3.24 (m, 2H), 3.18 - 2.98 (m, 2H), 2.80 - 2.70 (m, 2H), 2.02 - 1.95 (m, 2H), 1.05 (d, J = 6.4 Hz, 3H) ppm.

Similarly to the synthesis route of Examples 1-9 above, the compounds of the following examples were synthesized by selecting corresponding reactants and synthesis methods known to those skilled in the art. Specifically, taking the compound of Step I of Example 9, i.e., "(R)-7-(2-((3-((tert-butylphenylsilyl)oxy)-2,2-difluoropropyl)amino) propyl)-3-triphenyl benzo[d]oxazol-2(3H)-one", as a raw material, the following Examples 13-15 were synthesized; taking the compound of Step III of Example 5, i.e., "(R)-7-2-aminopropyl)(2,2,2-trifluoroethyl-3-triphenylmethylbenzo[d]oxazol-2(3H)-on e", as a raw material, the following Examples 10-12, 16, 18, and 25 were synthesized; and taking the compound of Step IV of Example 1, i.e., "(R)-7-(2-aminopropyl)-3-triphenylmethylbenzo[d]oxazol-2(3H)-one", as a raw material, the following Example 20 was synthesized.

**Example 10: (6S,8R)-6-(2,6-difluoro-4-((1-(3-fluoropropyl)azetidin-3-yl)amino)phenyl)-8-methy l-7-(2,2,2-trifluoroethyl)-6,7,8,9-tetrahydrooxazolo[5,4-f]isoquinolin-2(3H)-one (Compound 10)**

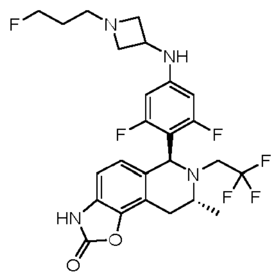

**$^1$H NMR:** (400 MHz, CDCl3) δ = 8.03 - 7.74 (m, 1H), 6.79 (s, 1H), 6.67 - 6.60 (m, 1H), 5.99 (d, J = 11.0 Hz, 2H), 5.21 (s, 1H), 4.59 - 4.51 (m, 1H), 4.48 - 4.40 (m, 1H), 4.38 - 4.25 (m, 1H), 4.12 - 3.95 (m, 1H), 3.78 - 3.67 (m, 2H), 3.63 - 3.51 (m, 1H), 3.26 - 3.09 (m, 2H), 3.00 - 2.86 (m, 3H), 2.78 (dd, J = 4.4, 16.4 Hz, 1H), 2.67 - 2.56 (m, 2H), 1.83 - 1.71 (m, 2H), 1.09 (d, J = 6.6 Hz, 3H) ppm.

**LCMS:** m/z (M+H)+ = 529.4

**Example 11: (6S,8R)-6-(2,6-difluoro-4-(((S)-1-(3-fluoropropane)pyrrolidin-3-yl)oxy)phenyl)-8-methyl-7-(2,2,2-trifluoroethyl)-6,7,8,9-tetrahydrooxazolo[5,4-f]isoquinolin-2(3H)-o ne (Compound 11)**

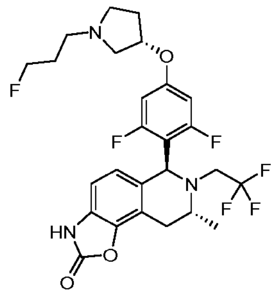

**$^1$H NMR:** (400MHz, ACETONITRILE-d3) δ = 6.81 (d, J = 8.0 Hz, 1H), 6.62 (d, J = 8.0 Hz, 1H), 6.44 (br d, J = 11.2 Hz, 2H), 5.28 (s, 1H), 4.79 (br s, 1H), 4.54 (t, J = 6.0 Hz, 1H), 4.42 (t, J = 6.0 Hz, 1H), 3.56 - 3.46 (m, 1H), 3.42 - 3.25 (m, 1H), 3.06 (br dd, J = 4.8, 16.6 Hz, 1H), 3.00 - 2.86 (m, 1H), 2.83 - 2.69 (m, 5H), 2.53 - 2.45 (m, 4H), 1.91 - 1.85 (m, 1H), 1.85 - 1.76 (m, 2H), 1.07 (d, J = 6.4 Hz, 3H) ppm.

**Example 12: (6S,8R)-6-(2,6-difluoro-4-((1-(3-fluoropropyl)azetidin-3-yl)oxy)phenyl)-8-methyl-7 -(2,2,2-trifluoroethyl)-6,7,8,9-tetrahydrooxazolo[5,4-f]isoquinolin-2(3H)-one (Compound 12)**

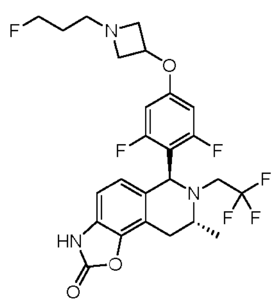

**$^1$H NMR:** (400MHz, CD3Cl) δ = 8.02 - 7.65 (m, 1H), 6.79 - 6.71 (m, 1H), 6.64 - 6.54 (m, 1H), 6.27 (d, J = 10.4 Hz, 2H), 5.29 - 5.22 (m, 1H), 4.72 (m, J = 6.0 Hz, 1H), 4.56 (t, J = 6.0 Hz, 1H), 4.44 (t, J = 6.0 Hz, 1H), 3.85 - 3.72 (m, 2H), 3.64 - 3.50 (m, 1H), 3.26 - 3.08 (m, 4H), 2.97 - 2.86 (m, 1H), 2.85 - 2.76 (m, 1H), 2.65 (t, J = 7.2 Hz, 2H), 1.83 - 1.70 (m, 2H), 1.10 (d, J = 6.4 Hz, 3H).

**LCMS:** m/z (M+H)+ = 530.2

**Example 13: (6S,8R)-7-(2,2-difluoro-3-hydroxypropyl)-6-(2,6-difluoro-4-(((S)-1-(3-fluoropropan e)pyrrolidin-3-yl)oxy)phenyl)-8-methyl-6,7,8,9-tetrahydrooxazolo[5,4-f]isoquinolin -2(3H)-one (Compound 13)**

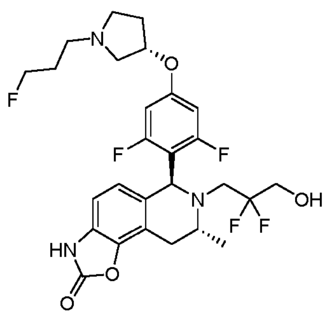

**$^1$H NMR:** (400 MHz, CHLOROFORM-d) δ = 8.36 (br d, J = 1.1 Hz, 1H), 6.79 (d, J = 8.1 Hz, 1H), 6.58 (d, J = 8.1 Hz, 1H), 6.40 (d, J = 10.4 Hz, 2H), 5.18 (s, 1H), 4.89 (br s, 1H), 4.61 (t, J = 5.6 Hz, 1H), 4.50 (t, J = 5.6 Hz, 1H), 3.77 - 3.61 (m, 3H), 3.29 - 2.92 (m, 7H), 2.91 - 2.75 (m, 3H), 2.54 - 2.38 (m, 1H), 2.24 - 2.01 (m, 3H), 1.10 (d, J = 6.6 Hz, 3H) ppm.

**Example 14: (6S,8R)-7-(2,2-difluoro-3-hydroxypropyl)-6-(2,6-difluoro-4-(6-(2-fluoroethyl)-2,6-d iazspiro[3.3] hept-2-yl)phenyl)-8-methyl-6,7,8,9-tetrahydrooxazolo[5,4-f]isoquinoli n-2(3H)-one (Compound 14)**

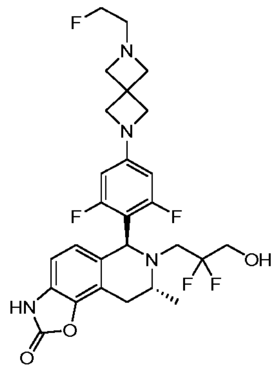

**$^1$H NMR:** (400 MHz, ACETONITRILE-d3) δ = 6.78 (br d, J = 6.6 Hz, 1H), 6.67 - 6.44 (m, 1H), 6.07 - 5.84 (m, 2H), 5.94 (br d, J = 11.4 Hz, 1H), 5.24 - 5.04 (m, 1H), 5.13 (br s, 1H), 4.50 - 4.26 (m, 2H), 3.89 (br s, 4H), 3.73 - 3.61 (m, 1H), 3.58 - 3.45 (m, 2H), 3.36 (br s, 4H), 3.13 - 3.00 (m, 2H), 3.18 - 2.97 (m, 1H), 2.77 - 2.59 (m, 5H), 2.80 - 2.57 (m, 1H), 1.07 - 0.97 (m, 3H), 1.03 (br d, J = 3.8 Hz, 1H) ppm.

**Example 15: (6S,8R)-7-(2,2-difluoro-3-hydroxypropyl)-6-(2,6-difluoro-4-(3-((3-fluoropropyl)am ino)azetidin-1-yl) phenyl)-8-methyl-6,7,8,9-tetrahydrooxazolo[5,4-f]isoquinolin-2(3 H)-one (Compound 15)**

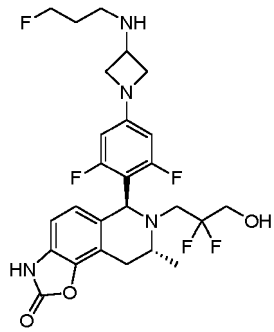

**$^1$H NMR:** (400MHz, ACETONITRILE-d3) δ = 6.85 (d, J = 8.1 Hz, 1H), 6.65 (d, J = 8.1 Hz, 1H), 5.95 (d, J = 11.5 Hz, 2H), 5.15 (s, 1H), 4.56 (t, J = 5.6 Hz, 1H), 4.44 (t, J = 5.6 Hz, 1H), 4.05 (t, J = 7.5 Hz, 2H), 3.91 - 3.81 (m, 3H), 3.72 - 3.48 (m, 3H), 3.43 - 3.38 (m, 2H), 3.18 - 3.02 (m, 2H), 2.82 - 2.64 (m, 2H), 2.13 - 2.01 (m, 4H), 1.03 (d, J = 6.5 Hz, 3H).

**Example 16: (E)-4-((2-(3,5-difluoro-4-((6S,8R)-8-methyl-2-oxy-7-(2,2,2-trifluoroethyl)-2,3,6,7,8, 9-hexahydrooxazolo[5,4-f]isoquinolin-6-yl)phenoxy)ethyl)amino)-N,N-dimethylbut -2-enamide (Compound 16)**

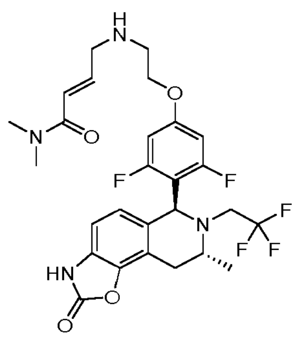

**$^1$H NMR:** (400 MHz, ACETONITRILE-d3) δ = 6.81 (d, J = 8.1 Hz, 1H), 6.75 - 6.65 (m, 1H), 6.61 (d, J = 8.1 Hz, 1H), 6.56 - 6.46 (m, 3H), 5.29 (s, 1H), 4.02 (t, J = 5.3 Hz, 2H), 3.57 - 3.50 (m, 2H), 3.41 - 3.38 (m, 2H), 3.37 - 3.30 (m, 1H), 3.11 - 3.04 (m, 1H), 3.02 (s, 3H), 2.94 - 2.91 (m, 2H), 2.90 - 2.88 (m, 3H), 2.79 - 2.72 (m, 1H), 1.11 (t, J = 7.0 Hz, 2H), 1.07 (d, J = 6.6

Hz, 3H) ppm.

LCMS: m/z 569.4 $[M+H]^+$.

**Example 18: (6S,8R)-6-(2,6-difluoro-4-(3-((3-fluoropropyl)amino)azetidin-1-yl)phenyl)-8-methy l-7-(2,2,2-trifluoroethyl)-6,7,8,9-tetrahydrooxazolo[5,4-f]isoquinolin-2(3H)-one (Compound 18)**

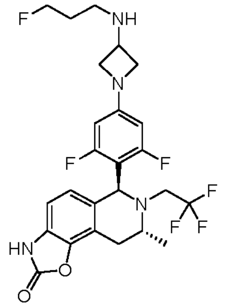

**$^1$H NMR:** (400MHz, ACETONITRILE-d3) δ = 6.81 (br d, J = 8.0 Hz, 1H), 6.61 (br d, J = 7.9 Hz, 1H), 5.95 (d, J = 11.6 Hz, 2H), 5.22 (s, 1H), 4.56 (t, J = 5.9 Hz, 1H), 4.44 (br t, J = 5.7 Hz, 1H), 4.03 (br t, J = 7.3 Hz, 2H), 3.75 - 3.63 (m, 1H), 3.58 - 3.44 (m, 3H), 3.36 - 3.24 (m, 1H), 3.10 - 2.86 (m, 2H), 2.80 - 2.71 (m, 1H), 2.64 (br t, J = 6.8 Hz, 2H), 1.86 - 1.71 (m, 3H), 1.06 (d, J = 6.6 Hz, 3H).

**Example 20: (6S,8R)-6-(2,6-difluoro-4-((1-(3-fluoropropyl)azetidin-3-yl)amino)phenyl)-7-((1-flu orocyclopropyl) methyl)-8-methyl-6,7,8,9-tetrahydrooxazolo[5,4-f]isoquinolin-2(3H )-one (Compound 20)**

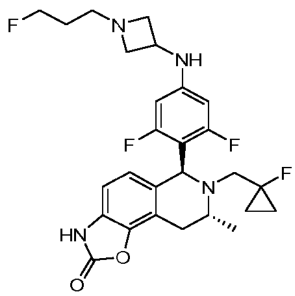

**$^1$H NMR:** (400 MHz, ACETONITRILE-d3) δ = 8.12 (s, 1H), 6.82 (d, J = 8.3 Hz, 1H), 6.61 (d, J = 8.0 Hz, 1H), 6.10 (d, J = 11.6 Hz, 2H), 5.38 (br d, J = 7.3 Hz, 1H), 5.12 (s, 1H), 4.55 (t, J = 6.1 Hz, 1H), 4.43 (t, J = 6.0 Hz, 1H), 4.08 - 3.97 (m, 1H), 3.75 (br d, J = 6.3 Hz, 3H), 3.12 - 2.99 (m, 2H), 2.97 - 2.90 (m, 2H), 2.83 - 2.70 (m, 2H), 2.62 (t, J = 7.1 Hz, 2H), 1.78 - 1.66 (m, 2H), 1.02 (d, J = 6.6 Hz, 3H), 0.97 - 0.86 (m, 2H), 0.62 - 0.40 (m, 2H) ppm.

**Example 25: (6S,8R)-6-(4-((1-(3-fluoropropyl)azetidin-3-yl)amino)-2-methoxyphenyl)-8-methyl-7-(2,2,2-trifluoroethyl)-6,7,8,9-6,7,8,9-tetrahydrooxazolo[5,4-f]isoquinolin-2(3H)-o ne (Compound 25)**

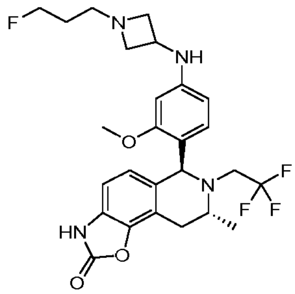

Step I: Synthesis of (6S,8R)-6-(4-bromo-2-methoxyphenyl)-8-methyl-7-(2,2,2-trifluoroethyl)-6,7,8,9-tetrah ydrooxazolo [5,4-flisoquinolin-2(3H)-one

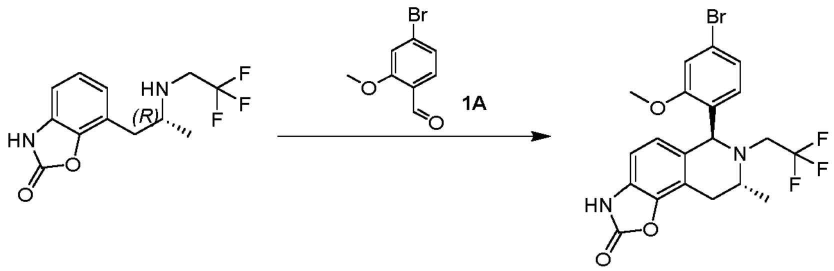


Compound (R)-7-(2-((2,2,2-trifluoroethyl)amino)propyl)benzo[d]oxazol-2(3H)-one (3.00 g, 10.9 mmol), 4-bromo-2-methoxybenzaldehyde (3.29 g, 5.32 mmol), and trifluoroacetic acid (6.24 g, 54.7 mmol) were dissolved in toluene (15 mL) and reacted under nitrogen protection at 110°C for 48 h. LCMS detected the completion of the reaction. The reaction solution was concentrated and then purified by column chromatography to obtain (6S,8R)-6-(4-bromo-2-methoxyphenyl)-8-methyl-7-(2,2,2-trifluoroethyl)-6,7,8,9-tetrah ydrooxazolo[5,4-flisoquinolin-2(3H)-one as a white solid (4.35 g, 9.23 mmol, yield 84.4%).

**LCMS:** m/z 470.0 $[M+H]^+$.

**$^1$H NMR** (400 MHz, DMSO-d6) $\delta$ = 11.53 (s, 1 H), 7.23 (d, J=1.75 Hz, 1 H), 6.94 - 7.11 (m, 1 H), 6.82 (d, J=8.00 Hz, 1 H), 6.68 (d, J=8.13 Hz, 1 H), 6.54 (d, J=8.13 Hz, 1 H), 5.28 - 5.52 (m, 1 H), 3.82 - 3.91 (m, 3 H), 3.27 - 3.41 (m, 2 H), 2.77 - 3.02 (m, 2 H), 2.68 (m, J=16.70, 7.30 Hz, 1 H), 1.02 (d, J=6.50 Hz, 3 H) ppm.

Step II: Synthesis of (6S,8R)-6-(4-bromo-2-methoxyphenyl)-8-methyl-7-(2,2,2-trifluoroethyl)-3-triphenylme thyl-6,7,8,9-tetrahydrooxazolo[5,4-f]isoquinolin-2(3H)-one

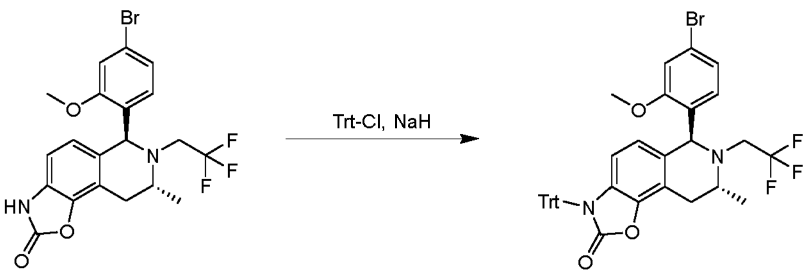


(6S,8R)-6-(4-bromo-2-methoxyphenyl)-8-methyl-7-(2,2,2-trifluoroethyl)-6,7,8,9-t etrahydrooxazolo[5,4-f]isoquinolin-2(3H)-one (2.77 g, 5.88 mmol) was dissolved in N,N-dimethylformamide (25 mL), sodium hydride (353 mg, 8.82 mmol) was added under nitrogen protection at 0°C, triphenylmethyl chloride (1.64 g, 5.88 mmol) was then added, and the reaction solution was reacted at 25°C for 2 h. TLC detected the completion of the reaction. The reaction solution was quenched by adding a saturated ammonium chloride aqueous solution and extracted with ethyl acetate, and the organic phase was washed with a saturated sodium chloride aqueous solution, dried with sodium sulfate and concentrated. The crude product was purified by column chromatography to obtain (6S,8R)-6-(4-bromo-2-methoxyphenyl)-8-methyl-7-(2,2,2-trifluoroethyl)-3-triphenylme thyl-6,7,8,9-tetrahydrooxazolo[5,4-f]isoquinolin-2(3H)-one as a yellow solid (3.75 g, 5.26 mmol, yield 89.4%).

**LCMS:** m/z 712.2 $[M+H]^+$.

**$^1$H NMR** (400 MHz, CHLOROFORM-d) δ = 7.18 - 7.42 (m, 15 H), 6.92 - 6.96 (m, 1 H), 6.88 (dd, J=8.13, 1.75 Hz, 1 H), 6.70 (d, J=8.25 Hz, 1 H), 6.14 (d, J=8.63 Hz, 1 H), 5.55 - 5.66 (m, 1 H), 5.23 (s, 1 H), 3.76 (s, 3 H), 3.35 - 3.48 (m, 1 H), 2.89 - 3.04 (m, 2 H), 2.64 - 2.79 (m, 1 H), 2.61 (br d, J=6.25 Hz, 1 H), 0.97 (d, J=6.63 Hz, 3 H) ppm.

Step III: Synthesis of (6S,8R)-6-(4-((1-(3-fluoropropyl)azetidin-3-yl)amino)-2-methoxyphenyl)-8-methyl-7-( 2,2,2-trifluoroethyl)-3-triphenylmethyl-6,7,8,9-tetrahydrooxazolo[5,4-f]isoquinolin-2(3 H)-one

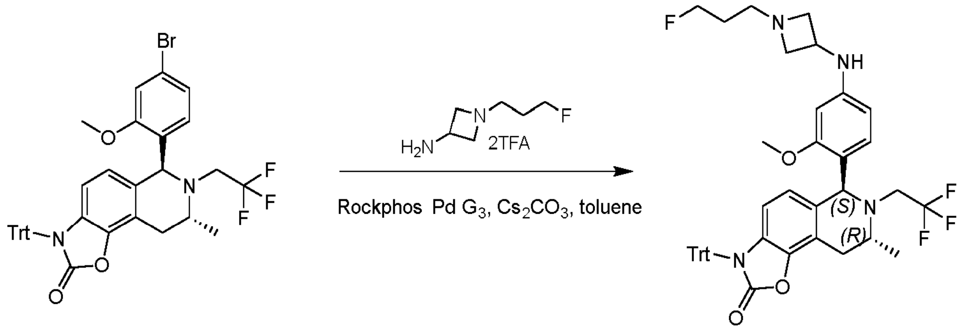


(6S,8R)-6-(4-bromo-2-methoxyphenyl)-8-methyl-7-(2,2,2-trifluoroethyl)-3-triphen ylmethyl-6,7,8,9-tetrahydrooxazolo[5,4-f]isoquinolin-2(3H)-one (2 g, 2.80 mmol), 1-(3-fluoropropyl)-3-aminoazetidine (1.41 g, 3.92 mmol), cesium carbonate (2.74 g, 8.41 mmol), and [(2-bis-tert-butylphosphine-3-methoxy-6-methyl-2,4,6-triisopropyl-1,1-biphenyl)-2-(2-a minobiphenyl)]palladium(II) methanesulfonate (118 mg, 140 umol) were dissolved in toluene (20 mL), and the mixture was reacted under nitrogen protection at 110°C for 12 h. LCMS monitored the completion of the reaction. The reaction product was concentrated and purified by column chromatography to obtain (6S,8R)-6-(4-((1-(3-fluoropropyl) azetidin-3-yl)amino)-2-methoxyphenyl)-8-methyl-7-( 2,2,2-trifluoroethyl)-3-triphenylmethyl-6,7,8,9-tetrahydrooxazolo [5,4-f]isoquinolin-2(3 H)-one as a yellow solid (1 g, 1.31 mmol, yield 46.7%).

**LCMS:** m/z 764.3 $[M+H]^+$.

**$^1$H NMR** (400 MHz, CHLOROFORM-d) δ = 7.18 (br d, J=2.38 Hz, 15 H), 6.58 (d, J=8.25 Hz, 1 H), 6.13 - 6.25 (m, 1 H), 5.97 - 6.04 (m, 1 H), 5.92 (dd, J=8.25, 2.13 Hz, 1 H), 5.50 - 5.65 (m, 1 H), 5.14 - 5.18 (m, 1 H), 4.48 (t, J=5.94 Hz, 1 H), 4.36 (t, J=5.94 Hz, 1 H), 3.90 (br d, J=6.75 Hz, 1 H), 3.61 - 3.79 (m, 6 H), 3.44 (br d, J=5.50 Hz, 1 H), 3.00 - 3.29 (m, 1 H), 2.87 - 3.00 (m, 2 H), 2.79 (br d, J=6.13 Hz, 2 H), 2.49 - 2.59 (m, 3 H), 1.64 - 1.73 (m, 2 H), 0.97 (d, J=6.50 Hz, 3 H) ppm.

Step IV: Synthesis of (6S,8R)-6-(4-((1-(3-fluoropropyl)azetidin-3-yl)amino)-2-methoxyphenyl)-8-methyl-7-( 2,2,2-trifluoroethyl)-6,7,8,9-tetrahydrooxazolo[5,4-f]isoquinolin-2(3H)-one (Compound 25)

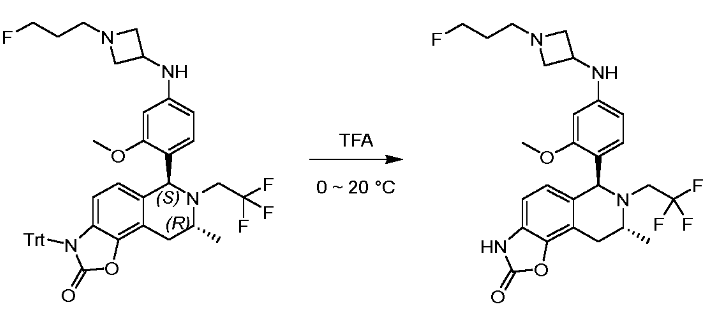


Trifluoroacetic acid (15.4 g, 135 mmol) and (6S,8R)-6-(4-((1-(3-fluoropropyl)azetidin-3-yl)amino)-2-methoxyphenyl)-8-methyl-7-( 2,2,2-trifluoroethyl)-3-triphenylmethyl-6,7,8,9-tetrahydrooxazolo[5,4-f]isoquinolin-2(3 H)-one (1 g, 1.31 mmol) were dissolved in water (1 mL), and the reaction solution was reacted under nitrogen protection at 20°C for 1 h. LCMS detected the completion of the reaction. The mixture was concentrated, then adjusted to pH = 7 with sodium

bicarbonate, and extracted with water and ethyl acetate, and the organic phase was concentrated. The crude product was purified by column chromatography and then subjected to SFC chiral resolution to obtain (6S,8R)-6-(4-((1-(3-fluoropropyl) azetidin-3-yl)amino)-2-methoxyphenyl)-8-methyl-7-( 2,2,2-trifluoroethyl)-6,7,8,9-tetrahydrooxazolo[5,4-f]isoquinolin-2(3H)-one as a yellow solid (300 mg, 571 umol, yield 43.7%).

**LCMS:** m/z 522.2 $[M+H]^+$.

**$^1$H NMR** (400 MHz, ACETONITRILE-d3) δ = 6.79 (d, J=8.13 Hz, 1 H), 6.44 - 6.59 (m, 2 H), 6.19 (d, J=2.00 Hz, 1 H), 5.97 (dd, J=8.32, 2.06 Hz, 1 H), 5.31 (s, 1 H), 4.75 (br d, J=7.00 Hz, 1 H), 4.52 (t, J=6.07 Hz, 1 H), 4.40 (t, J=6.07 Hz, 1 H), 3.92 - 4.09 (m, 1 H), 3.79 (s, 3 H), 3.68 (m, J=3.25, 2.13 Hz, 2 H), 3.43 - 3.54 (m, 1 H), 3.17 - 3.30 (m, 1 H), 2.87 - 3.01 (m, 2 H), 2.76, (m, J=6.38 Hz, 2 H), 2.61 - 2.67 (m, 1 H), 2.51 (t, J=7.00 Hz, 2 H), 1.63 - 1.76 (m, 2 H), 1.04 (d, J=6.50 Hz, 3 H) ppm.

**Example 26: (6S,8R)-6-(5-((1-(3-fluoropropane)azetidin-3-yl)amino)pyridin-2-yl)-8-methyl-7-(2, 2,2-trifluoroethyl)-6,7,8,9-tetrahydroxazolo[5,4-f]isoquinolin-2(3H)-one (Compound 26)**

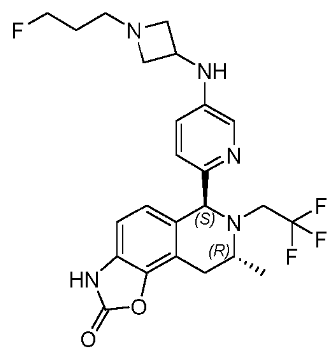

Step I: Synthesis of tert-butyl (R)-(1-(2-oxo-3-triphenylmethyl-2,3-dihydrobenzo[d]oxazol-7-yl)prop-2-yl)carbamate

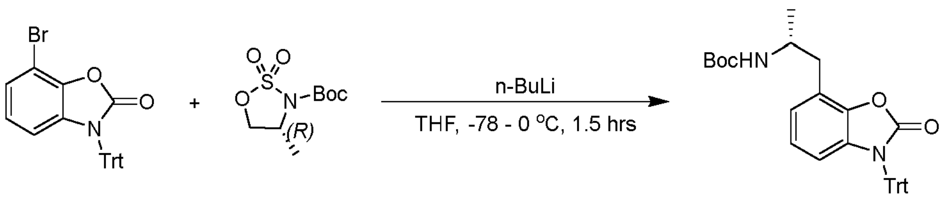


7-Bromo-3-triphenylmethyl-benzoxazol-2-one (100 g, 219 mmol, 1 eq) was dissolved in tetrahydrofuran (1000 mL), n-butyl lithium (2.5 M, 87.6 mL, 1 eq) was added under nitrogen protection at -78°C, and the mixture was stirred for 30 min. A solution of tert-butyl (R)-4-methyl-1,2,3-oxathiazolidine-3-carboxylate 2,2-dioxide (52.0 g, 219 mmol, 1 eq) in tetrahydrofuran (100 mL) was added to the reaction system at -78°C, which was then naturally recovered to 0°C and reacted for 2 h. TLC detected the completion of the reaction. The reaction solution was poured into a saturated ammonium chloride aqueous solution and quenched, extracted with ethyl acetate, dried and concentrated. The crude product was separated by column chromatography to obtain tert-butyl (R)-(1-(2-oxo-3-triphenylmethyl-2,3-dihydrobenzo[d]oxazol-7-yl)prop-2-yl)carbamate as a yellow solid (360 g, 673 mmol, yield 74.9%).

**$^1$H NMR** (400 MHz, CHLOROFORM-d) δ = 7.52 - 7.47 (m, 6H), 7.35 - 7.27 (m, 9H), 6.92 - 6.85 (m, 1H), 6.76 (t, *J* = 8.0 Hz, 1H), 5.93 (br d, *J* = 8.0 Hz, 1H), 4.00 (s, 1H), 3.81 - 3.72 (m, 1H), 2.84 (br d, *J* = 6.0 Hz, 2H), 1.40 (s, 9H), 1.16 (d, *J* = 6.4 Hz, 3H) ppm.

Step II: Synthesis of (R)-7-(2-aminopropyl)-3-triphenylmethylbenzo[d]oxazol-2(3H)-one

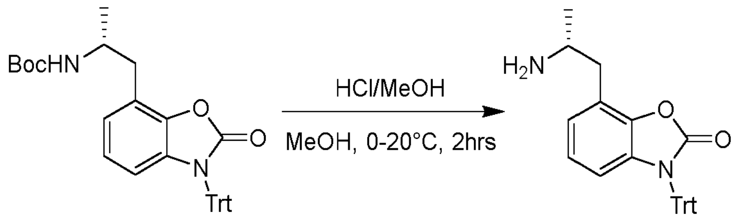


Tert-butyl (R)-(1-(2-oxo-3-triphenylmethyl-2,3-dihydrobenzo[d]oxazol-7-yl)prop-2-yl)carbamate (240 g, 448 mmol, 1 eq) was dissolved in methanol (400 ml) under nitrogen protection at 0°C, and a methanolic hydrochloride solution (6 M, 800 mL, 10.6 eq) was then added. After 2 hours of reaction at 0°C, TLC detected the completion of the reaction. The reaction solution was concentrated, neutralized by adding saturated sodium bicarbonate, extracted with ethyl acetate and concentrated to obtain a crude product, which was slurried with petroleum ether : ethyl acetate = 1 : 1 and filtered to obtain (R)-7-(2-aminopropyl)-3-triphenylmethylbenzo[d]oxazol-2(3H)-one as a yellow solid (198 g, 455 mmol, yield 84.6%).

**$^1$H NMR** (400 MHz, CHLOROFORM-d) δ = 7.48 - 7.34 (m, 6H), 7.23 - 7.14 (m, 9H), 6.81 - 6.73 (m, 1H), 6.66 (t, *J* = 8.0 Hz, 1H), 5.88 (m, 1H), 3.33 (s, *J* = 6.4 Hz, 1H), 2.82 - 2.65 (m, 2H), 1.12 (d, *J* = 6.4 Hz, 3H) ppm.

Step III: Synthesis of (R)-7-(2-((2,2,2-trifluoroethyl)amino)propyl)-3-triphenylmethylbenzo[d]oxazol-2(3H)-one

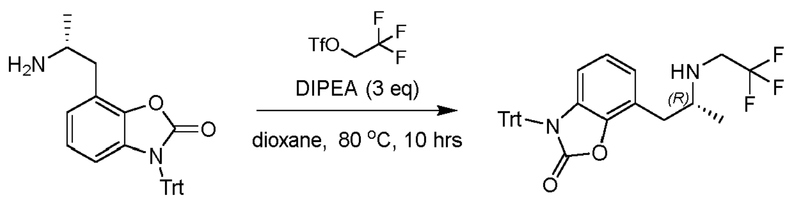


(R)-7-(2-aminopropyl)-3-triphenylmethylbenzo[d]oxazol-2(3H)-one (10.0 g, 23.0 mmol, 1 eq), 2,2,2-trifluoroethyl trifluoromethanesulfonate (6.14 g, 26.5 mmol, 1.15 eq) and diisopropyl ethylenediamine (8.92 g, 69.0 mmol, 12.0 mL, 3 eq) were dissolved in dioxane (100 mL) under nitrogen protection at 20°C and then reacted under nitrogen protection at 80°C for 10 h. TLC detected the completion of the reaction. The reaction solution was concentrated, and the crude product was separated by column chromatography to obtain (R)-7-(2-((2,2,2-trifluoroethyl)amino)propyl)-3-triphenylmethylbenzo[d]oxazol-2(3H)-one as a white solid (9.00 g, 17.4 mmol, yield 75.7%).

**$^1$H NMR** (400 MHz, CHLOROFORM-d) δ = 7.48 (br d, *J* = 7.2 Hz, 6H), 7.33 - 7.27 (m, 9H), 6.86 (br d, *J* = 7.6 Hz, 1H), 6.79 - 6.73 (m, 1H), 5.95 (br d, *J* = 8.0 Hz, 1H), 3.50 (s, 1H), 3.26 (br d, *J* = 9.3 Hz, 3H), 2.98 - 2.85 (m, 1H), 2.82 - 2.71 (m, 1H), 1.15 (br d, *J* = 5.6 Hz, 3H) ppm.

Step IV: Synthesis of (6S,8R)-6-(5-bromopyridin-2-yl)-8-methyl-7-(2,2,2-trifluoroethyl)-6,7,8,9-tetrahydrois oquinolino [5,4-f]oxazol-2(3H)-one

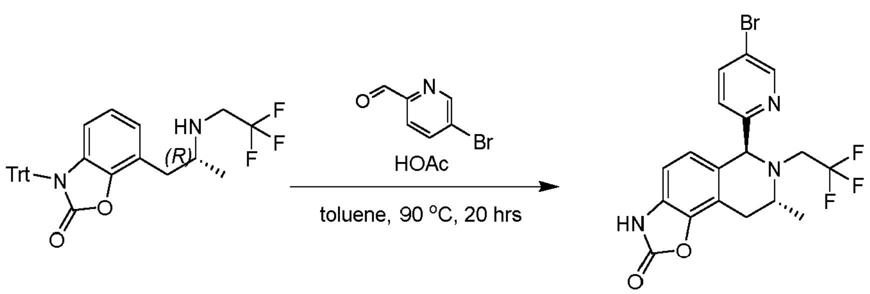


(R)-7-(2-((2,2,2-trifluoroethyl)amino)propyl)-3-triphenylmethylbenzo[d]oxazol-2( 3H)-one (1.00 g, 1.94 mmol, 1 eq) and 5-bromo-2-pyridinecarboxaldehyde (540 mg, 2.90 mmol, 1.5 eq) was dissolved in toluene (50 mL), trifluoroacetic acid (662 mg, 5.81 mmol, 430 uL, 3 eq) was added, and the mixture was reacted at 90°C for 20 h. TLC detected the completion of the reaction. The reaction solution was concentrated, and the crude product was purified by-TLC (petroleum

ether : ethyl acetate = 10 : 1) to obtain (6S,8R)-6-(5-bromopyridin-2-yl)-8-methyl-7-(2,2,2-trifluoroethyl)-6,7,8,9-tetrahydrois oquinolino[5,4-f]oxazol-2(3H)-one as a yellow solid (780 mg, 1.76 mmol, yield 91.1%).

**$^1$H NMR** (400 MHz, CHLOROFORM-d) δ = 8.89 (s, 1H), 8.54 (d, *J* = 2.4 Hz, 1H), 7.84 - 7.80 (m, 1H), 7.48 (d, *J* = 8.4 Hz, 1H), 6.79 - 6.74 (m, 2H), 5.09 (s, 1H), 4.13 (q, *J* = 7.2 Hz, 1H), 3.50 - 3.39 (m, 1H), 3.35 - 3.22 (m, 1H), 3.09 - 3.03 (m, 1H), 3.00 - 2.89 (m, 1H), 2.70 - 2.64 (m, 1H), 1.13 (d, *J* = 6.4 Hz, 3H) ppm.

Step V: Synthesis of (6S,8R)-6-(5-bromopyridin-2-yl)-8-methyl-7-(2,2,2-trifluoroethyl)-3-triphenylmethyl-6, 7,8,9-tetrahydroxazolo[5,4-f]isoquinolin-2(3H)-one

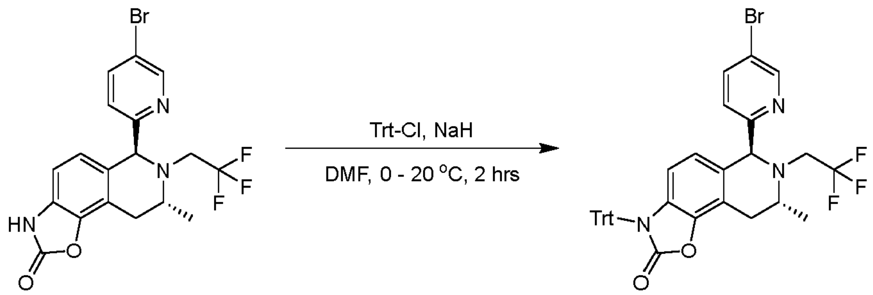


(6S,8R)-6-(5-bromopyridin-2-yl)-8-methyl-7-(2,2,2-trifluoroethyl)-6,7,8,9-tetrahy droisoquinolino[5,4-f]oxazol-2(3H)-one (1.00 g, 2.26 mmol, 1 eq) was dissolved in N,N-dimethylformamide (20 mL), sodium hydride (181 mg, 4.52 mmol, 60% purity, 2 eq) was added at 0°C, triphenylmethyl chloride (693 mg, 2.49 mmol, 1.1 eq) was then added at 20°C, and the mixture was reacted for 2 h. TLC detected the completion of the reaction. The reaction solution was quenched by adding water, extracted with ethyl acetate, dried and concentrated. The crude product was separated by column chromatography to obtain (6S,8R)-6-(5-bromopyridin-2-yl)-8-methyl-7-(2,2,2-trifluoroethyl)-3-triphenylmethyl-6, 7,8,9-tetrahydroxazolo[5,4-f]isoquinolin-2(3H)-one as a white solid (1.20 g, 1.56 mmol, yield 69.0%).

**$^1$H NMR** (400 MHz, CHLOROFORM-d) δ = 8.52 (s, 1H), 7.75 (dd, $J_1$ = 2.4 Hz, $J_2$ =8.4 Hz, 1H), 7.49 - 7.45 (m, 6H), 7.36 (d, *J* = 8.4 Hz, 1H), 7.32 - 7.27 (m, 9H), 6.39 (d, *J* = 8.4 Hz, 1H), 5.75 (d, *J* = 8.4 Hz, 1H), 4.94 (s, 1H), 3.53 - 3.42 (m, 1H), 3.29 - 3.18 (m, 1H), 3.01 (dd, $J_1$ = 4.8 Hz, $J_2$ =16.8 Hz, 1H), 2.90 (br dd, $J_1$ = 9.2 Hz, $J_2$ =15.6 Hz, 1H), 2.68 (dd, $J_1$ = 6.8 Hz, $J_2$ =16.8 Hz, 1H), 1.10 (d, *J*= 6.4 Hz, 3H) ppm.

Step VI: Synthesis of tert-butyl 3-((6-((6S,8R)-8-methyl-2-oxo-7-(2,2,2-trifluoroethyl)-3-triphenylmethyl-2,3,6,7,8,9-hexahydrooxazolo[5,4-f]isoquinolin-6-yl)pyridin-3-yl)amino)azetidine-1-carboxylate

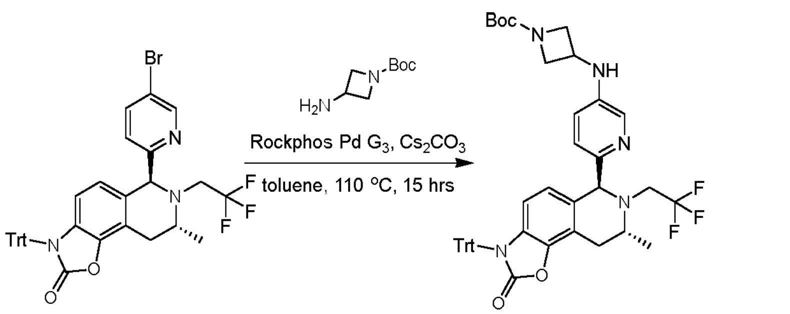


(6S,8R)-6-(5-bromopyridin-2-yl)-8-methyl-7-(2,2,2-trifluoroethyl)-3-triphenylmet hyl-6,7,8,9-tetrahydroxazolo[5,4-f]isoquinolin-2(3H)-one (200 mg, 292 umol, 1 eq) and tert-butyl 3-aminoazetidine-1-carboxylate (126 mg, 730 umol, 2.5 eq) were dissolved in 8 ml of toluene, [(2-2-tert-butylphosphine -3-methoxy-6-methyl-2,4,6-triisopropyl-1,1-biphenyl)-2-(2-aminobiphenyl)]palladium( II) methanesulfonate (24.5 mg, 29.2 umol, 0.1 eq) and cesium carbonate (286 mg, 876 umol, 3 eq) were added, and the reaction solution was reacted at 110°C for 15 h. TLC detected the completion of the reaction. The reaction solution was quenched by adding water, extracted with ethyl acetate, dried and concentrated. The crude product was separated by column chromatography to obtain tert-butyl 3-((6-((6S,8R)-8-methyl-2-oxo-7-(2,2,2-trifluoroethyl)-3-triphenylmethyl-2,3,6,7,8,9-he xahydrooxazolo[5,4-f]isoquinolin-6-yl)pyridin-3-yl)amino)azetidine-1-carboxylate as a yellow solid (120 mg, 137 umol, yield 47.2 %).

**$^1$H NMR** (400 MHz, CHLOROFORM-d) δ = 7.77 (d, *J* = 2.4 Hz, 1H), 7.52 - 7.42 (m, 7H), 7.32 - 7.28 (m, 4H), 7.26 - 7.17 (m, 9H), 6.89 - 6.69 (m, 1H), 6.37 (d, *J* = 8.4 Hz, 1H), 5.80 - 5.64 (m, 1H), 5.39 - 5.20 (m, 1H), 4.98 - 4.77 (m, 1H), 4.33 - 4.27 (m, 2H), 4.23 - 4.11 (m, 1H), 4.08 - 3.98 (m, 1H), 3.74 (m, 2H), 3.53 - 3.43 (m, 2H), 3.26 - 3.11 (m, 1H), 3.04 - 2.85 (m, 2H), 2.72 - 2.59 (m, 1H), 1.45 (s, 9H), 1.09 (d, *J* = 6.4 Hz, 3H) ppm.

Step VII: Synthesis of (6S,8R)-6-(5-(azetidin-3-ylamino)pyridin-2-yl)-8-methyl-7-(2,2,2-trifluoroethyl)-6,7,8, 9-tetrahydrooxazolo[5,4-f]isoquinolin-2(3H)-one

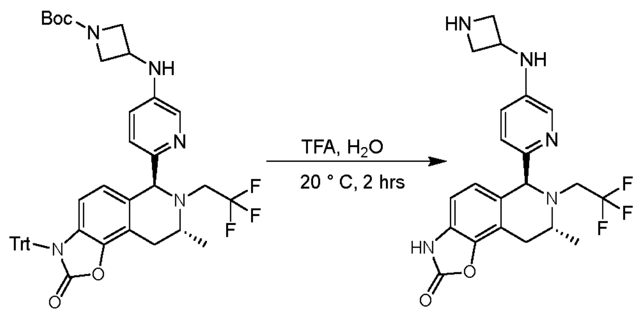


Trifluoroacetic acid (9.00 mL) and water (1.00 mL) were added to tert-butyl 3-((6-((6S,8R)-8-methyl-2-oxo-7-(2,2,2-trifluoroethyl)-3-triphenylmethyl-2,3,6,7,8,9-he xahydrooxazolo[5,4-f]isoquinolin-6-yl)pyridin-3-yl)amino) azetidine-1-carboxylate (120 mg, 154 umol, 1 eq) at 0°C, and the mixture was reacted at 20°C for 2 h. TLC monitored the completion of the reaction. The reaction solution was quenched by adding water, extracted with ethyl acetate, dried and concentrated to obtain (6S,8R)-6-(5-(azetidin-3-ylamino)pyridin-2-yl)-8-methyl-7-(2,2,2-trifluoroethyl)-6,7,8, 9-tetrahydrooxazolo[5,4-f]isoquinolin-2(3H)-one as a yellow solid (120 mg, crude product).

**$^1$H NMR** (400 MHz, CHLOROFORM-d) δ = 8.21 - 7.93 (m, 2H), 7.60 - 7.35 (m, 2H), 6.95 - 6.62 (m, 1H), 5.55 - 5.17 (m, 1H), 4.39 - 4.13 (m, 1H), 4.06 - 3.90 (m, 1H), 3.63 - 3.42 (m, 1H), 2.33 - 2.13 (m, 2H), 2.07 - 1.96 (m, 2H), 1.14 - 1.08 (m, 2H), 0.88 (br d, *J* = 4.8 Hz, 3H) ppm.

Step VIII: Synthesis of (6S,8R)-6-(5-((1-(3-fluoropropane)azetidin-3-yl)amino)pyridin-2-yl)-8-methyl-7-(2,2,2 -trifluoroethyl)-6,7,8,9-tetrahydroxazolo[5,4-f]isoquinolin-2(3H)-one

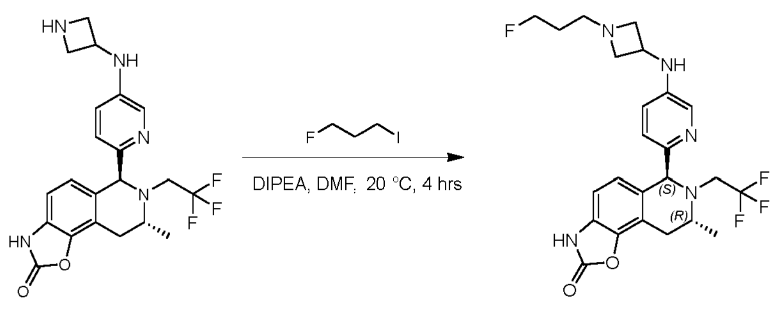


(6S,8R)-6-(5-(azetidin-3-ylamino)pyridin-2-yl)-8-methyl-7-(2,2,2-trifluoroethyl)-6 ,7,8,9-tetrahydroxazolo[5,4-f] isoquinolin-2(3H)-one (66.0 mg, 152 umol, 1 eq) and 3-fluoro-1-iodopropane (28.6 mg, 152 umol, 1 eq) were dissolved in N,N-dimethylformamide (1 mL) under nitrogen protection at 20°C, diisopropyl ethylenediamine (98.4 mg, 761 umol, 132 uL, 5 eq) was added, and the mixture was reacted at 20°C for 4 h. LCMS monitored the completion of the reaction. The reaction solution was quenched by adding water, extracted with ethyl acetate, dried and concentrated. The crude product was separated by a reversed-phase column (column: 3_Phenomenex Luna C18 75 * 30 mm * 3 um; mobile phase: [water (0.05% $NH_3H_2O$ + 10 mM $NH_4HCO_3$)-ACN]; B%: 45-75%, 30 min) and then subjected to chiral column separation (column: REGIS (R,R) WHELK-O1 (250 mm * 25 mm, 10 um); mobile phase: 0.1% $NH_3H_2O$ ETOH; B%: 30-30%, 0 min) to obtain (6S,8R)-6-(5-((1-(3-fluoropropane)azetidin-3-yl)amino)pyridin-2-yl)-8-methyl-7-(2,2,2 -trifluoroethyl)-6,7,8,9-tetrahydroxazolo[5,4-f]isoquinolin-2(3H)-one as a yellow solid (27.0 mg, 54.2 umol, yield 35.6%, purity 97.7%).

**LCMS:** m/z 494.3 $[M+H]^+$.

**$^1$H NMR** (400 MHz, $CD_3OD$) δ = 7.76 - 7.71 (m, 1H), 7.13 (d, *J* = 8.4 Hz, 1H), 6.93 (m, 1H), 6.80 (d, *J* = 8.0 Hz, 1H),

6.56 (d, $J$ = 8.0 Hz, 1H), 4.52 (t, $J$ = 6.0 Hz, 1H), 4.40 (t, $J$ = 6.0 Hz, 1H), 4.13 - 4.06 (m, 1H), 3.82 - 3.76 (m, 2H), 3.54 - 3.47 (m, 1H), 3.15 - 3.08 (m, 1H), 3.00 - 2.91 (m, 3H), 2.76 (m, 1H), 2.64 (t, $J$ = 7.6 Hz, 2H), 1.83 - 1.69 (m, 2H), 1.06 (d, $J$ = 6.6 Hz, 3H) ppm.

**Example 27: (6S,8R)-6-(3-fluoro-5-((1-(3-fluoropropyl)azetidin-3-yl)amino)pyridin-2-yl)-8-meth yl-7-(2,2,2-trifluoromethyl)-6,7,8,9-tetrahydroxazolo[5,4-f]isoquinolin-2(3H)-one (Compound 27)**

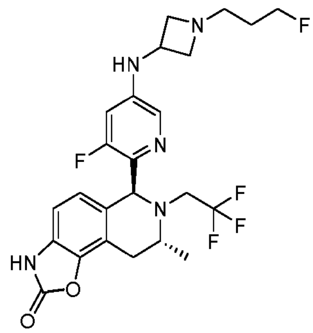

Step I: Synthesis of (6S,8R)-6-(5-bromo-3-fluoropyridin-2-yl)-8-methyl-7-(2,2,2-trifluoroethyl)-6,7,8,9-tetr ahydroxazolo [5,4-f]isoquinolin-2(3H)-one

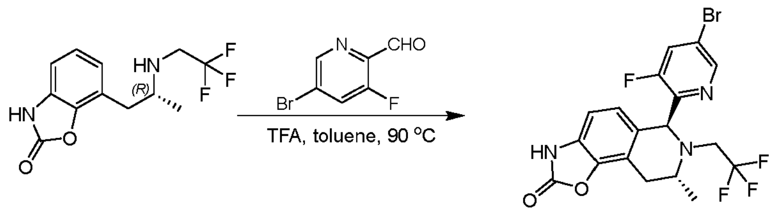


Compound (R)-7-(2-(2,2,2-trifluoroethyl)amino)propyl)benzo[d]oxazol-2(3H)-one (500 mg, 1.82 mmol), 5-bromo-3-fluoro-pyridine-2-formaldehyde (390 mg, 1.91 mmol) and trifluoroacetic acid (1.04 g, 9.12 mmol) were dissolved in toluene (10 mL) and reacted under nitrogen protection at 90°C for 12 h. LCMS detected the completion of the reaction. The reaction solution was concentrated, then adjusted to pH = 7-8, and extracted with water and ethyl acetate, and the organic phase was washed with a saturated sodium chloride aqueous solution, dried and concentrated, and then purified by column chromatography to obtain (6S,8R)-6-(5-bromo-3-fluoropyridin-2-yl)-8-methyl-7-(2,2,2-trifluoroethyl)-6,7,8,9-tetr ahydroxazolo[5,4-f]isoquinolin-2(3H)-one as a yellow solid (605 mg, 1.22 mmol, yield 66.8%).

**LCMS:** m/z 461.8 $[M+H]^+$.

**$^1$H NMR** (400 MHz, CHLOROFORM-d) δ = 8.20 - 8.50 (m, 2 H) 7.61 (dd, J=9.01, 1.88 Hz, 1 H) 6.80 (d, J=8.00 Hz, 1 H) 6.65 (d, J=8.00 Hz, 1 H) 5.37 (s, 1 H) 3.56 - 3.68 (m, 1 H) 3.23 - 3.31 (m, 1 H) 2.88 - 3.13 (m, 2 H) 2.67 (dd, J=17.13, 8.25 Hz, 1 H) 1.07 - 1.18 (m, 3 H) ppm.

Step II: Synthesis of (6S,8R)-6-(5-bromo-3-fluoropyridin-2-yl)-8-methyl-7-(2,2,2-trifluoroethyl)-3-triphenyl methyl-6,7,8,9-tetrahydroxazolo[5,4-f]isoquinolin-2(3H)-one

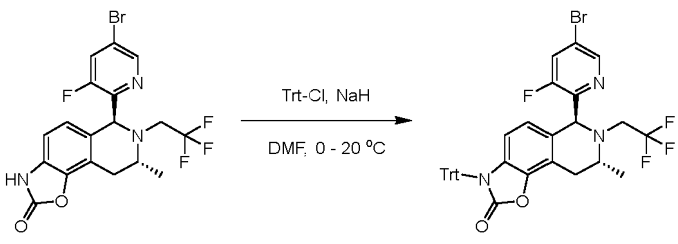

(6S,8R)-6-(5-bromo-3-fluoropyridin-2-yl)-8-methyl-7-(2,2,2-trifluoroethyl)-6,7,8, 9-tetrahydroxazolo[5,4-f]isoquinolin-2(3H)-one (600 mg, 1.21 mmol) was dissolved in N,N-dimethylformamide (12 mL), sodium hydride (58 mg, 1.45 mmol) was added under nitrogen protection at 0°C and stirred for 30 min, triphenylmethyl chloride (336 mg, 1.21 mmol) was then added, and the reaction solution was reacted at 20°C for 30 min. LCMS detected the completion of the reaction. The reaction solution was quenched by adding a saturated ammonium chloride aqueous solution and extracted with ethyl acetate, and the organic phase was washed with a saturated sodium chloride aqueous solution, dried and concentrated. The crude product was purified by column chromatography to obtain (6S,8R)-6-(5-bromo-3-fluoropyridin-2-yl)-8-methyl-7-(2,2,2-trifluoroethyl)-3-triphenyl methyl-6,7,8,9-tetrahydroxazolo[5,4-f]isoquinolin-2(3H)-one as a white solid (754 mg, 991 umol, yield 82.1%).

**LCMS:** m/z 704 $[M+H]^+$.

**$^1$H NMR** (400 MHz, CHLOROFORM-d) δ = 8.27 (d, *J*=1.63 Hz, 1 H), 7.49 (dd, *J*=9.01, 1.88 Hz, 1 H), 7.37 - 7.43 (m, 6 H), 7.18 - 7.22 (m, 9 H), 6.21 (d, *J*=8.50 Hz, 1 H), 5.67 (d, *J*=8.38 Hz, 1 H), 5.19 (s, 1 H), 3.48 - 3.69 (m, 1 H), 3.06 - 3.19 (m, 1 H), 2.80 - 2.97 (m, 2 H), 2.54 (dd, *J*=17.13, 8.25 Hz, 1 H), 1.02 (d, *J*=6.75 Hz, 3 H) ppm.

Step III: Synthesis of (6S,8R)-6-(3-fluoro-5-((1-(3-fluoropropyl)azetidin-3-yl)amino)pyridin-2-yl)-8-methyl-7-(2,2,2-trifluoroethyl)-3-triphenylmethyl-6,7,8,9-tetrahydroxazolo[5,4-f]isoquinolin-2( 3H)-one

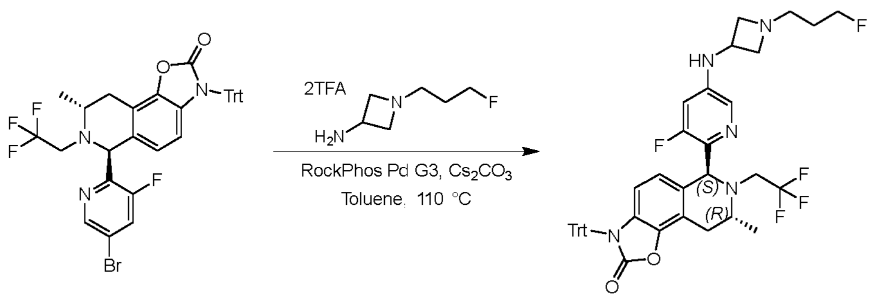


(6S,8R)-6-(5-bromo-3-fluoropyridin-2-yl)-8-methyl-7-(2,2,2-trifluoroethyl)-3-trip henylmethyl-6,7,8,9-tetrahydroxazolo[5,4-f]isoquinolin-2(3H)-one (400 mg, 526 umol), 1-(3-fluoropropyl)aminoazetidine (379 mg, 1.05 mmol), cesium carbonate (685 mg, 2.10 mmol) and [(2-bis-tert-butylphosphine-3-methoxy-6-methyl-2,4,6-triisopropyl-1,1-biphenyl)-2-(2-a minobiphenyl)]palladium(II) methanesulfonate (44.1 mg, 52.6 umol) were dissolved in toluene (10 mL), and the mixture was reacted under nitrogen protection at 110°C for 12 h. LCMS monitored the completion of the reaction. The reaction product was concentrated and purified by column chromatography to obtain (6S,8R)-6-(3-fluoro-5-((1-(3-fluoropropyl)azetidin-3-yl)amino)pyridin-2-yl)-8-methyl-7-(2,2,2-trifluoroethyl)-3-triphenylmethyl-6,7,8,9-tetrahydroxazolo[5,4-f]isoquinolin-2( 3H)-one as a light yellow solid (163 mg, 188 umol, yield 35.8%).

**LCMS:** m/z 754.1 $[M+H]^+$.

**$^1$H NMR** (400 MHz, CHLOROFORM-d) δ = 7.57 (d, *J*=1.88 Hz, 1 H), 7.36 - 7.41 (m, 6 H), 7.21 (s, 9 H), 7.16 (s, 1 H), 6.41 (dd, *J*=11.57, 2.31 Hz, 1 H), 6.23 (d, *J*=8.50 Hz, 1 H) ,5.64 (d, *J*=8.50 Hz, 1 H), 5.12 (s, 1 H), 4.31 - 4.53 (m, 2 H), 4.09 - 4.19 (m, 1 H), 3.93 - 4.00 (m, 1 H), 3.62 - 3.68 (m, 2 H), 3.00 - 3.14 (m, 1 H), 2.78 - 2.98 (m, 4 H), 2.46 - 2.60 (m, 3 H), 1.65 (s, 2 H), 1.02 (d, *J*=6.63 Hz, 3 H) ppm.

Step IV: Synthesis of (6S,8R)-6-(3-fluoro-5-((1-(3-fluoropropyl)azetidin-3-yl)amino)pyridin-2-yl)-8-methyl-7-(2,2,2-trifluoromethyl)-6,7,8,9-tetrahydroxazolo[5,4-f]isoquinolin-2(3H)-one

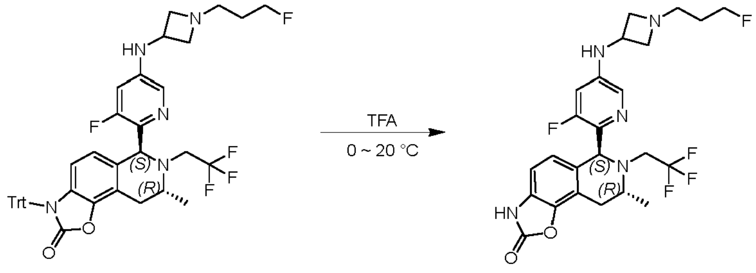

Trifluoroacetic acid (2.02 g, 17.6 mmol) was dissolved in water (0.05 mL), (6S,8R)-6-(3-fluoro-5-((1-(3-fluoropropyl)azetidin-3-yl)amino)pyridin-2-yl)-8-methyl-7-(2,2,2-trifluoroethyl)-3-triphenylmethyl-6,7,8,9-tetrahydroxazolo [5,4-f]isoquinolin-2( 3H)-one (120 mg, 138 umol) was then added, and the mixture was reacted under nitrogen protection at 20°C for 5 h. LCMS detected the completion of the reaction. The mixture was concentrated, then adjusted to pH = 7-8, and extracted with water and ethyl acetate, and the organic phase was washed with a saturated sodium chloride aqueous solution, dried with sodium sulfate and concentrated. The crude product was subjected to prep-HPLC chromatography to obtain (6S,8R)-6-(3-fluoro-5-((1-(3-fluoropropyl)azetidin-3-yl)amino)pyridin-2-yl)-8-methyl-7-(2,2,2-trifluoromethyl)-6,7,8,9-tetrahydroxazolo[5,4-f]isoquinolin-2(3H)-one as a yellow solid (70 mg, 124.27 umol).

**LCMS:** m/z 511.2 [M+H]+.

Step V: Synthesis of (6S,8R)-6-(3-fluoro-5-((1-(3-fluoropropyl)azetidin-3-yl)amino)pyridin-2-yl)-8-methyl-7-(2,2,2-trifluoromethyl)-6,7,8,9-tetrahydroxazolo[5,4-f]isoquinolin-2(3H)-one

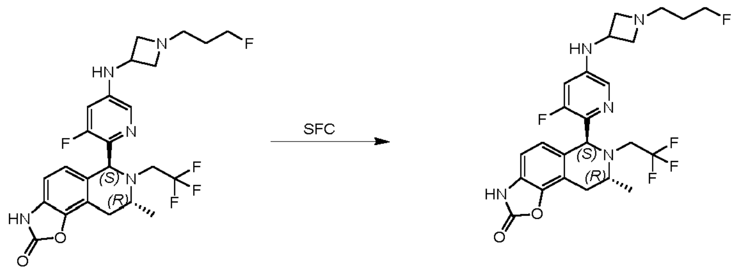


(6S,8R)-6-(3-fluoro-5-((1-(3-fluoropropyl)azetidin-3-yl)amino)pyridin-2-yl)-8-met hyl-7-(2,2,2-trifluoromethyl)-6,7,8,9-tetrahydroxazolo[5,4-f]isoquinolin-2(3H)-one was subjected to chiral resolution to obtain (6S,8R)-6-(3-fluoro-5-((1-(3-fluoropropyl)azetidin-3-yl)amino)pyridin-2-yl)-8-methyl-7-(2,2,2-trifluoromethyl)-6,7,8,9-tetrahydroxazolo[5,4-f]isoquinolin-2(3H)-one (42.7 mg, 83.5 umol, yield 61.0%).

**$^1$H NMR** (400 MHz, ACETONITRILE-d3) $\delta$ = 7.56 - 7.59 (m, 1 H), 6.82 (d, J=8.00 Hz, 1 H), 6.61 - 6.67 (m, 2 H), 5.28 (s, 1 H), 5.14 - 5.19 (m, 1 H), 4.36 - 4.54 (m, 2 H), 3.93 - 4.02 (m, 1 H), 3.57 - 3.71 (m, 3 H), 3.27 - 3.40 (m, 1 H), 2.83 - 3.01 (m, 2 H), 2.76 - 2.82 (m, 2 H), 2.63 (d, J=9.01 Hz, 1 H), 2.50 (t, J=7.00 Hz, 2 H), 1.61 - 1.76 (m, 2 H), 1.06 (d, J=6.75 Hz, 3 H) ppm.

**Example 28: (6S,8R)-6-(5-((1-(3-fluoropropyl)azetidin-3-yl)amino)-3-methoxypyridin-2-yl)-8-m ethyl-7-(2,2,2-trifluoroethyl)-6,7,8,9-tetrahydroxazolo[5,4-f]isoquinolin-2(3H)-one (Compound 28)**

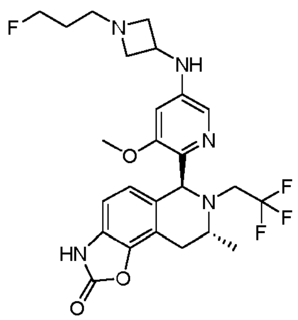

Step I: Synthesis of (6S,8R)-6-(5-bromo-3-methoxypyridin-2-yl)-8-methyl-7-(2,2,2-trifluoroethyl)-6,7,8,9-t etrahydroxazolo[5,4-f]isoquinolin-2(3H)-one

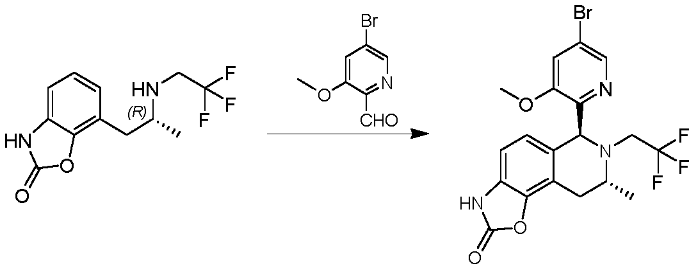


(R)-7-(2-((2,2,2-trifluoroethyl)amino)propyl)benzo[d]oxazol-2(3H)-one (500 mg, 1.82 mmol) and 5-bromo-3-methoxypyridinecarboxaldehyde (400 mg, 1.85 mmol) were dissolved in toluene (10 mL), and trifluoroacetic acid (1.04 g, 9.12 mmol) was then added. The mixture was reacted at 90°C for 12 h. LCMS detected the completion of the reaction. The reaction solution was concentrated and then purified by a silica gel column to obtain (6S,8R)-6-(5-bromo-3-methoxypyridin-2-yl)-8-methyl-7-(2,2,2-trifluoroethyl)-6,7,8,9-t etrahydroxazolo[5,4-f]isoquinolin-2(3H)-one (750 mg, 1.59 mmol, yield 87.1%).

Step II: Synthesis of (6S,8R)-6-(5-bromo-3-methoxypyridin-2-yl)-8-methyl-7-(2,2,2-trifluoroethyl)-3-triphe nylmethyl-6,7,8,9-tetrahydroxazolo[5,4-f]isoquinolin-2(3H)-one

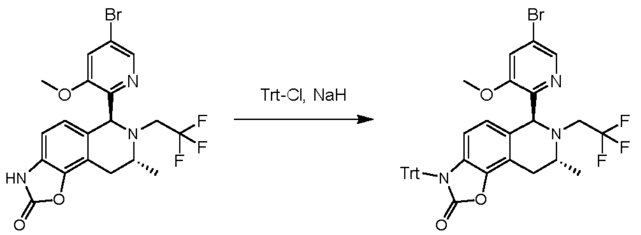


(6S,8R)-6-(5-bromo-3-methoxypyridin-2-yl)-8-methyl-7-(2,2,2-trifluoroethyl)-6,7, 8,9-tetrahydroxazolo[5,4-f]isoquinolin-2(3H)-one (700 mg, 1.48 mmol) was dissolved in N,N-dimethylformamide (14 mL). Sodium hydride (71.1 mg, 1.78 mmol, 60% purity) was added at 0°C. After stirring for 30 min, triphenylmethyl chloride (371 mg, 1.33 mmol) was added and the mixture was heated to 20°C and reacted for 2 h. LCMS detected the completion of the reaction. The reaction solution was extracted with ice water and extracted with ethyl acetate. The organic phase was washed three times with a saturated sodium chloride aqueous solution, concentrated and then purified by a silica gel column to obtain (6S,8R)-6-(5-bromo-3-methoxypyridin-2-yl)-8-methyl-7-(2,2,2-trifluoroethyl)-3-triphe nylmethyl-6,7,8,9-tetrahydroxazolo[5,4-f]isoquinolin-2(3H)-one (750 mg, 1.05 mmol, yield 70.8%).

**$^1$H NMR** (400 MHz, CHLOROFORM-d) $\delta$ = 8.12 (d, *J* = 1.9 Hz, 1H), 7.54 - 7.40 (m, 6H), 7.32 - 7.26 (m, 9H), 6.28 (d, *J* = 8.5 Hz, 1H), 5.73 (d, *J* = 8.4 Hz, 1H), 5.53 - 5.31 (m, 1H), 3.82 (s, 3H), 3.77 - 3.69 (m, 1H), 3.25 - 3.09 (m , 1H), 3.02 - 2.83 (m, 2H), 2.64 - 2.50 (m,1H), 1.10 (d, *J* = 6.6 Hz, 3H) ppm.

Step III: Synthesis of (6S,8R)-6-(5-((1-(3-fluoropropyl)azetidin-3-yl)amino)-3-methoxypyridin-2-yl)-8-methy l-7-(2,2,2-trifluoroethyl)-3-triphenylmethyl-6,7,8,9-tetrahydroxazolo[5,4-f]isoquinolin-2(3H)-one

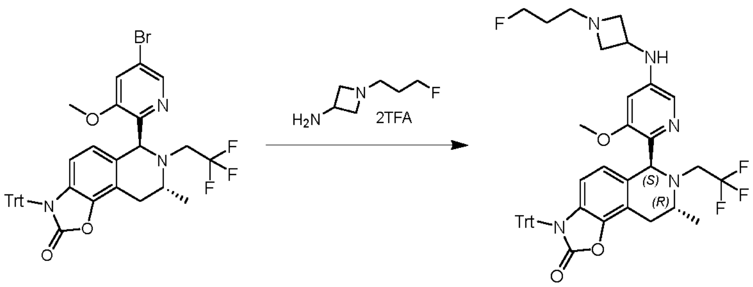


(6S,8R)-6-(5-bromo-3-methoxypyridin-2-yl)-8-methyl-7-(2,2,2-trifluoroethyl)-3-tr iphenylmethyl-6,7,8,9-tetrahydroxazolo[5,4-f]isoquinolin-2(3H)-one (400 mg, 560 umol), 1-(3-fluoropropyl)azetidine-3-amine (403 mg, 1.12 mmol), [(2-bis-tert-butylphosphine-3-methoxy-6-methyl-2,4,6-triisopropyl-1,1-biphenyl)-2-(2-a minobiphenyl)]palladium(II) methanesulfonate (46.9 mg, 56.0 umol) and cesium carbonate (730 mg, 2.24 mmol) were dissolved in toluene (2 mL). The mixture was reacted at 110°C under nitrogen protection for 12 h. LCMS detected the completion of the reaction. The reaction solution was concentrated and then purified by a silica gel column to obtain (6S,8R)-6-(5-((1-(3-fluoropropyl) azetidin-3-yl)amino)-3-methoxypyridin-2-yl)-8-methy l-7-(2,2,2-trifluoroethyl)-3-triphenylmethyl-6,7,8,9-tetrahydroxazolo[5,4-f]isoquinolin-2(3H)-one (160 mg, 209 umol, yield 33.2%).

Step IV: Synthesis of (6S,8R)-6-(5-((1-(3-fluoropropyl)azetidin-3-yl)amino)-3-methoxypyridin-2-yl)-8-methy l-7-(2,2,2-trifluoroethyl)-6,7,8,9-tetrahydroxazolo[5,4-f]isoquinolin-2(3H)-one

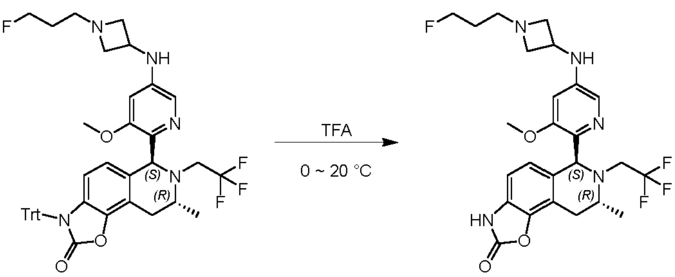


(6S,8R)-6-(5-((1-(3-fluoropropyl)azetidin-3-yl)amino)-3-methoxypyridin-2-yl)-8-methyl-7-(2,2,2-trifluoroethyl)-3-triphenyl-6,7,8,9-tetrahydroxazolo[5,4-f]isoquinolin-2 (3H)-one (160 mg, 208 umol) was dissolved in water (0.5 mL) and trifluoroacetic acid (4.5 mL). After 2 hours of reaction at 20°C, LCMS detected the completion of the reaction. The reaction product was adjusted to pH = 7-8 with sodium bicarbonate and extracted with ethyl acetate, and the organic phase was washed three times with brine, concentrated and then purified by a silica gel column to obtain (6S,8R)-6-(5-((1-(3-fluoropropyl)azetidin-3-yl)amino)-3-methoxypyridin-2-yl)-8-methy l-7-(2,2,2-trifluoroethyl)-6,7,8,9-tetrahydroxazolo[5,4-f]isoquinolin-2(3H)-one (70 mg, 134 umol, yield 64.0%).

**$^1$H NMR** (400 MHz, ACETONITRILE-$d_3$) $\delta$ = 7.32 (d, *J* = 2.3 Hz, 1H), 6.80 (d, *J* = 8.0 Hz, 1H), 6.63 - 6.51 (m, 2H), 5.46 (s, 1H), 4.89 (d, *J* = 7.0 Hz, 1H), 4.54 (t, *J*= 6.1 Hz, 1H), 4.42 (t, *J* = 6.1 Hz, 1H), 4.20 - 3.96 (m, 1H), 3.82 (s, 4H), 3.74 - 3.65 (m, 2H), 3.40 - 3.26 (m, 1H), 3.02 - 2.84 (m, 2H), 2.82 - 2.74 (m, 2H), 2.64 - 2.56 (m, 1H), 2.52 (t, *J*= 6.9 Hz, 2H), 1.77 - 1.65 (m, 2H), 1.09 (d, *J* = 6.8 Hz, 3H) ppm.

**Example 29: (6S,8R)-6-(5-(((S)-1-(3-fluoropropane)pyrrolidin-3-yl)oxy)pyridin-2-yl)-8-methyl-7 -(2,2,2-trifluoroethyl)-6,7,8,9-tetrahydrooxazolo[5,4-f]isoquinolin-2(3H)-one (Compound 29)**

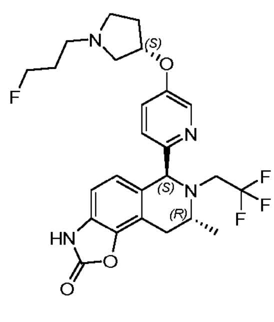

Step I: Synthesis of tert-butyl (S)-3-((6-((6S,8R)-8-methyl-2-oxo-7-(2,2,2-trifluoroethyl)-3-triphenylmethyl-2,3,6,7,8, 9-hexahydrooxazolo[5,4-f]isoquinolin-6-yl)pyridin-3-yl)oxy)pyrrolidine-1-carboxylate

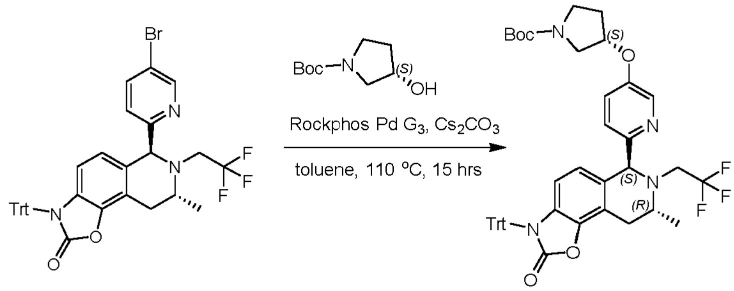


(6S,8R)-6-(5-bromopyridin-2-yl)-8-methyl-7-(2,2,2-trifluoroethyl)-3-triphenylmet hyl-6,7,8,9-tetrahydroxazolo [5,4-f]isoquinolin-2(3H)-one (150 mg, 219 umol, 1 eq) and tert-butyl (S)-3-hydroxypyrrolidine-1-carboxylate (102 mg, 547 umol, 2.5 eq) were dissolved in toluene (20 mL), (18.3 mg, 21.9 umol, 0.1 eq) and cesium carbonate (214 mg, 657 umol, 3 eq) were then added, and the reaction solution was reacted at 115°C for 15 h. TLC monitored the completion of the reaction. The reaction solution was quenched by adding water, extracted with ethyl acetate, dried and concentrated. The crude product was separated by column chromatography to obtain tert-butyl (S)-3-((6-((6S,8R)-8-methyl-2-oxo-7-(2,2,2-trifluoroethyl)-3-triphenylmethyl-2,3,6,7,8, 9-hexahydrooxazolo[5,4-f]isoquinolin-6-yl)pyridin-3-yl)oxy)pyrrolidine-1-carboxylate as a yellow solid (120 mg, 138 umol, 31.5% yield).

**$^1$H NMR** (400 MHz, CHLOROFORM-d) $\delta$ = 8.02 (d, *J* = 2.8 Hz, 1H), 7.39 (d, *J* = 7.2 Hz, 6H), 7.24 - 7.20 (m, 5H), 7.18 - 7.14 (m, 4H), 7.08 - 7.04 (m, 1H), 6.37 - 6.25 (m, 1H), 5.66 (d, *J* = 8.4 Hz, 1H), 4.86 - 4.80 (m, 2H), 4.39 (br s, 1H), 3.55 - 3.34 (m, 2H), 3.34 - 3.23 (m, 1H), 3.16 - 3.04 (m, 1H), 3.01 - 2.89 (m, 1H), 2.83 (br dd, $J_1$ = 8.8 Hz, $J_2$ = 15.6 Hz, 1H), 2.59 (dd, $J_1$ = 6.4 Hz, $J_2$ =16.8 Hz, 1H), 2.15 - 2.01 (m, 2H), 1.95 - 1.81 (m, 2H), 1.40 (s, 9H), 1.02 (d, *J* = 6.4 Hz, 3H) ppm.

Step II: Synthesis of (6S,8R)-8-methyl-6-(5-((S)-pyrrolidin-3-yl)oxy)pyridin-2-yl)-7-(2,2,2-trifluoroethyl)-6, 7,8,9-tetrahydroxazolo[5,4-f]isoquinolin-2(3H)-one

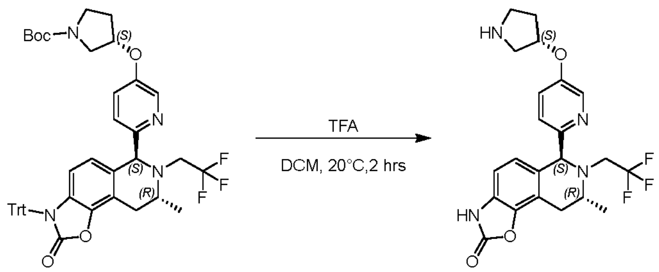


Trifluoroacetic acid (1.54 g, 13.4 mmol, 997 uL, 87.3 eq) was added and dissolved in tert-butyl (S)-3-((6-((6S,8R)-8-methyl-2-oxo-7-(2,2,2-trifluoroethyl)-3-triphenylmethyl-2,3,6,7,8, 9-hexahydrooxazolo[5,4-f]isoquinolin-6-yl)pyridin-3-yl)oxy)pyrrolidine-1-carboxylate (120 mg, 154 $\mu$mol, 1 eq) in dichloromethane (2 mL) at 0°C, and the mixture was reacted at 20°C for 2 h. TLC and LCMS monitored the completion of the reaction. The reaction solution was quenched by adding water, extracted with ethyl acetate, dried and concentrated. The crude product was separated by thin layer chromatography on a silica gel plate to obtain (6S,8R)-8-methyl-6-(5-((S)-pyrrolidin-3-yl)oxy)pyridin-2-yl)-7-(2,2,2-trifluoroethyl)-6, 7,8,9-tetrahydroxazolo[5,4-f]isoquinolin-2(3H)-one as a yellow solid (105 mg, crude product).

**LCMS:** m/z $(M+H)^+$ = 449.2

**$^1$H NMR** (400MHz, DMSO-$d_6$) $\delta$ = 8.13 (d, *J* = 2.8 Hz, 1H), 7.45 - 7.34 (m, 1H), 7.32 - 7.26 (m, 1H), 6.87 - 6.82 (m, 1H), 6.74 - 6.67 (m, 1H), 5.14 - 5.02 (m, 2H), 3.00 - 2.92 (m, 2H), 2.90 - 2.78 (m, 2H), 2.72 - 2.71 (m, 1H), 2.74 - 2.58 (m, 1H), 2.21 - 2.07 (m, 2H), 2.06 - 1.93 (m, 1H), 1.23 (s, 1H), 1.06 (d, *J*= 6.4 Hz, 3H) ppm.

Step III: Synthesis of (6S,8R)-6-(5-(((S)-1-(3-fluoropropane)pyrrolidin-3-yl)oxy)pyridin-2-yl)-8-methyl-7-(2, 2,2-trifluoroethyl)-6,7,8,9-tetrahydroxazolo[5,4-f]isoquinolin-2(3H)-one

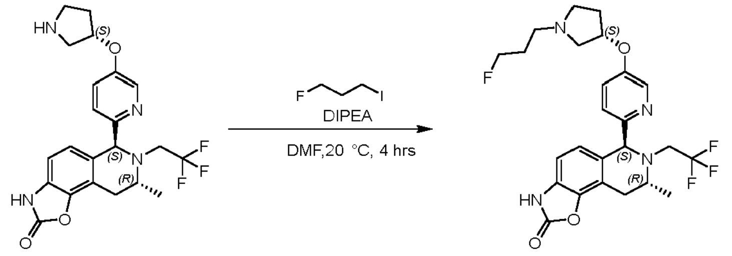


(6S,8R)-8-methyl-6-(5-((S)-pyrrolidin-3-yl)oxy)pyridin-2-yl)-7-(2,2,2-trifluoroeth yl)-6,7,8,9-tetrahydroxazolo [5,4-f]isoquinolin-2(3H)-one (105 mg, 187 umol, 1 eq) and 3-fluoro-1-iodopropane (35.2 mg, 187 umol, 1 eq) were dissolved in N,N-dimethylformamide (2 mL) under nitrogen protection at 20°C, diisopropyl ethylenediamine (121 mg, 936 umol, 163 uL, 5 eq) was added, and the mixture was reacted at 20°C for 4 h. LCMS detected the completion of the reaction. The reaction solution was quenched by adding water, extracted with ethyl acetate, dried and concentrated. The crude product was separated by a reversed-phase column (column: Phenomenex Gemini-NX C18 75 * 30 mm * 3 um; mobile phase: [water (0.04 % $NH_3H_2O$ + 10 mM $NH_4HCO_3$)-ACN]; B%: 18-58%, 14 min) and SFC(column: REGIS (R,R)WHELK-O1(250 mm * 25 mm, 10 um); mobile phase: 0.1% $NH_3H_2O$ ETOH; B%: 30-30%, 10 min) to obtain (6S,8R)-6-(5-(((S)-1-(3-fluoropropane)pyrrolidin-3-yl)oxy)pyridin-2-yl)-8-methyl-7-(2, 2,2-trifluoroethyl)-6,7,8,9-tetrahydroxazolo[5,4-f]isoquinolin-2(3H)-one as a white solid (10.5 mg, 19.8 umol, yield 10.6%, purity 96%).

**LCMS:** m/z $(M+H)^+$ = 509.4

**$^1$H NMR** (400MHz, $CD_3OD$) δ = 8.05 (d, *J* = 2.8 Hz, 1H), 7.40 - 7.30 (m, 2H), 6.83 (d, *J* = 8.0 Hz, 1H), 6.63 (d, *J* = 8.0 Hz, 1H), 4.54 (t, *J*= 6.0 Hz, 1H), 4.42 (t, *J* = 6.0 Hz, 1H), 3.53 - 3.48 (m, 1H), 3.40 (br d, *J* = 6.0 Hz, 1H), 3.10 (dd, $J_1$ = 4.8 Hz, $J_2$ =16.8 Hz, 1H), 3.02 - 2.87 (m, 4H), 2.78 (dd, $J_1$ = 6.2 Hz, $J_2$ =16.8 Hz, 1H), 2.68 - 2.58 (m, 2H), 2.56 - 2.49 (m, 1H), 2.38 (dt, $J_1$ = 7.6 Hz, $J_2$ =13.6 Hz, 1H), 1.99 - 1.84 (m, 3H), 1.29 (br s, 1H), 1.12 (d, *J* = 6.4 Hz, 3H) ppm.

**Example 30: (6S,8R)-6-(4-(((S)-1-(3-fluoropropyl)pyrrolidin-3-yl)oxy)-2-methoxyphenyl)-8-met hyl-7-(2,2,2-trifluoroethyl)-6,7,8,9-tetrahydroxazolo[5,4-f]isoquinolin-2(3H)-one (Compound 30)**

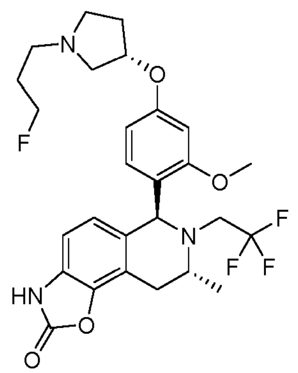

Step I: Synthesis of tert-butyl (S)-3-(3-methoxy-4-((6S,8R)-8-methyl-2-oxo-7-(2,2,2-trifluoroethyl)-3-triphenylmethy l-2,3,6,7,8,9-hexahydrooxazolo[5,4-f]isoquinolin-6-yl)phenoxy)pyrrolidine-1-carboxyla te

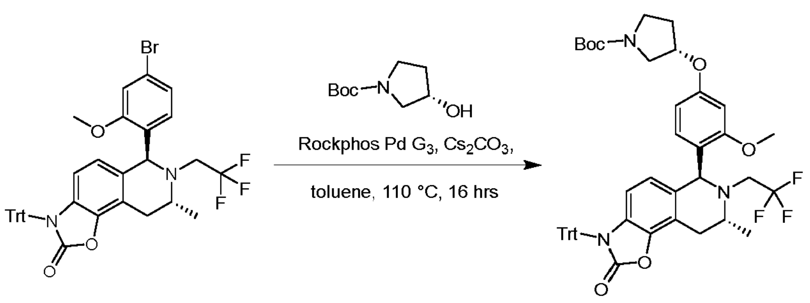

(6S,8R)-6-(4-bromo-2-methoxyphenyl)-8-methyl-7-(2,2,2-trifluoroethyl)-3-triphen ylmethyl-6,7,8,9-tetrahydroxazolo[5,4-f]isoquinolin-2(3H)-one (500 mg, 700 umol), tert-butyl (S)-3-hydroxypyrrolidine-1-carboxylate (236 mg, 1.26 mmol), cesium carbonate (685 mg, 2.10 mmol), and [(2-bis-tert-butylphosphine-3-methoxy-6-methyl-2,4,6-triisopropyl-1,1-biphenyl)-2-(2-a minobiphenyl)]palladium(II) methanesulfonate (23.5 mg, 28.0 umol) were dissolved in toluene (8 mL), and the mixture was reacted under nitrogen protection at 110°C for 16 h. Two reactions were carried out in parallel. LCMS monitored the completion of the reaction. The reaction products were combined, concentrated and purified by column chromatography to obtain tert-butyl (S)-3-(3-methoxy-4-((6S,8R)-8-methyl-2-oxo-7-(2,2,2-trifluoroethyl)-3-triphenylmethy l-2,3,6,7,8,9-hexahydrooxazolo[5,4-f]isoquinolin-6-yl)phenoxy)pyrrolidine-1-carboxyla te as a yellow solid (180 mg, yield15.7%).

**$^1$H NMR** (400 MHz, CHLOROFORM-d) δ = 7.51 - 7.43 (m, 6H), 7.33 - 7.28 (m, 4H), 7.27 - 7.19 (m, 5H), 6.90 - 6.73 (m, 1H), 6.43 (br s, 1H), 6.36 - 6.19 (m, 2H), 5.68 (br d, *J= 8.5* Hz, 1H), 5.29 (s, 1H), 4.85 (br s, 1H), 3.85 - 3.75 (m, 3H), 3.71 - 3.43 (m, 5H), 3.14 - 2.96 (m, 2H), 2.85 (qd, *J* = 9.5, 15.4 Hz, 1H), 2.66 (br dd, *J* = 6.1, 16.9 Hz, 1H), 2.16 (br s, 2H), 1.48 (s, 9H), 1.07 (br d, *J* = 6.5 Hz, 3H) ppm.

Step II: Synthesis of (6S,8R)-6-(2-methoxy-4-((S)-pyrrolidin-3-yloxy)phenyl)-8-methyl-7-(2,2,2-trifluoroeth yl)-6,7,8,9-tetrahydroxazolo[5,4-f]isoquinolin-2(3H)-one

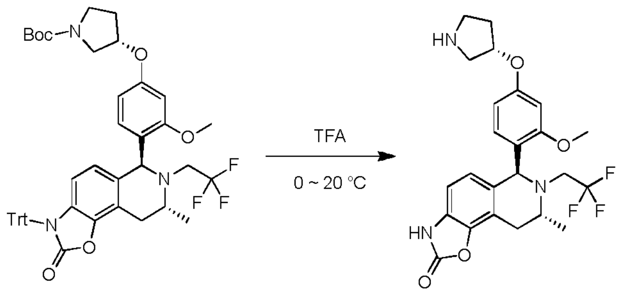


Tert-butyl (S)-3-(3-methoxy-4-((6S,8R)-8-methyl-2-oxo-7-(2,2,2-trifluoroethyl)-3-triphenylmethy l-2,3,6,7,8,9-hexahydrooxazolo[5,4-f]isoquinolin-6-yl)phenoxy)pyrrolidine-1-carboxyla te (180 mg, 220 umol) was dissolved in trifluoroacetic acid (2 ml) and water (0.2 mL), and the mixture was reacted at 20°C for 2 h. LCMS detected the completion of the reaction. The mixture was concentrated, then adjusted to pH = 7-8, and extracted with water and ethyl acetate, and the organic phase was washed with a saturated sodium chloride aqueous solution, dried with sodium sulfate and concentrated. The product was separated by column chromatography to obtain (6S,8R)-6-(2-methoxy-4-((S)-pyrrolidin-3-yloxy) phenyl)-8-methyl-7-(2,2,2-trifluoroeth yl)-6,7,8,9-tetrahydroxazolo[5,4-f]isoquinolin-2(3H)-one as a yellow solid (110 mg, yield 77.7%).

**$^1$H NMR** (400 MHz, CHLOROFORM-d) δ = 6.73 (dd, *J* = 8.3, 12.6 Hz, 2H), 6.54 (d, *J* = 8.1 Hz, 1H), 6.45 (d, *J* = 2.4 Hz, 1H), 6.24 (dd, *J* = 2.3, 8.6 Hz, 1H), 5.35 (s, 1H), 3.84 (s, 3H), 3.50 - 3.35 (m, 5H), 3.16 - 3.01 (m, 3H), 2.90 - 2.78 (m, 1H), 2.66 (br dd, *J* = 6.5, 16.9 Hz, 1H), 2.37 - 2.27 (m, 1H), 2.26 - 2.15 (m, 1H), 1.06 (d, *J* = 6.6 Hz, 3H) ppm.

Step III: Synthesis of (6S,8R)-6-(4-(((S)-1-(3-fluoropropyl)pyrrolidin-3-yl)oxy)-2-methoxyphenyl)-8-methyl-7-(2,2,2-trifluoroethyl)-6,7,8,9-tetrahydroxazolo[5,4-f]isoquinolin-2(3H)-one

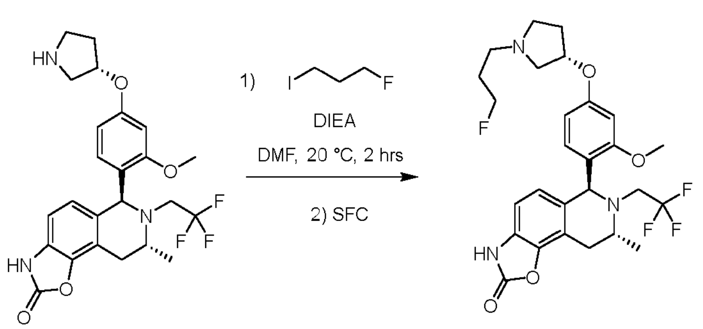

(6S,8R)-6-(2-methoxy-4-((S)-pyrrolidin-3-yloxy)phenyl)-8-methyl-7-(2,2,2-trifluo roethyl)-6,7,8,9-tetrahydroxazolo[5,4-f]isoquinolin-2(3H)-one (110 mg, 170 umol) and diisopropylethylamine (66.1 mg, 511 umol) was dissolved in N,N-dimethylformamide (3 mL), 3-fluoro-1-iodopropane (25.6 mg, 136 umol) was added at 20°C, and the mixture was reacted for 3 h. TLC monitored the completion of the reaction. The reaction product was separated by HPLC to obtain 60 mg of a crude product and then subjected to SFC chiral separation to obtain (6S,8R)-6-(4-(((S)-1-(3-fluoropropyl)pyrrolidin-3-yl)oxy)-2-methoxyphenyl)-8-methyl-7-(2,2,2-trifluoroethyl)-6,7,8,9-tetrahydroxazolo[5,4-f]isoquinolin-2(3H)-one as a yellow solid (47.3 mg, yield 51.4%).

**$^1$H NMR** (400 MHz, CHLOROFORM-d) δ = 8.62 - 8.02 (m, 1H), 6.81 - 6.68 (m, 2H), 6.59 (d, *J* = 8.1 Hz, 1H), 6.45 (d, *J* = 2.4 Hz, 1H), 6.25 (dd, *J* = 2.4, 8.5 Hz, 1H), 5.38 (s, 1H), 4.81 (br s, 1H), 4.61 - 4.43 (m, 2H), 3.84 (s, 3H), 3.61 - 3.51 (m, 1H), 3.16 - 3.06 (m, 2H), 2.97 - 2.79 (m, 4H), 2.76 - 2.62 (m, 3H), 2.31 (br dd, *J* = 6.8, 13.8 Hz, 1H), 2.05 - 1.88 (m, 3H), 1.30 - 1.23 (m, 1H), 1.10 (d, *J* = 6.6 Hz, 3H) ppm.

**Example 31: (6S,8R)-7-((1-fluorocyclopropyl)methyl)-6-(4-((1-(3-fluoropropyl)azetidin-3-yl))am ino)-2-methoxyphenyl)-8-methyl-6,7,8,9-tetrahydroxazolo[5,4-f]isoquinolin-2(3H)-one**

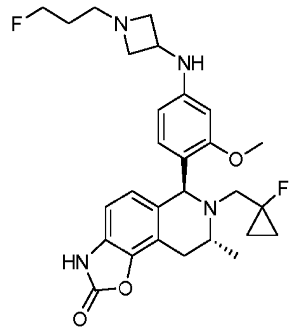

Step I: Synthesis of (6S,8R)-6-(4-bromo-2-methoxyphenyl)-7-((1-fluorocyclopropyl)methyl)-8-methyl-6,7, 8,9-tetrahydroxazolo[5,4-f]isoquinolin-2(3H)-one

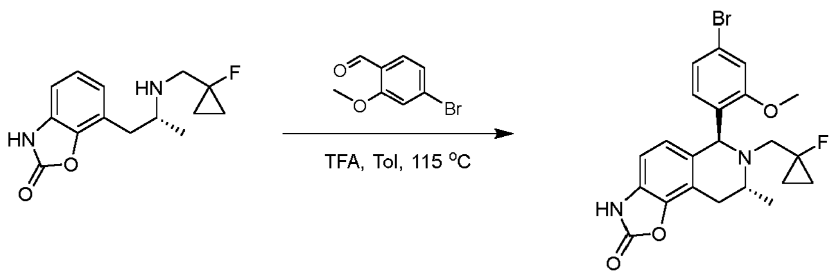


(R)-7-(2-(((1-fluorocyclopropyl)methyl)amino)propyl)benzo[d]oxazol-2(3H)-one (1.05 g, 3.97 mmol), 4-bromo-2-methoxybenzaldehyde (1.54 g, 7.15 mmol) and trifluoroacetic acid (2.26 g, 19.8 mmol) were dissolved in toluene (8 mL), and the mixture was reacted at 110°C for 120 h. LCMS monitored the completion of the reaction. The reaction solution was concentrated and purified by column chromatography to obtain (6S,8R)-6-(4-bromo-2-methoxyphenyl)-7-((1-fluorocyclopropyl)methyl)-8-methyl-6,7, 8,9-tetrahydroxazolo[5,4-f]isoquinolin-2(3H)-one as a yellow solid (249 mg, 539 umol, yield 13.5%).

**LCMS:** m/z 462.8 $[M+H]^+$.

Step II: Synthesis of (6S,8R)-7-((1-fluorocyclopropyl)methyl)-6-(4-((1-(3-fluoropropyl)azetidin-3-yl))amino )-2-methoxyphenyl)-8-methyl-6,7,8,9-tetrahydroxazolo[5,4-f]isoquinolin-2(3H)-one

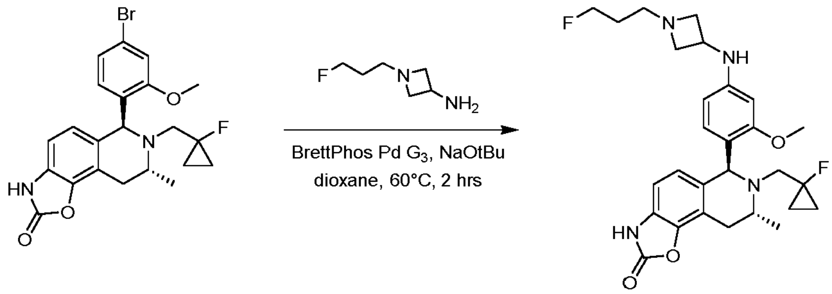


((6S,8R)-6-(4-bromo-2-methoxyphenyl)-7-((1-fluorocyclopropyl)methyl)-8-methy 1-6,7,8,9-tetrahydroxazolo [5,4-f]isoquinolin-2(3H)-one (140 mg, 303 umol), 1-(3-fluoropropyl)aminoazetidine (112 mg, 849 umol), (2-dicyclohexylphosphine)-3,6-dimethoxy-2',4',6'-triisopropyl-1,1'-biphenyl)(2'-amino -1,1'-biphenyl-2-yl)palladium(II) methanesulfonate (27.5 mg, 30.3 umol) and sodium tert-butoxide (87.4 mg, 910 umol) were dissolved in dioxane (1 mL) and reacted at 60°C for 2 h. LCMS monitored the completion of the reaction. The reaction solution was filtered and then purified by prep-HPLC, and the product was then purified by chiral SFC to obtain (6S,8R)-7-((1-fluorocyclopropyl)methyl)-6-(4-((1-(3-fluoropropyl)azetidin-3-yl))amino )-2-methoxyphenyl)-8-methyl-6,7,8,9-tetrahydroxazolo[5,4-f]isoquinolin-2(3H)-one as a yellow solid (7.2 mg, yield 4.49%).

**LCMS:** m/z 513.2 $[M+H]^+$.

**$^1$H NMR** (400 MHz, ACETONITRILE-$d_3$) δ = 6.75 (d, *J*=8.13 Hz, 1 H), 6.67 (d, *J*=8.38 Hz, 1 H), 6.54 (d, *J*=8.13 Hz, 1 H), 6.18 (d, *J*=2.00 Hz, 1 H), 6.00 (dd, *J*=8.38, 2.00 Hz, 1 H), 5.20 (s, 1 H), 4.69 (m, *J*=6.90 Hz, 1 H), 4.52 (t, *J*=6.07 Hz, 1 H), 4.40 (t, *J*=6.07 Hz, 1 H), 3.95 - 4.04 (m, 1 H), 3.80 (s, 3 H), 3.64 - 3.72 (m, 3 H), 3.03 (dd, *J*=16.26, 4.88 Hz, 1 H), 2.86 - 2.97 (m, 1 H), 2.68 - 2.78 (m, 3 H), 2.56 - 2.65 (m, 1 H), 2.50 (t, *J*=7.00 Hz, 2 H), 1.64 - 1.74 (m, 2 H), 0.99 (d, *J*=6.63 Hz, 3 H), 0.86 - 0.94 (m, 2 H), 0.46 - 0.58 (m, 2 H) ppm.

Similarly to the synthesis route of Example 25 above, the following Examples 32, 33 and 34 were synthesized by selecting corresponding reactants and synthesis methods known to those skilled in the art.

**Example 32: (6S,8R)-7-(2,2-difluoropropyl)-6-(4-((1-(3-fluoropropyl)azetidin-3-yl)-2-methoxyph enyl)-8-methyl-6,7,8,9-tetrahydroxazolo[5,4-f]isoquinolin-2(3H)-one**

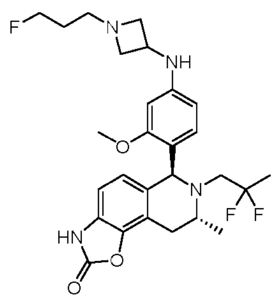

**LCMS:** m/z 519.2 $[M+H]^+$.

**$^1$H NMR** (400 MHz, ACETONITRILE-$d_3$) δ = 6.75 (d, *J* = 8.1 Hz, 1H), 6.48 (dd, *J* = 8.2, 19.9 Hz, 2H), 6.18 (d, *J* = 2.1 Hz, 1H), 5.97 (dd, *J* = 2.1, 8.4 Hz, 1H), 5.22 (s, 1H), 4.73 (br s, 1H), 4.55 - 4.37 (m, 2H), 4.02 - 3.95 (m, 1H), 3.79 (s, 3H), 3.69 - 3.64 (m, 2H), 3.54 - 3.38 (m, 2H), 2.96 - 2.85 (m, 2H), 2.74 (q, *J* = 6.4 Hz, 2H), 2.65 - 2.55 (m, 2H), 2.49 (t, *J* = 7.0 Hz, 2H), 2.21 - 2.11 (m, 1H), 1.75 - 1.62 (m, 2H), 1.54 (t, *J* = 19.2 Hz, 3H), 1.00 (d, *J* = 6.6 Hz, 3H) ppm.

**Example 33: (6S,8R)-6-(4-((1-(3-fluoropropyl)azetidin-3-yl)amino)-2-methoxy-6-chloro-phenyl)-8-methyl-7-(2,2,2-trifluoroethyl)-6,7,8,9-6,7,8,9-tetrahydrooxazolo[5,4-f]isoquinoli n-2(3H)-one (Compound 33)**

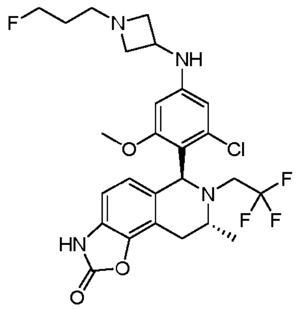

**LCMS:** m/z 557.4 [M+H]+.

**$^1$H NMR** (400 MHz, ACETONITRILE-$d_3$) δ = 6.73 (br d, *J* = 8.1 Hz, 1H), 6.55 - 6.30 (m, 1H), 6.25 - 5.95 (m, 2H), 5.60 - 5.33 (m, 1H), 5.04 (br d, *J* = 6.1 Hz, 1H), 4.59 - 4.34 (m, 2H), 4.11 - 3.97 (m, 1H), 3.92 - 3.63 (m, 4H), 3.59 (br s, 1H), 3.36 - 3.12 (m, 3H), 2.95 - 2.72 (m, 4H), 2.58 (t, *J* = 7.1 Hz, 2H), 1.78 - 1.67 (m, 2H), 1.01 (d, *J* = 6.5 Hz, 3H) ppm.

**Example 34: (6S,8R)-6-(4-((1-(3-fluoropropyl)azetidin-3-yl)amino)-2-methoxy-6-fluoro-phenyl)-8-methyl-7-(2,2,2-trifluoroethyl)-6,7,8,9-6,7,8,9-tetrahydrooxazolo[5,4-f]isoquinoli n-2(3H)-one (Compound 34)**

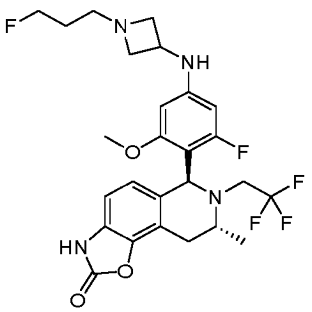

**LCMS:** m/z 541.2 [M+H]+.

**$^1$H NMR** (400 MHz, DMSO-$d_6$) δ = 11.46 (br s, 1H), 6.75 (d, *J* = 8.0 Hz, 1H), 6.51 - 6.36 (m, 2H), 5.99 (br s, 1H), 5.75 (br d, *J* = 12.8 Hz, 1H), 5.22 (br s, 1H), 4.51 (t, *J* = 5.9 Hz, 1H), 4.40 (t, *J* = 6.0 Hz, 1H), 4.03 - 3.89 (m, 1H), 3.70 (br s, 5H), 3.50 - 3.39 (m, 1H), 3.39 - 3.33 (m, 1H), 3.31 - 3.25 (m, 1H), 3.08 - 2.62 (m, 6H), 1.74 - 1.59 (m, 2H), 1.01 (br d, *J* = 6.5 Hz, 3H) ppm.

**Example 35: (E)-4-(3-((3,5-difluoro-4-((6S,8R)-8-methyl-2-oxo-7-(2,2,2-trifluoroethyl)-2,3,6,7,8, 9-hexahydrooxazolo[5,4-f]isoquinolin-6-yl)phenyl)amino)azetidin-1-yl)-N,N-dimet** hylbut-2-enamide (Compound 35)

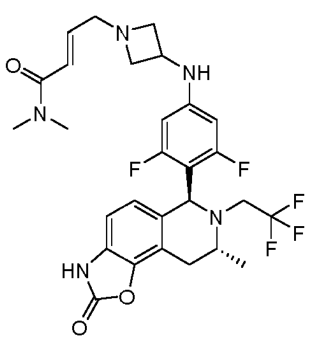

Step I: Synthesis of tert-butyl 3-((3,5-difluoro-4-((6S,8R)-8-methyl-2-oxo-7-(2,2,2-trifluoroethyl)-3-triphenylmethyl-2,3,6,7,8,9-hexahydrooxazolo[5,4-f]isoquinolin-6-yl)phenyl)amino)azetidine-1-carboxy late

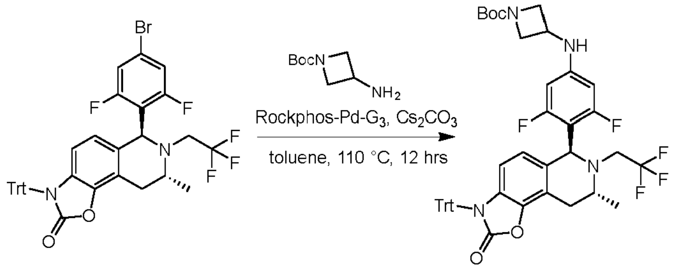


Tert-butyl (6S,8R)-6-(4-bromo-2,6-difluorophenyl)-8-methyl-7-(2,2,2-trifluoroethyl)-3-triphenylm ethyl-6,7,8,9-tetrahydroxazolo[5,4-f]isoquinolin-2(3H)-one (1.0 g, 1.39 mmol), 3-aminoazetidine-1-carboxylate (359 mg, 2.08 mmol), cesium carbonate (996 mg, 3.06 mmol), and [(2-bis-tert-butylphosphine-3-methoxy-6-methyl-2,4,6-triisopropyl-1,1-biphenyl)-2-(2-a minobiphenyl)]palladium(II) methanesulfonate (58.3 mg, 69.5 umol) were dissolved in toluene (20 mL), and the mixture was reacted under nitrogen protection at 110°C for 12 h. LCMS monitored the completion of the reaction. The mixture was concentrated and then extracted with water and ethyl acetate, and the organic phase was washed with a saturated sodium chloride aqueous solution, dried with sodium sulfate and concentrated. The crude product was concentrated and purified by column chromatography to obtain tert-butyl 3-((3,5-difluoro-4-((6S,8R)-8-methyl-2-oxo-7-(2,2,2-trifluoroethyl)-3-triphenylmethyl-2,3,6,7,8,9-hexahydrooxazolo[5,4-f]isoquinolin-6-yl)phenyl)amino)azetidine-1-carboxy late as a yellow solid (880 mg, 1.09 mmol, yield 78.1%).

**LCMS:** m/z 810.3 $[M+H]^+$.

**$^1$H NMR** (400 MHz, CHLOROFORM-*d*) δ = 7.36 - 7.42 (m, 7 H), 7.17 - 7.22 (m, 10 H), 6.15 - 6.24 (m, 1 H), 5.87 (d, *J*=10.76 Hz, 2 H), 5.58 (d, *J*=8.63 Hz, 1 H), 5.05 (s, 1 H), 4.21 (t, *J*=8.00 Hz, 2 H), 3.65 (ddd, *J*=8.88, 4.63, 2.13 Hz, 2 H), 3.43 - 3.52 (m, 1 H), 3.06 (m, *J*=16.70, 5.70 Hz, 2 H), 2.62 - 2.85 (m, 2 H), 1.37 (s, 9 H), 0.98 (d, *J*=6.50 Hz, 3 H) ppm.

Step II: Synthesis of (6S,8R)-6-(4-(azetidin-3-ylamino)-2,6-difluorophenyl)-8-methyl-7-(2,2,2-trifluoroethyl )-6,7,8,9-tetrahydroxazolo[5,4-f]isoquinolin-2(3H)-one

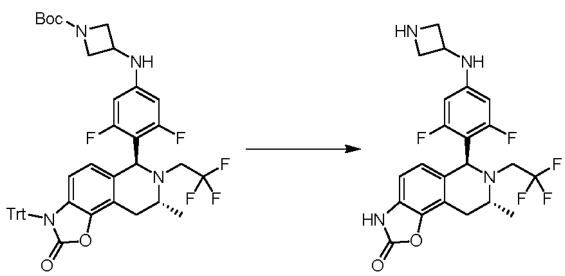

Trifluoroacetic acid (15.5 g, 135 mmol) was dissolved in water (1 mL), tert-butyl 3-((3,5-difluoro-4-((6S,8R)-8-methyl-2-oxo-7-(2,2,2-trifluoroethyl)-3-triphenylmethyl-2,3,6,7,8,9-hexahydrooxazolo[5,4-f]isoquinolin-6-yl)phenyl)amino)azetidine-1-carboxy late (400 mg, 493 umol) was then added, and the mixture was reacted under nitrogen protection at 20°C for 2 h. LCMS detected the completion of the reaction. The mixture was concentrated to obtain crude (6S,8R)-6-(4-(azetidin-3-ylamino)-2,6-difluorophenyl)-8-methyl-7-(2,2,2-trifluoroethyl )-6,7,8,9-tetrahydroxazolo[5,4-f]isoquinolin-2(3H)-one as a yellow solid (400 mg, crude product).

**LCMS:** m/z 468.2 $[M+H]^+$.

Step III: Synthesis of (E)-4-(3-((3,5-difluoro-4-((6S,8R)-8-methyl-2-oxo-7-(2,2,2-trifluoroethyl)-2,3,6,7,8,9-h exahydrooxazolo[5,4-f]isoquinolin-6-yl)phenyl)amino)azetidin-1-yl)-N,N-dimethylbut-2-enamide

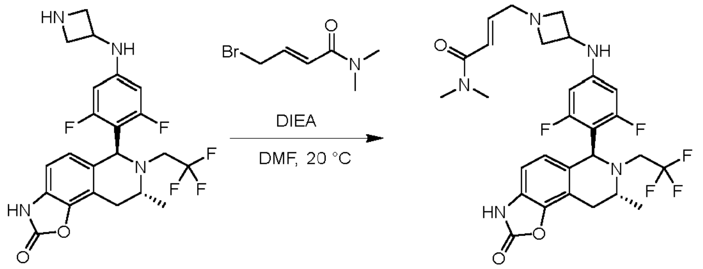


(6S,8R)-6-(4-(azetidin-3-ylamino)-2,6-difluorophenyl)-8-methyl-7-(2,2,2-trifluoro ethyl)-6,7,8,9-tetrahydroxazolo[5,4-f]isoquinolin-2(3H)-one (100 mg, 213 umol), (E)-4-bromo-N,N-dimethylbut-2-enamide(41 mg, 213 umol), and diisopropylethylamine (60.7 mg, 469 umol) were dissolved in N,N-dimethylformamide (3 mL), and the mixture was reacted under nitrogen protection at 20°C for 2 h. LCMS detected the completion of the reaction. The reaction solution was filtered and purified by prep-HPLC to obtain a product, which was separated and purified by SFC to obtain (E)-4-(3-((3,5-difluoro-4-((6S,8R)-8-methyl-2-oxo-7-(2,2,2-trifluoroethyl)-2,3,6,7,8,9-h exahydrooxazolo[5,4-f]isoquinolin-6-yl)phenyl) amino)azetidin-1-yl)-N,N-dimethylbut-2-enamide as a yellow solid (38.8 mg, 65.3 umol, yield 30.6%).

**LCMS:** m/z 579.2 $[M+H]^+$.

**$^1$H NMR** (400 MHz, ACETONITRILE-$d_3$) δ = 6.81 (d, *J*=8.00 Hz, 1 H), 6.50 - 6.66 (m, 2 H), 6.40 - 6.48 (m, 1 H), 6.08 (d, *J*=11.88 Hz, 2 H), 5.27 (m, *J*=7.00 Hz, 1 H), 5.20 (s, 1 H), 3.92 - 4.04 (m, 1 H), 3.69 (t, *J*=6.82 Hz, 2 H), 3.43 - 3.58 (m, 1 H), 3.22 - 3.38 (m, 1 H), 3.19 (dd, *J*=5.19, 1.31 Hz, 2 H), 3.00 - 3.09 (m, 4 H), 2.91 - 2.99 (m, 1 H), 2.88 - 2.91 (m, 3 H), 2.80 - 2.87 (m, 2 H), 2.73 (dd, *J*=16.45, 4.94 Hz, 1 H), 1.06 (d, *J*=6.50 Hz, 3 H) ppm.

**Example 36: (6S,8R)-6-(4-((1-acryloylazetidin-3-yl)amino)-2,6-difluorophenyl)-8-methyl-7-(2,2, 2-trifluoroethyl)-6,7,8,9-tetrahydroxazolo[5,4-f]isoquinolin-2(3H)-one (Compound 36)**

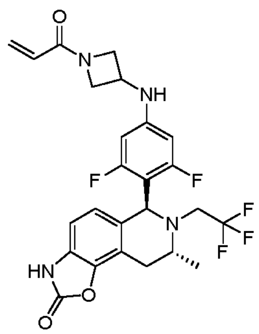

Step I: Synthesis of (6S,8R)-6-(4-((1-acryloylazetidin-3-yl)amino)-2,6-difluorophenyl)-8-methyl-7-(2,2,2-tr ifluoroethyl)-6,7,8,9-tetrahydroxazolo[5,4-f]isoquinolin-2(3H)-one

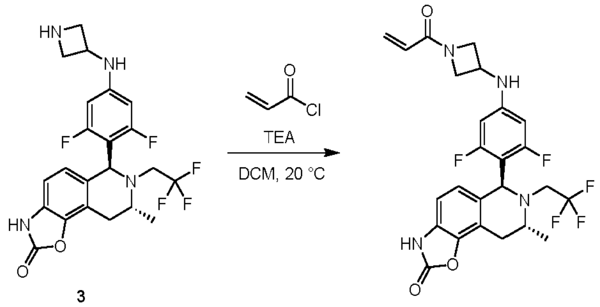


3

(6S,8R)-6-(4-(azetidin-3-ylamino)-2,6-difluorophenyl)-8-methyl-7-(2,2,2-trifluoro ethyl)-6,7,8,9-tetrahydroxazolo[5,4-f]isoquinolin-2(3H)-one (250 mg, 533 umol) and diisopropylethylamine (827 mg, 6.40 mmol) were dissolved in N,N-dimethylformamide (2 mL), acryloyl chloride (9.66 mg, 106 umol) was then added at 0°C, and the mixture was

reacted at 0°C for 2 h. LCMS detected the completion of the reaction. The reaction solution was filtered and purified by prep-HPLC to obtain a product, which was separated and purified by SFC to obtain (6S,8R)-6-(4-((1-acryloylazetidin-3-yl) amino)-2,6-difluorophenyl)-8-methyl-7-(2,2,2-tr ifluoroethyl)-6,7,8,9-tetrahydroxazolo[5,4-f]isoquinolin-2(3H)-one as a yellow solid (29.4 mg, 56.1 umol, yield 10.5%).

**LCMS:** m/z 522.2 $[M+H]^+$.

**$^1$H NMR** (400 MHz, ACETONITRILE-$d_3$) δ = 8.96 (s, 1 H), 6.82 (d, *J*=8.13 Hz, 1 H), 6.63 (d, *J*=8.13 Hz, 1 H), 6.21 - 6.36 (m, 1 H), 6.12 - 6.19 (m, 1 H), 6.09 (d, *J*=11.63 Hz, 2 H), 5.63 (dd, *J*=10.19, 1.94 Hz, 1 H), 5.44 (m, *J*=6.38 Hz, 1 H), 5.22 (s, 1 H), 4.49 - 4.59 (m, 1 H), 4.15 - 4.34 (m, 2 H), 3.94 (dt, *J*=9.10, 4.64 Hz, 1 H), 3.68 - 3.78 (m, 1 H), 3.45 - 3.57 (m, 1 H), 3.23 - 3.38 (m, 1 H), 2.87 - 3.09, (m, 2 H), 2.74 (dd, *J*=16.32, 5.19 Hz, 1 H), 1.06 (d, *J*=6.63 Hz, 3 H) ppm.

**Example 37: (E)-4-((R)-3-((3,5-difluoro4-((6S,8R)-8-methyl-2-oxo-7-(2,2,2-trifluoroethyl)-2,3,6,7 ,8,9-hexahydrooxazolo[5,4-f]isoquinolin-6-yl)phenoxy)pyrrolidin-1-yl)-N,N-dimeth ylbut-2-enamide (Compound 37)**

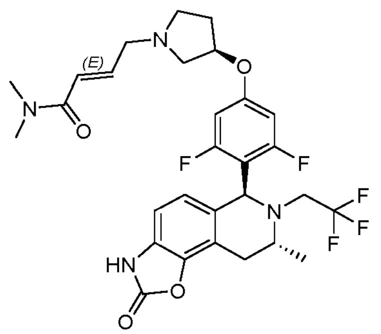

Step I: Synthesis of tert-butyl (R)-3-(3,5-difluoro-4-((6S,8R)-8-methyl-2-oxo-7-(2,2,2-trifluoroethyl)-3-triphenylmeth yl-2,3,6,7,8,9-hexahydrooxazolo[5,4-f]isoquinolin-6-yl)phenoxy)pyrrolidine-1-carboxyl ate

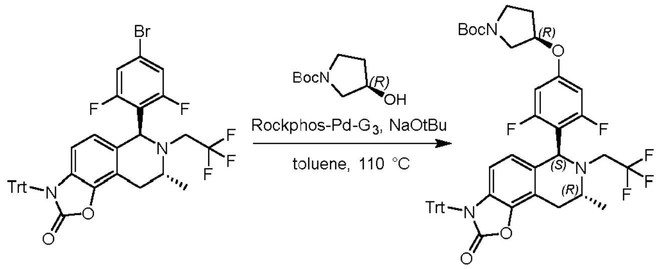


(6S,8R)-6-(4-bromo-2,6-difluorophenyl)-8-methyl-7-(2,2,2-trifluoroethyl)-3-triphe nylmethyl-6,7,8,9-tetrahydroxazolo[5,4-f]isoquinolin-2(3H)-one (1.7 g, 2.36 mmol), R-3-hydroxy-1-Boc-pyrrolidine (663 mg, 3.54 mmol), [(2-bis-tert-butylphosphine-3-methoxy-6-methyl-2,4,6-triisopropyl-1,1-biphenyl)-2-(2-a minobiphenyl)]palladium(II) methanesulfonate (99.0 mg, 118 umol), and cesium carbonate (1.69 g, 5.20 mmol) were dissolved in toluene (20 mL), and the mixture was reacted under nitrogen protection at 110°C for 12 h. LCMS monitored the completion of the reaction. The mixture was concentrated and then purified by column chromatography to obtain tert-butyl (R)-3-(3,5-difluoro-4-((6S,8R)-8-methyl-2-oxo-7-(2,2,2-trifluoroethyl)-3-triphenylmeth yl-2,3,6,7,8,9-hexahydrooxazolo[5,4-f]isoquinolin-6-yl)phenoxy)pyrrolidine-1-carboxyl ate as a light yellow solid (503 mg, 609 umol, yield 25.7%).

**LCMS:** m/z 825.3 $[M+H]^+$.

**$^1$H NMR** (400 MHz, CHLOROFORM-*d*) δ = 7.19 - 7.42 (m, 15 H), 6.26 (d, *J*=10.38 Hz, 2 H), 6.20 (d, *J*=8.63 Hz, 1 H), 5.60 (d, *J*=8.50 Hz, 1 H), 5.09 (s, 1 H), 3.46 - 3.59 (m, 4 H), 3.20 - 3.44 (m, 2 H), 2.86 - 3.17 (m, 3 H), 2.62 - 2.80 (m, 2 H), 2.08 (m, *J*=6.80 Hz, 1 H), 1.38 - 1.41 (m, 9 H), 0.98 (d, *J*=6.50 Hz, 3 H) ppm.

Step II: Synthesis of (6S,8R)-6-[2,6-difluoro-4-(((R)-pyrrolidin-3-yl)oxy)phenyl]-8-methyl-7-(2,2,2-trifluoro ethyl)-6,7,8,9-tetrahydroxazolo[5,4-f]isoquinolin-2(3H)-one

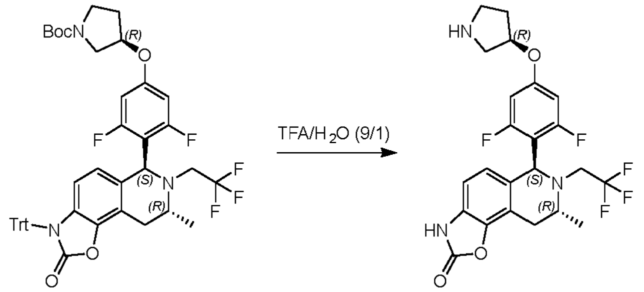


Trifluoroacetic acid (6.16 g, 54.0 mmol) was dissolved in water (0.4 mL), tert-butyl (R)-3-(3,5-difluoro-4-((6S,8R)-8-methyl-2-oxo-7-(2,2,2-trifluoroethyl)-3-triphenylmeth yl-2,3,6,7,8,9-hexahydrooxazolo[5,4-f]isoquinolin-6-yl)phenoxy)pyrrolidine-1-carboxyl ate (503 mg, 609 umol) was then added, and the mixture was reacted under nitrogen protection at 20°C for 2 h. LCMS detected the completion of the reaction. The mixture was concentrated, then adjusted to pH = 7 with saturated sodium bicarbonate, and extracted with ethyl acetate, and the organic phase was filtered, concentrated and subjected to column chromatography to obtain (6S,8R)-6-[2,6-difluoro-4-(((R)-pyrrolidin-3-yl)oxy) phenyl]-8-methyl-7-(2,2,2-trifluoro ethyl)-6,7,8,9-tetrahydroxazolo[5,4-f]isoquinolin-2(3H)-one as a yellow solid (183 mg, 378 umol, yield 62.2%).

**LCMS:** m/z 483.2 $[M+H]^+$.

Step III: Synthesis of (E)-4-((R)-3-((3,5-difluoro4-((6S,8R)-8-methyl-2-oxo-7-(2,2,2-trifluoroethyl)-2,3,6,7,8, 9-hexahydrooxazolo[5,4-f]isoquinolin-6-yl)phenoxy)pyrrolidin-1-yl)-N,N-dimethylbut-2-enamide

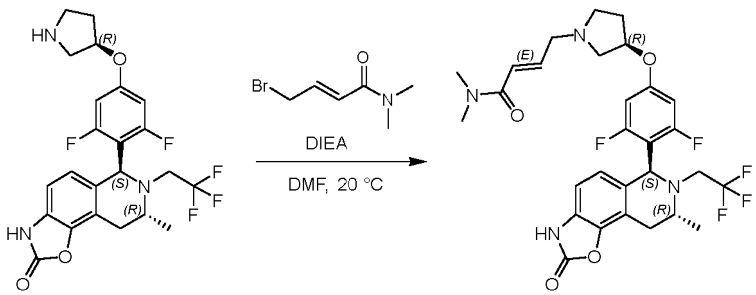


(6S,8R)-6-[2,6-difluoro-4-(((R)-pyrrolidin-3-yl)oxy)phenyl)-8-methyl-7-(2,2,2-trif luoroethyl)-6,7,8,9-tetrahydroxazolo[5,4-f]isoquinolin-2(3H)-one (90 mg, 186 umol), (E)-4-bromo-N,N-dimethylbut-2-enamide(35.7 mg, 186 umol), and N,N-diisopropylethylamine (52.9 mg, 409 umol) were dissolved in N,N-dimethylformamide (1 mL), and the mixture was reacted under nitrogen protection at 0°C for 1 h. LCMS detected the completion of the reaction. The reaction solution was filtered and separated by prep-HPLC to obtain a product, which was purified by SFC to obtain (E)-4-((R)-3-((3,5-difluoro4-((6S,8R)-8-methyl-2-oxo-7-(2,2,2-trifluoroethyl)-2,3,6,7,8, 9-hexahydrooxazolo[5,4-f]isoquinolin-6-yl)phenoxy)pyrrolidin-1-yl)-N,N-dimethylbut-2-enamide as a yellow solid (18.5 mg, 30.7 umol, yield 16.5%).

**LCMS:** m/z 579.2 $[M+H]^+$.

**$^1$H NMR** (400 MHz, ACETONITRILE-$d_3$) δ = 6.82 (d, *J*=8.25 Hz, 1 H), 6.60 - 6.71 (m, 2 H), 6.45 - 6.54 (m, 2 H), 6.44 (s, 1 H), 5.29 (s, 1 H), 4.81 (m, *J*=7.30, 4.60 Hz, 1 H), 3.52 (m, *J*=6.00 Hz, 1 H), 3.31 (s, 1 H), 3.22 (m, *J*=6.00 Hz, 2 H), 3.04 - 3.09 (m, 1 H), 3.01 (s, 3 H), 2.93 - 2.98 (m, 1 H), 2.89 (s, 3 H), 2.66 - 2.87 (m, 5 H), 2.42 (m, *J*=7.50 Hz, 1 H), 2.30 (m, *J*=7.60 Hz, 1 H), 1.07 (d, *J*=6.50 Hz, 3 H) ppm.

**Example 38: (6S,8R)-6-(4-(((R)-1-acryloylpyrrolidin-3-yl)oxy)-2,6-difluorophenyl)-8-methyl-7-( 2,2,2-trifluoroethyl)-6,7,8,9-tetrahydroxazolo[5,4-f]isoquinolin-2(3H)-one (Compound 38)**

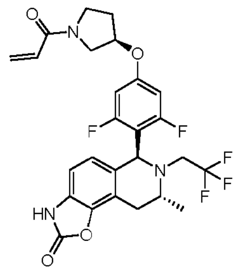

Step I: Synthesis of (6S,8R)-6-(4-(((R)-1-acryloylpyrrolidin-3-yl)oxy)-2,6-difluorophenyl)-8-methyl-7-(2,2, 2-trifluoroethyl)-6,7,8,9-tetrahydroxazolo[5,4-f]isoquinolin-2(3H)-one

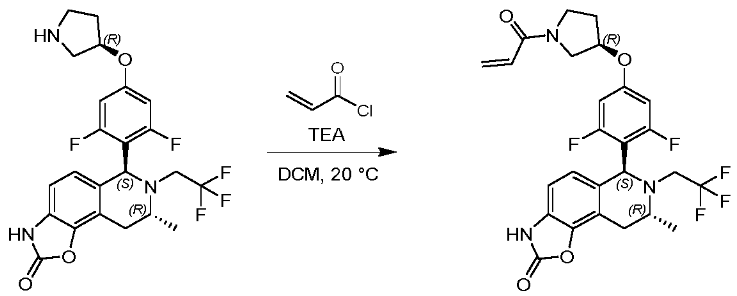


(6S,8R)-6-[2,6-difluoro-4-(((R)-pyrrolidin-3-yl)oxy)phenyl)-8-methyl-7-(2,2,2-trif luoroethyl)-6,7,8,9-tetrahydroxazolo[5,4-f]isoquinolin-2(3H)-one (90 mg, 186 umol) and diisopropylethylamine (240 mg, 1.86 mmol) were dissolved in N,N-dimethylformamide (1 mL), acryloyl chloride (16.8 mg, 186 umol) was then added at 0°C, and the mixture was reacted at 0°C for 1 h. LCMS detected the completion of the reaction. The reaction solution was filtered and separated by prep-HPLC to obtain a product, which was purified by SFC to obtain (6S,8R)-6-(4-(((R)-1-acryloylpyrrolidin-3-yl)oxy)-2,6-difluorophenyl)-8-methyl-7-(2,2, 2-trifluoroethyl)-6,7,8,9-tetrahydroxazolo[5,4-f]isoquinolin-2(3H)-one as a white solid (24.8 mg, 45.9 umol, yield 24.6%).

**LCMS:** m/z 537.2 $[M+H]^+$.

**$^1$H NMR** (400 MHz, ACETONITRILE-*$d_3$*) δ = 8.70 - 9.00 (m, 1 H), 6.82 (d, *J*=8.13 Hz, 1 H), 6.63 (d, *J*=8.00 Hz, 1 H), 6.44 - 6.60 (m, 3 H), 6.15 - 6.25 (m, 1 H), 5.64 (ddd, *J*=10.35, 8.10, 2.31 Hz, 1 H), 5.30 (s, 1 H), 4.92 - 5.06 (m, 1 H), 3.54 - 3.88 (m, 4 H), 3.31 - 3.52 (m, 2 H), 3.07 (dd, *J*=16.45, 4.94 Hz, 1 H), 2.94 (dq, *J*=15.90, 9.63 Hz, 1 H), 2.76 (dd, *J*=16.51, 5.13 Hz, 1 H), 2.16 - 2.25 (m, 2 H), 1.07 (d, J=6.63 Hz, 3 H) ppm.

Similarly to the synthesis route of Examples 37 and 38 above, the following Examples 39 and 40 were synthesized by selecting corresponding reactants and synthesis methods known to those skilled in the art.

**Example 39: (E)-4-((S)-3-((3,5-difluoro4-((6S,8R)-8-methyl-2-oxo-7-(2,2,2-trifluoroethyl)-2,3,6,7, 8,9-hexahydrooxazolo[5,4-f]isoquinolin-6-yl)phenoxy)pyrrolidin-1-yl)-N,N-dimeth ylbut-2-enamide (Compound 39)**

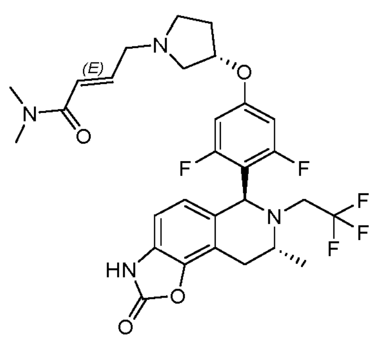

**LCMS:** m/z 579.2 $[M+H]^+$.

**$^{1}$H NMR** (400 MHz, ACETONITRILE-d3) δ = 6.82 (d, J=8.13 Hz, 1 H), 6.60 - 6.71 (m, 2 H), 6.48 - 6.53 (m, 1 H), 6.45 - 6.48 (m, 1 H), 6.43 - 6.45 (m, 1 H), 5.29 (s, 1 H), 4.77 - 4.84 (m, 1 H), 3.47 - 3.57 (m, 1 H), 3.32 (m, J=9.60 Hz, 1 H), 3.22 (dd, J=6.07, 1.44 Hz, 2 H), 3.07 (dd, J=16.76, 4.75 Hz, 1 H), 3.00 - 3.04 (m, 3 H), 2.93 - 2.99 (m, 1 H), 2.89 (s, 3 H), 2.83 (s, 5 H), 2.37 - 2.49 (m, 1 H), 2.26 - 2.36 (m, 1 H), 1.07 (d, J=6.50 Hz, 3 H) ppm.

**Example 40: (6S,8R)-6-(4-(((S)-1-acryloylpyrrolidin-3-yl)oxy)-2,6-difluorophenyl)-8-methyl-7-(2 ,2,2-trifluoroethyl)-6,7,8,9-tetrahydroxazolo[5,4-f]isoquinolin-2(3H)-one (Compound 40)**

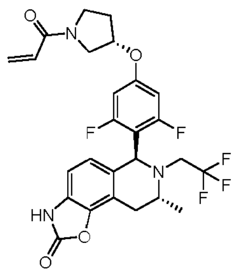

**LCMS:** m/z 537.2 [M+H]$^{+}$.

**$^{1}$H NMR** (400 MHz, ACETONITRILE-$d_3$) δ = 6.82 (d, *J*=8.00 Hz, 1 H), 6.62 (d, *J*=8.00 Hz, 1 H), 6.44 - 6.59 (m, 3 H), 6.16 - 6.26 (m, 1 H), 5.64 (m, *J*=10.40, 10.40, 2.30 Hz, 1 H), 5.30 (s, 1 H), 4.92 - 5.06 (m, 1 H), 3.55 - 3.88 (m, 4 H), 3.45 - 3.53 (m, 1 H), 3.34 (m, *J*=16.10, 9.60 Hz, 1 H), 3.03 - 3.12 (m, 1 H), 2.89 - 3.00 (m, 1 H), 2.76 (dd, *J*=16.70, 5.32 Hz, 1 H), 1.77 (m, *J*=4.90, 2.40 Hz, 2 H), 1.07 (d, *J*=6.63 Hz, 3 H) ppm.

**Example 41: (E)-4-(4-(3,5-difluoro-4-((6S,8R)-8-methyl-2-oxo-7-(2,2,2-trifluoroethyl)-2,3,6,7,8,9 -hexahydrooxazolo[5,4-f]isoquinolin-6-yl)phenyl)piperazin-1-yl)-N,N-dimethylbut-2-enamide (Compound 41)**

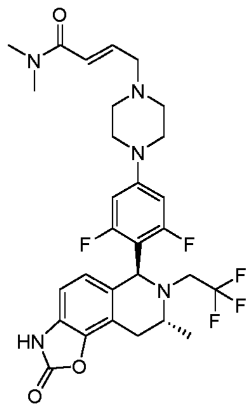

Step I: Synthesis of tert-butyl 4-(3,5-difluoro-4-((6S,8R)-8-methyl-2-oxo-7-(2,2,2-trifluoroethyl)-3-triphenylmethyl-2 ,3,6,7,8,9-hexahydrooxazolo[5,4-f]isoquinolin-6-yl)phenyl)piperazine-1-carboxylate

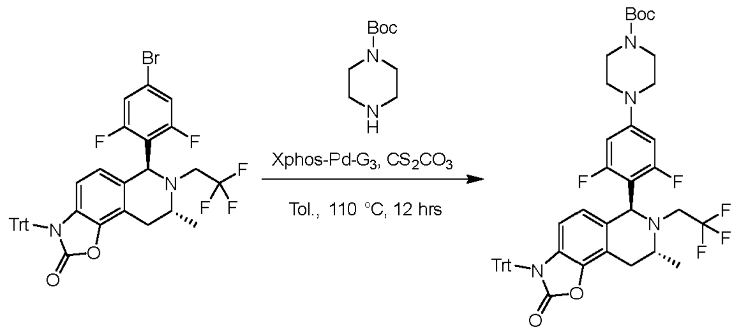


(6S,8R)-6-(4-bromo-2,6-difluorophenyl)-8-methyl-7-(2,2,2-trifluoroethyl)-3-triphe nylmethyl-6,7,8,9-tetrahydroxazolo[5,4-f]isoquinolin-2(3H)-one (700 mg, 972 umol), N-Boc piperazine (272 mg, 1.46 mmol), (2-dicyclohexylphosphino-2',4',6'-tri-isopropyl-1,1'-biphenyl)(2'-amino-1,1'-biphenyl-2-yl)palladium(II) methanesulfonate (41.2 mg, 48.6 umol) and cesium carbonate (697 mg, 2.14 mmol) were dissolved in toluene (15 mL), and the mixture was reacted under nitrogen protection at 110°C for 12 h. LCMS monitored the completion of the reaction. The mixture was concentrated and then extracted with water and ethyl acetate, and the organic phase was washed with a saturated sodium chloride aqueous solution, dried with sodium sulfate and concentrated. The crude product was purified by column chromatography to obtain tert-butyl 4-(3,5-difluoro-4-((6S,8R)-8-methyl-2-oxo-7-(2,2,2-trifluoroethyl)-3-triphenylmethyl-2 ,3,6,7,8,9-hexahydrooxazolo[5,4-f]isoquinolin-6-yl)phenyl)piperazine-1-carboxylate as a white solid (413 mg, 501 umol, yield 51.5%).

**LCMS:** m/z 824.3 $[M+H]^+$.

**$^1$H NMR** (400 MHz, CHLOROFORM-*d*) δ = 7.27 - 7.53 (m, 15 H), 6.24 - 6.42 (m, 3 H), 5.63 - 5.74 (m, 1 H), 5.17 (s, 1 H), 3.52 - 3.68 (m, 5 H), 3.10 - 3.39 (m, 6 H), 2.77 (m, *J*=16.40, 4.30 Hz, 2 H), 1.52 (s, 9 H), 0.89 - 1.11 (m, 3 H) ppm.

Step II: Synthesis of (6S,8R)-6-(2,6-difluoro-4-(piperazin-1-yl)phenyl)-8-methyl-7-(2,2,2-trifluoroethyl)-6,7, 8,9-tetrahydrooxazolo[5,4-f]isoquinolin-2(3H)-one

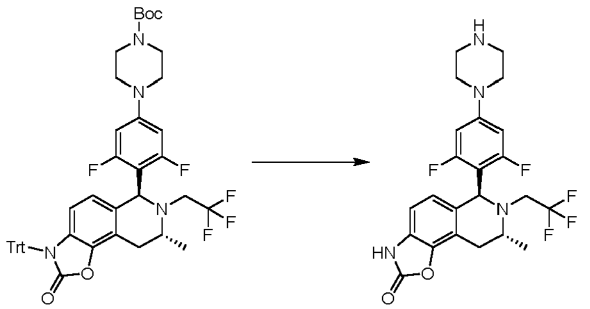

Trifluoroacetic acid (12.3 g, 107 mmol) was dissolved in water (0.1 mL), tert-butyl 4-(3,5-difluoro-4-((6S,8R)-8-methyl-2-oxo-7-(2,2,2-trifluoroethyl)-3-triphenylmethyl-2 ,3,6,7,8,9-hexahydrooxazolo[5,4-f]isoquinolin-6-yl)phenyl)piperazine-1-carboxylate (413 mg, 500 umol) was then added, and the mixture was reacted under nitrogen protection at 20°C for 2 h. LCMS detected the completion of the reaction. The crude product was subjected to column chromatography to obtain (6S,8R)-6-(2,6-difluoro-4-(piperazin- 1-yl)phenyl)-8-methyl-7-(2,2,2-trifluoroethyl)-6,7, 8,9-tetrahydrooxazolo[5,4-f]isoquinolin-2(3H)-one as a yellow solid (110 mg, 228 umol, yield 45.5%).

**LCMS:** m/z 482.2 $[M+H]^+$.

**$^1$H NMR** (400 MHz, DMSO-$d_6$) δ = 6.81 (d, *J*=8.00 Hz, 1 H), 6.48 - 6.60 (m, 3 H), 5.16 (s, 1 H), 3.06 - 3.10 (m, 4 H), 2.86 - 2.99 (m, 3 H), 2.67 - 2.80 (m, 6 H), 1.05 (d, *J*=6.50 Hz, 3 H.) ppm.

Step III: Synthesis of (E)-4-(4-(3,5-difluoro-4-((6S,8R)-8-methyl-2-oxo-7-(2,2,2-trifluoroethyl)-2,3,6,7,8,9-h exahydrooxazolo[5,4-f]isoquinolin-6-yl)phenyl)piperazin-1-yl)-N,N-dimethylbut-2-ena mide

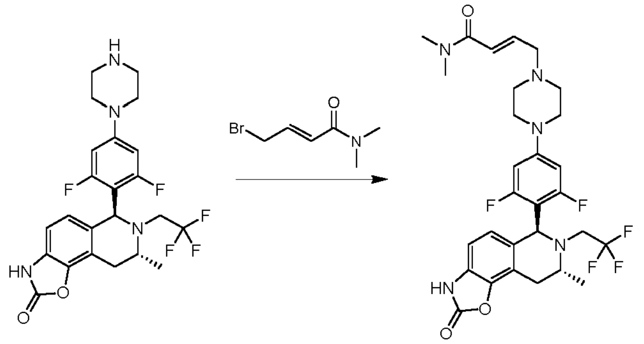


6S,8R)-6-(2,6-difluoro-4-(piperazin-1-yl)phenyl)-8-methyl-7-(2,2,2-trifluoroethyl) -6,7,8,9-tetrahydrooxazolo [5,4-f]isoquinolin-2(3H)-one (100 mg, 207 umol), (E)-4-bromo-N,N-dimethylbut-2-enamide(39.8 mg, 207.3 umol), and diisopropylethylamine (58.9 mg, 456 umol) were dissolved in N,N-dimethylformamide (2 mL), and the mixture was reacted under nitrogen protection at 20°C for 1 h. LCMS detected the completion of the reaction. The reaction solution was filtered and separated by prep-HPLC to obtain a product, which was purified by SFC to obtain (E)-4-(4-(3,5-difluoro-4-((6S,8R)-8-methyl-2-oxo-7-(2,2,2-trifluoroethyl)-2,3,6,7,8,9-h exahydrooxazolo[5,4-f]isoquinolin-6-yl)phenyl)piperazin-1-yl)-N,N-dimethylbut-2-ena mide as a yellow solid (37 mg, 62.3 umol, yield 30.1%).

**LCMS:** m/z 593.2 $[M+H]^+$.

**$^1$H NMR** (400 MHz, ACETONITRILE-$d_3$) δ = 8.78 - 8.97 (m, 1 H), 6.81 (d, *J*=8.13 Hz, 1 H), 6.50 - 6.69 (m, 3 H), 6.38 - 6.47 (m, 2 H), 5.25 (s, 1 H), 3.45 - 3.59 (m, 1 H), 3.24 - 3.40 (m, 1 H), 3.12 - 3.22 (m, 6 H), 3.04 - 3.10 (m, 1 H), 3.03 (s, 3 H), 2.92 - 3.00 (m, 1 H), 2.90 (s, 3 H), 2.70 - 2.79 (m, 1 H), 2.46 - 2.60 (m, 4 H), 1.07 (d, *J*=6.63 Hz, 3 H) ppm.

**Example 42: 3-(4-((2-(4-(3-((6-((6S,8R)-8-methyl-2-oxo-7-(2,2,2-trifluoroethyl)-2,3,6,7,8,9-hexah ydrooxazolo [5,4-f]isoquinolin-6-yl)pyridin-3-yl)oxy)ethyl)piperazin-1-yl)ethyl)ami no)-1-oxoisoindolin-2-yl)piperidine-2,6-dione (Compound 42)**

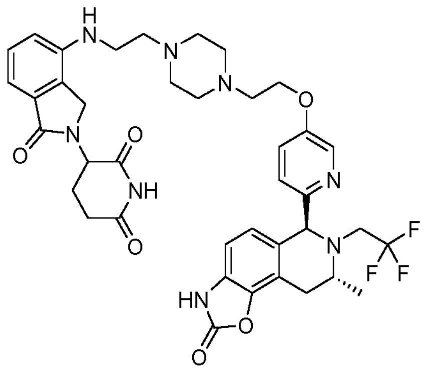

Step I: Synthesis of tert-butyl 4-(2-((6-((6S,8R)-8-methyl-2-oxo-7-(2,2,2-trifluoroethyl)-2,3,6,7,8,9-hexahydrooxazol o [5,4-f]isoquinolin-6-yl)pyridin-3-yl)oxy)ethyl)piperazine-1-carboxylate

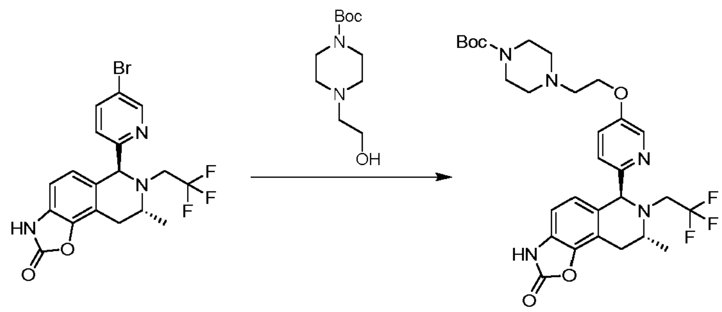

Tert-butyl (6S,8R)-6-(5-bromopyridin-2-yl)-8-methyl-7-(2,2,2-trifluoroethyl)-6,7,8,9-tetrahydroox azolo[5,4-f] isoquinolin-2(3H)-one (700 mg, 1.58 mmol), 4-(2-hydroxyethyl)piperazine-1-carboxylate (546 mg, 2.37 mmol), [(2-bis-tert-butylphosphine-3-methoxy-6-methyl-2,4,6-triisopropyl-1,1-biphenyl)-2-(2-a minobiphenyl)]palladium(II) methanesulfonate (132 mg, 158 umol) and sodium tert-butoxide (456 mg, 4.75 mmol) were dissolved in toluene (10 mL), and the mixture was reacted under nitrogen protection at 60°C for 12 h. TLC monitored the completion of the reaction. The reaction solution was concentrated. The crude product was purified by column chromatography to obtain tert-butyl 4-(2-((6-((6S,8R)-8-methyl-2-oxo-7-(2,2,2-trifluoroethyl)-2,3,6,7,8,9-hexahydrooxazol o[5,4-f]isoquinolin-6-yl)pyridin-3-yl)oxy)ethyl)piperazine-1-carboxylate as a yellow solid (400 mg, 676 umol, yield 42.7%).

**LCMS:** m/z 591.3 $[M+H]^+$.

**$^1$H NMR** (400 MHz, DMSO-$d_6$) δ = 11.36 - 11.63 (m, 1 H), 7.93 - 8.18 (m, 1 H), 7.37 (dd, *J*=8.69, 2.94 Hz, 1 H), 7.11 - 7.27 (m, 1 H), 6.84 (d, *J*=8.13 Hz, 1 H), 6.68 (d, *J*=8.13 Hz, 1 H), 5.02 (s, 1 H), 4.01 - 4.19 (m, 2 H), 3.37 - 3.64 (m, 3 H), 3.17 (d, *J*=5.13 Hz, 1 H), 2.89 - 3.03 (m, 1 H), 2.80 - 2.89 (m, 1 H), 2.55 - 2.76 (m, 3 H), 2.24 - 2.45 (m, 6 H), 1.38 (s, 9 H), 1.06 (m, *J*=6.50 Hz, 3 H) ppm.

Step II: Synthesis of (6S,8R)-8-methyl-6-(5-(2-(piperazin-1-yl)ethoxy)pyridin-2-yl)-7-(2,2,2-trifluoroethyl)-6,7,8,9-tetrahydroxazolo[5,4-f]isoquinolin-2(3H)-one

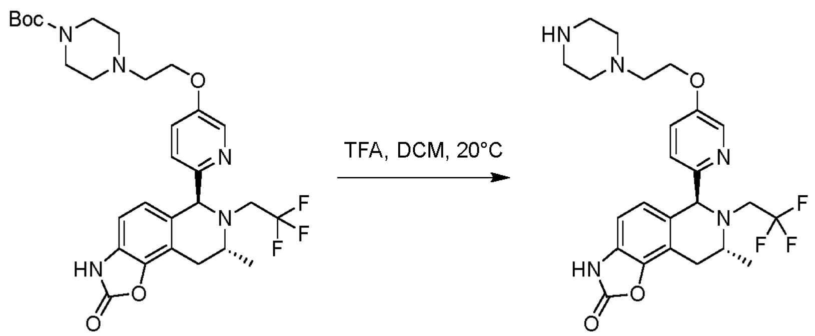


Tert-butyl 4-(2-((6-((6S,8R)-8-methyl-2-oxo-7-(2,2,2-trifluoroethyl)-2,3,6,7,8,9-hexahydrooxazol o[5,4-f]isoquinolin-6-yl)pyridin-3-yl)oxy)ethyl)piperazine-1-carboxylate (392 mg, 662.5 umol) and trifluoroacetic acid (6.16 g, 54.0 mmol) were dissolved in dichloromethane (10 mL), and the mixture was reacted at 20°C for 1 h. LCMS monitored the completion of the reaction. The reaction solution was concentrated, then adjusted to pH = 7-8 with a saturated sodium bicarbonate solution, and extracted with water and ethyl acetate, and the organic phase was washed with a saturated sodium chloride aqueous solution, dried with sodium sulfate and concentrated. The crude product was purified by column chromatography to obtain (6S,8R)-8-methyl-6-(5-(2-(piperazin-1-yl)ethoxy)pyridin-2-yl)-7-(2,2,2-trifluoroethyl)-6,7,8,9-tetrahydroxazolo[5,4-f]isoquinolin-2(3H)-one as a yellow solid (156 mg, 317 umol, yield 47.9%).

**LCMS:** m/z 491.2 $[M+H]^+$.

**$^1$H NMR** (400 MHz, DMSO-$d_6$) δ = 8.08 - 8.20 (m, 1 H), 7.36 (dd, *J*=8.63, 2.88 Hz, 1 H), 7.25 (d, *J*=8.63 Hz, 1 H), 6.84 (d, *J*=8.13 Hz, 1 H), 6.68 (d, *J*=8.13 Hz, 1 H), 5.03 (s, 1 H), 4.11 (br t, *J*=5.44 Hz, 2 H), 3.52 - 3.59 (m, 1 H), 3.34 - 3.42 (m, 1 H), 2.80 - 2.99 (m, 6 H), 2.63 - 2.76 (m, 3 H), 2.53 - 2.63 (m, 4 H), 1.06 (d, *J*=6.63 Hz, 3 H) ppm.

Step III: Synthesis of 3-(4-((2-bromoethyl)amino)-1-oxoisoindolin-2-yl)piperidine-2,6-dione

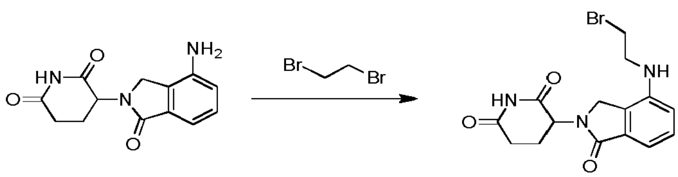

3-(4-Amino-1-oxoisoindolin-2-yl)piperidine-2,6-dione (2.00 g, 7.71 mmol), 1,2-dibromoethane (17.3 g, 92.5 mmol) and N,N-dimethylisopropylamine (2.99 g, 23.1 mmol) were dissolved in N-methylpyrrolidone (20 mL), and after nitrogen displacement, the mixture was reacted at 100°C for 4 h. LCMS monitored the completion of the reaction. The reaction solution was washed with water and extracted with ethyl acetate, and the organic phase was washed with a

saturated sodium chloride aqueous solution, dried with anhydrous sodium sulfate and concentrated. The crude product was purified by column chromatography to obtain 3-(4-((2-bromoethyl)amino)-1-oxoisoindolin-2-yl)piperidine-2,6-dione as a yellow solid (403 mg, 1.10 mmol, yield 14.27%).

**LCMS:** m/z 365.0 [M+H]+.

**$^1$H NMR** (400 MHz, DMSO-$d_6$) δ = 11.00 (s, 1 H), 7.31 (t, *J*=7.75 Hz, 1 H), 6.98 (d, *J*=7.25 Hz, 1 H), 6.84 (d, *J*=8.00 Hz, 1 H), 5.93 (br s, 1 H), 5.11 (dd, *J*=13.26, 5.00 Hz, 1 H), 4.18 - 4.28 (m, 1 H), 4.09 - 4.17 (m, 1 H), 3.53 - 3.64 (m, 4 H), 2.86 - 2.99 (m, 1 H), 2.64 (br s, 1 H), 2.23 - 2.37 (m, 1 H), 1.98 - 2.09 (m, 1 H) ppm.

Step IV: Synthesis of 3-(4-((2-(4-(3-((6-((6S,8R)-8-methyl-2-oxo-7-(2,2,2-trifluoroethyl)-2,3,6,7,8,9-hexahyd rooxazolo [5,4-f]isoquinolin-6-yl)pyridin-3-yl)oxy)ethyl)piperazin-1-yl)ethyl)amino)-1-oxoisoindolin-2-yl)piperidine-2,6-dione

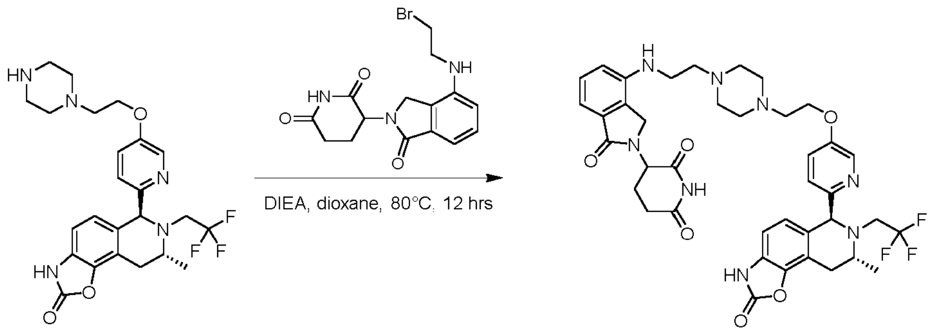


(6S,8R)-8-methyl-6-(5-(2-(piperazin-1-yl)ethoxy)pyridin-2-yl)-7-(2,2,2-trifluoroet hyl)-6,7,8,9-tetrahydroxazolo [5,4-f]isoquinolin-2(3H)-one (70 mg, 142 umol), 3-(4-((2-bromoethyl)amino)-1-oxoisoindolin-2-yl)piperidine-2,6-dione (57.3 mg, 156 umol), and N,N-diisopropylethylamine (55.2 mg, 427 umol) were dissolved in dioxane (2 mL), and the mixture was then reacted under nitrogen protection at 80°C for 12 h. LCMS monitored the completion of the reaction. The reaction solution was spin-dried and then purified by prep-HPLC to obtain 3-(4-((2-(4-(3-((6-((6S,8R)-8-methyl-2-oxo-7-(2,2,2-trifluoroethyl)-2,3,6,7,8,9-hexahyd rooxazolo[5,4-f]isoquinolin-6-yl)pyridin-3-yl)oxy)ethyl)piperazin-1-yl) ethyl)amino)-1-oxoisoindolin-2-yl)piperidine-2,6-dione as a yellow solid (20 mg, 25.8 umol, yield 18.1%).

**LCMS:** m/z 776.3 [M+H]+.

**$^1$H NMR** (400 MHz, ACETONITRILE-$d_3$) δ = 8.91 (br s, 1 H), 8.10 (d, *J*=2.50 Hz, 1 H), 7.31 - 7.37 (m, 1 H), 7.28 (s, 1 H), 7.26 (br d, *J*=2.75 Hz, 1 H), 7.05 (d, *J*=7.38 Hz, 1 H), 6.83 (t, *J*=7.69 Hz, 2 H), 6.70 (d, *J*=8.00 Hz, 1 H), 4.98 - 5.18 (m, 2 H), 4.54 - 4.91 (m, 1 H), 4.22 - 4.31 (m, 1 H), 4.19 (s, 1 H), 4.13 (t, *J*=5.57 Hz, 2 H), 3.36 - 3.49 (m, 2 H), 3.28 - 3.35 (m, 2 H), 2.84 - 3.07 (m, 3 H), 2.69 - 2.83 (m, 7 H), 2.68 (br s, 8 H), 2.39 - 2.43 (m, 1 H), 1.28 (br d, *J*=6.25 Hz, 1 H), 1.08 (d, *J*=6.75 Hz, 3 H) ppm.

Similarly to the synthesis route of Example 42 above, the following Example 43 was synthesized by selecting corresponding reactants and synthesis methods known to those skilled in the art.

**Example 43: 3-(4-((2-(4-(3-((6-((6S,8R)-8-methyl-2-oxo-7-(2,2,2-trifluoroethyl)-2,3,6,7,8,9-hexah ydrooxazolo [5,4-f]isoquinolin-6-yl)pyridin-3-yl)oxy)propyl)piperazin-1-yl)ethyl)a mino)-1-oxoisoindolin-2-yl)piperidine-2,6-dione (Compound 43)**

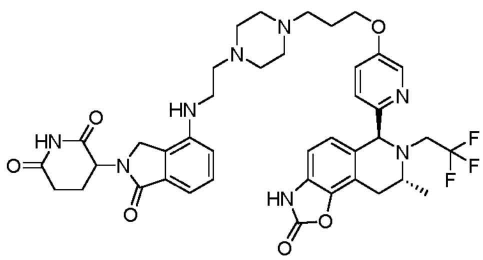

**LCMS:** m/z 790.3 [M+H]+.

**$^1$H NMR** (400 MHz, ACETONITRILE-$d_3$) δ = 8.71 - 9.01 (m, 1 H), 8.09 (d, *J*=2.50 Hz, 1 H), 7.40 (s, 1 H), 7.26 - 7.30

(m, 1 H), 7.20 - 7.26 (m, 1 H), 7.04 (d, *J*=7.38 Hz, 1 H), 6.83 (t, *J*=7.69 Hz, 2 H), 6.70 (d, *J*=8.13 Hz, 1 H), 5.02 - 5.17 (m, 2 H), 4.50 (br s, 1 H), 4.18 (q, *J*=16.38 Hz, 2 H), 4.06 (t, *J*=6.32 Hz, 2 H), 3.43 - 3.53 (m, 1 H), 3.33 - 3.43 (m, 1 H), 3.26 (br d, *J*=5.25 Hz, 2 H), 2.90 - 3.06 (m, 2 H), 2.73 - 2.87 (m, 2 H), 2.70 (br dd, *J*=4.57, 2.69 Hz, 1 H), 2.59 (br t, *J*=6.19 Hz, 3 H), 2.29 - 2.53 (m, 12 H), 1.28 (br d, *J*=6.13 Hz, 1 H), 1.08 (d, *J*=6.63 Hz, 3 H) ppm.

**Test Examples**

In the following test examples, AZD9496 (cat. no.: HY-12870), AZD9833 (cat. no.: HY-136255) and tamoxifen (cat. no.: HY-13757A) were purchased from MedChemExpress; and Comparative Compound 1 (chemical structure: (6S,8R)-6-(2,6-difluoro-4-(2-(3-(fluoromethyl)azetidin-1-yl)ethoxy)phenyl)-7-(2-fluoro -2-methylpropyl)-8-methyl-6,7,8,9-tetrahydro-3H-benzopyrazolo[4,3-f]isoquinoline) was prepared using the method in Example 1 of WO 2017/182493 A1.

**Test Example 1: ERα TR-FRET Test**

1X Tris-HCl (Sigma, PHG0002) protein buffer was prepared and mixed for later use. The compound to be detected was prepared into a stock solution with a concentration of 2 mM and then subjected to serial 3-fold gradient dilution, resulting in a total of 10 concentrations. The diluted compounds were respectively added to a reaction plate by means of Echo 550, with 100 nL per well, and at the same time 5 nL of estradiol (Sigma, 491187; final concentration 1.5 nM) was added to each well.

Preparation of 1x protein mixed solution: firstly, 2x GST-ERα-LBD (Invitrogen, A15677)/MAb anti-GST-Eu (Cisbio, 61GSTKLA) mixed solution was prepared according to the following table.

| Substance | Final concentration (nM) | Working concentration (nM) | Concentration of stock solution (nM) |
|---|---|---|---|
| GST-ERα-LBD | 2 | 4 | 20100 |
| MAb anti-GST-Eu | 2.5 ng/well | 50 nl/well | 50 μg/ml |

2x biotin-SRC2/streptavidin-XL665 (Cisbio, 610SAXLA) mixed solution was prepared.

| Substance | Final concentration (nM) | Working concentration (nM) | Concentration of stock solution (nM) |
|---|---|---|---|
| Biotin-SRC2 | 75 | 150 | 1000000 |
| Streptavidin-XL665 | 50 ng/well | 50 nl/well | 1 mg/ml |

The above 2x GST-NR-LBD/ MAb anti-GST-Eu solution and 2x biotin-SRC2/streptavidin-XL665 solution were uniformly mixed at a volume ratio of 1: 1; and the 1x protein mixed solution was added to each well of a 384-well plate, with 20 μL being added per well, the 384-well plate was put into a centrifuge and centrifuged at room temperature at 1000 rpm for 10 seconds, and it was taken out, then left to stand at room temperature for 3 h, and then read by EnVision multifunctional microplate reader.

The values at 665 and 615 (nm) were read out, and with the value at 615 as the correction value, the final value was expressed as the value at 665/the value at 615. The inhibition rate was calculated according to the following formula:

$$\%\text{Inhibition} = \left(\frac{\text{Max} - \text{X}}{\text{Max} - \text{Min}}\right) \times 100\%$$

X was "the value at 665 vs. the value at 615" for each concentration. Min was the average value of "the value at 665 vs. the value at 615" where 0.2 mM positive control compound and 5 nl of 3 nM (1.5 nM) estradiol were added. Max was the average value of "the value at 665 vs. the value at 615" where DMSO and 5 nl of 3 nM (1.5 nM) estradiol were added. The data were imported into Graphpad Prism, and Log(agonist) vs. response-variable slope was used for curve fitting.

Fitting formula: $Y = Bottom + (Top - Bottom) / (1 + 10^{((LogEC_{50} - X) * HillSlope)})$

**ERα TR-FRET Experimental Data**

| Compound | $IC_{50}$ (nM) | Maximum inhibition rate |
|---|---|---|
| Example 1 | 9.00 | 100.2 |
| Example 9 | 21.6 | 100.1 |
| Example 10 | 7.80 | 100.2 |
| Example 12 | 15.6 | 100.3 |
| Example 14 | 40.3 | 100.2 |
| Example 25 | 11.4 | 100.8 |
| Example 26 | 58.4 | 100.4 |
| Example 30 | 8.85 | 99.8 |
| Example 31 | 45.6 | 100.9 |
| Example 32 | 14.7 | 100.3 |
| Example 33 | 11.8 | 99.9 |
| Example 34 | 19.4 | 100.2 |
| AZD9833 | 62.0 | 99.8 |
| Tamoxifen | 578.1 | 100.4 |

**Test Example 2: MCF-7 Cell Proliferation Experiment**

Human breast cancer cells MCF-7 (HTB-22, purchased from ATCC) were inoculated into a 96-well plate. The culture medium was an MEM (Hyclone, SH30024.01B) culture medium containing 10% of FBS (Gibco, 10099-141), 1% of pyruvic acid (Sigma, S8636), and 1% of nonessential amino acid (Sigma, M7145), and the inoculation density was 4,000 cells/well. The cells were cultured at 37°C, 5% $CO_2$. The compound was prepared into a 20 mM stock solution, which was subjected to gradient dilution to a final concentration of 1000 with 100% DMSO (Sigma, D2650) and then diluted 20 folds with a culture medium containing 2% of FBS. After 24 hours of culture, the culture medium was removed, 90 μL of a culture medium containing 2% of FBS and 10 μL of drug were added to each well, while 10 μL of DMSO was added to the control group, and they were gently shaken and mixed. The blank group only contained 100 μL of 2% FBS culture medium. They were placed in an incubator and cultured at 37°C, 5% $CO_2$. After 72 hours, 50 μL of mixed Cell Titer-Glo (Promega, G7571) was added. They were shaken and mixed uniformly, and placed at room temperature for 10 min. The chemiluminescence signal value was determined. The negative control was 0.5% DMSO well.

**Experimental Data of MCF-7 Cell Proliferation Experiment**

| Compound | $IC_{50}$ (nM) |
|---|---|
| Example 1 | 0.39 |
| Example 9 | 0.19 |
| Example 10 | 0.20 |
| Example 11 | 0.38 |

(continued)

| Compound | $IC_{50}$ (nM) |
|---|---|
| Example 12 | 0.49 |
| Example 13 | 0.30 |
| Example 14 | 0.44 |
| Example 16 | 0.80 |
| Example 25 | 0.24 |
| Example 26 | 0.55 |
| Example 27 | 1.63 |
| Example 28 | 0.40 |
| Example 29 | 0.71 |
| Example 30 | 0.21 |
| Example 31 | 0.85 |
| Example 32 | 0.39 |
| Example 33 | 0.39 |
| Example 34 | 0.19 |
| Example 35 | 0.82 |
| Example 37 | 0.61 |
| Example 39 | 0.49 |
| Example 43 | 0.69 |
| AZD9496* | 3.84 |
| AZD9833* | 2.45 |
| Tamoxifen* | 274.50 |
| Comparative Compound 1* | 2.68 |

**Test Example 3: Degradation Effect of Compound on ERα**

In this test example, ERα DuoSet IC ELISA Kit (R&D, DYC5715) was used to detect the degradation effect of the compound on ERα. On the first day of the experiment, human breast cancer cells MCF-7 (the source was as above) were inoculated into a 24-well plate. The culture medium was an MEM culture medium containing 10% of activated-carbon-treated FBS (Tocyto, UT61204), 1% of pyruvic acid and 1% of nonessential amino acid. The inoculation density was 150,000 cells/well. The cells were cultured at 37°C, 5% CO2 for 24 h.

On the second day of the experiment, the compound to be tested was diluted in gradient and added to a 24-well plate containing cells. The cells were cultured for 24 h at 37°C, 5% CO2. ERα capture antibody was diluted to 1 μg/ml with PBS (Corning, 21-040-CVR) and added to a 96-well plate at 100 μL/well, and the plate was blocked and coated overnight at room temperature.

On the third day of the experiment, the coated 96-well plate was washed three times with PBS, 300 μL of a blocking solution (R&D, DY995) was added to each well, and the plate was blocked at room temperature for 1 h. The 24-well plate was washed once with PBS, and the residual liquid was sucked off. 60 μL of a lysis solution (6 M urea (Sigma, U5378), 1 mM EDTA (Sigma, E9884), 0.5% TritonX-100 (Sigma, T8787), 1 mM PMSF (Sigma, 93482), and protease inhibitor mix (Sigma, 05892970001)) was added to each well. After lysis on ice for 15 min, a diluent (1 mM EDTA, 0.5% TritonX-100 dissolved in PBS) was added. The diluted cell lysate was taken and added to the blocked 96-well plate, with 100 μL per well, and incubated at room temperature for 2 h. The plate was washed three times with a washing solution (R&D, WA126), and a primary antibody was then added. After incubation for 1 h, the 96-well plate was washed four times, a secondary antibody was added, and incubation was carried out at room temperature in the dark for 30 min. The plate was washed four times with the washing solution, and TMB (R&D, DY999) was added. After 15 minutes of development, the reaction was terminated with a stop solution (R&D, DY994), and the light absorption at 450 nm was read.

**$IC_{50}$ value of ERα degradation by compound**

| Compound | $IC_{50}$ (nM) |
|---|---|
| Example 1 | 0.62 |
| Example 9 | 0.39 |
| Example 10 | 0.41 |
| Example 11 | 0.62 |
| Example 12 | 0.57 |
| Example 13 | 0.23 |
| Example 25 | 0.34 |
| Example 30 | 0.35 |
| Example 31 | 0.75 |
| Example 32 | 0.42 |
| Example 33 | 0.35 |
| Example 34 | 0.24 |
| Example 43 | 0.63 |
| AZD9496 | 1.38 |
| AZD9833 | 1.26 |
| Tamoxifen | >625 |
| Comparative Compound 1 | 1.89 |

**Test Example 4: Test for stability in human liver microsomes *in vitro***

Eight 96-well incubation plates were prepared and named T0, T5, T15, T30, T45, T60, Blank60 and NCF60, respectively. The reaction time points for the first six incubation plates were 0, 5, 15, 30, 45 and 60 min, respectively. No test compound or control compound was added to Blank60 plate, and samples were taken after incubation for 60 min. NCF60 plate was incubated with a potassium phosphate buffer instead of an NADPH regeneration system solution (Sigma, N0505) for 60 min.

To T0, T5, T15, T30, T45, T60 and NCF60 plates, respectively, 2 μL of a working solution of the compound to be tested or a control working solution and 100 μL of a human liver microsome working solution (Corning, 452117) were added, wherein the concentration of the microsome protein was 1 mg/mL. Only the microsome working solution was added to Blank60 plate, and the incubation plates Blank60, T5, T15, T30, T45 and T60 except T0 and NCF60 were then placed in a water bath at 37°C and pre-incubated for about 10 min.

To the samples of T0 plate, 600 μL of a stop solution (acetonitrile : methanol (95 : 5, v/v) solution containing 100 ng/mL of tolbutamide (Sigma, T0891)) was added first, and the NADPH regeneration system working solution was then added.

98 μL of a potassium phosphate buffer was added to each well of NCF60 plate and incubated for 60 min.

After the pre-incubation in the incubation plates Blank60, T5, T15, T30, T45 and T60 was completed, 98 μL of the NADPH regeneration system working solution was added to each sample well to initiate the reaction.

After an appropriate incubation time (such as 5, 15, 30, 45 and 60 min), 600 μL of a stop solution was added to each sample well and the control sample well in Blank60, T5, T15, T30, T45, T60 and NCF60 plates, respectively, to stop the reaction.

All the sample plates were shaken uniformly and centrifuged at 3220×g for 20 min. 200 μL of test sample supernatant was separately taken and diluted in 200 μL of an aqueous solution containing 0.3% formic acid for LC-MS/MS analysis, and 100 μL of control sample supernatant was taken and diluted in 300 μL of pure water for LC-MS/MS analysis. Calculation was performed according to the following formula:

$$C_t = C_0 \bullet e^{-k_e \bullet t}$$

when

$$C_t = \frac{1}{2} C_0,$$

$$T_{1/2} = \frac{Ln2}{k_e} = \frac{0.693}{k_e}$$

$$CL_{int(mic)} = \frac{0.693}{\text{In vitro } T_{1/2}} \bullet \frac{1}{\text{mg/microsomal protein in a reaction system}}$$

$$CL_{int(liver)} = CL_{int(mic)} \bullet \frac{\text{mg microsome}}{\text{g liver}} \bullet \frac{\text{g liver}}{\text{kg body weight}}$$

**Experimental data of stability in microsomes *in vitro***

| Test sample | $T_{1/2}$ (min) | $CL_{int(mic)}$ (μL/min/mg) | $CL_{int(liver)}$ (mL/min/kg) |
|---|---|---|---|
| Example 1 | 91.8 | 15.1 | 13.6 |
| Example 5 | 75.5 | 18.4 | 16.5 |
| Example 8 | 85.9 | 16.1 | 14.5 |
| Example 10 | 116.5 | 9.0 | 8.1 |
| Example 12 | 103.7 | 9.8 | 8.9 |
| Example 14 | 109.0 | 9.5 | 8.7 |
| Example 25 | 105.7 | 9.6 | 8.4 |
| Example 30 | 94.6 | 15.8 | 14.4 |
| Example 31 | 83.4 | 17.5 | 18.4 |
| Example 32 | 102.6 | 9.8 | 9.0 |
| Example 33 | 111.2 | 8.3 | 7.9 |
| Example 34 | 106.9 | 10.1 | 8.9 |
| AZD9833 | 80.0 | 17.3 | 15.6 |
| Comparative Compound 1 | 42.2 | 32.9 | 29.6 |

## Claims

**1.** A compound of Formula (I) or a stereoisomer or pharmaceutically acceptable salt thereof:

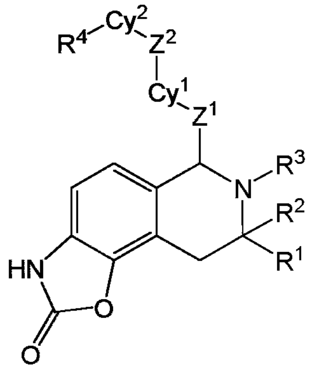

(I)

wherein:

$Z^1$ is a bond;
$Z^2$ is selected from $-O-CH_2-CH_2-$, $-O-CH_2-$, $-NH-CH_2-CH_2-$, $-NH-CH_2-$, -NH-, or -O-;
$Cy^1$ is phenyl or pyridinyl, each optionally substituted with a halogen atom or $C_{1-6}$alkoxy;
$Cy^2$ is selected from a bond, azetidinylidene, pyrrolidinylidene, piperazinylidene,

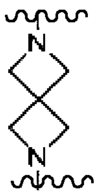

, and

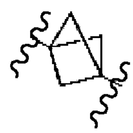

;

$R^1$ is $C_{1-6}$alkyl;
$R^2$ is H;
$R^3$ is $-CH_2-CR^{31}R^{32}R^{33}$;
$R^{31}$, $R^{32}$ and $R^{33}$ are independently selected from H, a halogen atom, cyano, and $C_{1-6}$alkyl optionally substituted with hydroxyl or a halogen atom, and $R^{31}$ and $R^{32}$ can jointly form $C_{3-8}$cycloalkylene optionally substituted with hydroxyl or a halogen atom;
$R^4$ is $C_{1-6}$alkyl, $C_{1-6}$alkylamino, amino$C_{1-6}$alkyl, $C_{2-6}$alkenyl, or $C_{2-6}$alkenylamino, optionally substituted with a group selected from a halogen atom, oxo, $C_{1-6}$alkylamino, $(C_{1-6}alkyl)_2$amino, and

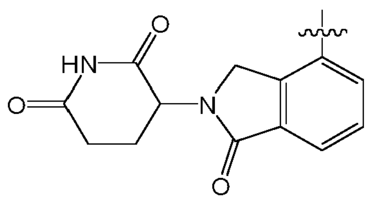

.

2. The compound or the stereoisomer or pharmaceutically acceptable salt thereof according to claim 1, wherein

$Cy^1$ is phenyl or pyridinyl, each optionally substituted with F or methoxy;
$R^1$ is methyl;
$R^{31}$, $R^{32}$ and $R^{33}$ are independently selected from H, F, cyano, and methyl optionally substituted with hydroxyl or F, and $R^{31}$ and $R^{32}$ can jointly form cyclopropylidene optionally substituted with hydroxyl or F;
$R^4$ is $C_{1-6}$alkyl, $C_{1-6}$alkylamino, amino$C_{1-6}$alkyl, $C_{2-6}$alkenyl, or $C_{2-6}$alkenylamino, optionally substituted with a group selected from F, oxo, methylamino, dimethylamino, and

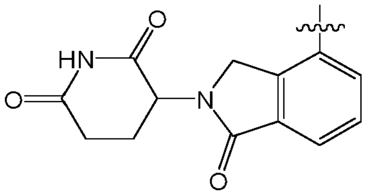.

3. The compound or the stereoisomer or pharmaceutically acceptable salt thereof according to claim 1, wherein

$Cy^2$ is selected from a bond, azetidinylidene, and pyrrolidinylidene, and
$R^4$ is $C_{1-6}$alkyl, $C_{1-6}$alkylamino, $C_{2-6}$alkenyl, or $C_{2-6}$alkenylamino, optionally substituted with a group selected from a halogen atom, oxo, $C_{1-6}$alkylamino, and $(C_{1-6}alkyl)_2$amino.

4. The compound or the stereoisomer or pharmaceutically acceptable salt thereof according to claim 3, wherein $R^4$ is $C_{1-6}$alkyl, $C_{1-6}$alkylamino, $C_{2-6}$alkenyl, or $C_{2-6}$alkenylamino, optionally substituted with a group selected from F, oxo, methylamino, and dimethylamino.

5. The compound or the stereoisomer or pharmaceutically acceptable salt thereof according to claim 1, wherein

$Z^1$ is a bond;
$Z^2$ is selected from -NH- or -O-;
$Cy^1$ is phenyl or pyridinyl, optionally substituted with F or methoxy;
$Cy^2$ is selected from azetidinylidene, pyrrolidinylidene, and piperazinylidene;
$R^1$ is methyl;
$R^2$ is H;
$R^3$ is $-CH_2-CR^{31}R^{32}R^{33}$;
$R^{31}$ and $R^{32}$ are each F, or $R^{31}$ and $R^{32}$ jointly form cyclopropylidene;
$R^{33}$ is selected from F, hydroxymethyl, and fluoromethyl;
$R^4$ is $F-(CH_2)_3-$ or

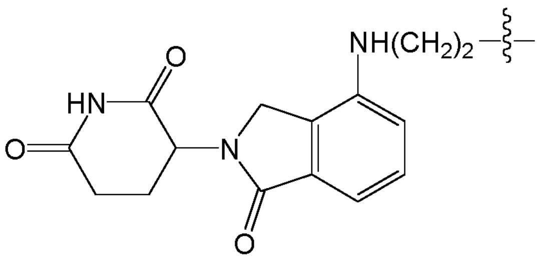.

6. The compound or the stereoisomer or pharmaceutically acceptable salt thereof according to claim 5, wherein $Cy^1$ is phenyl optionally substituted with F or methoxy.

7. The compound or the stereoisomer or pharmaceutically acceptable salt thereof according to claim 5, wherein $Cy^2$ is selected from azetidinylidene and pyrrolidinylidene, and $R^4$ is $F-(CH_2)_3-$.

8. A compound or a stereoisomer or pharmaceutically acceptable salt thereof, wherein the compound is selected from the following compounds:

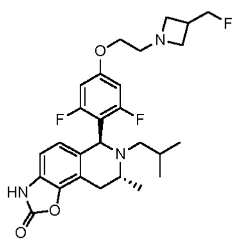 1, 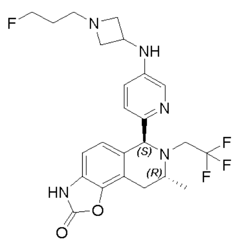 5, 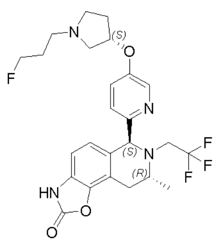 7, 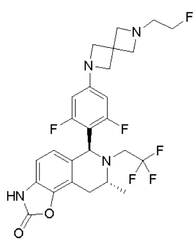 8,

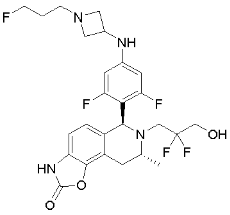 9, 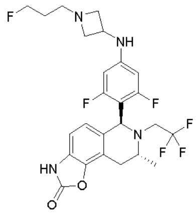 10, 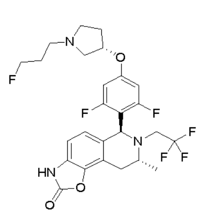 11, 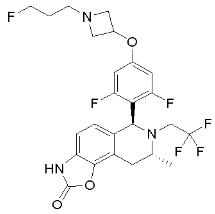 12,

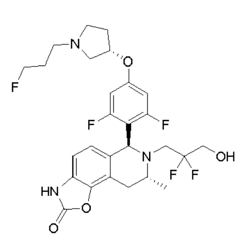 13, 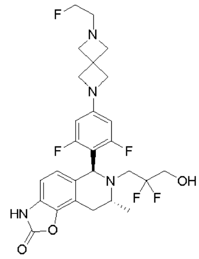 14, 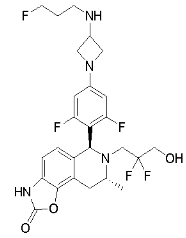 15, 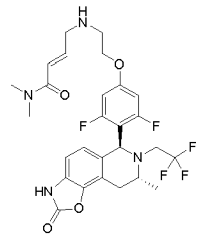 16,

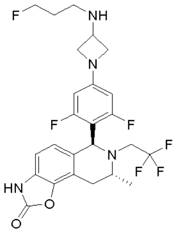 18, 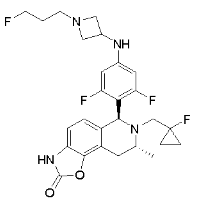 20, 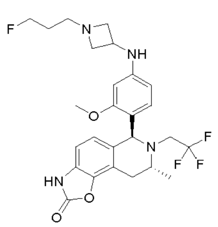 25, 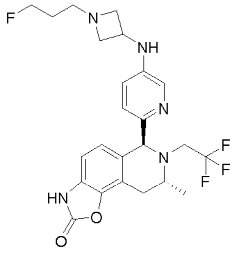 26,

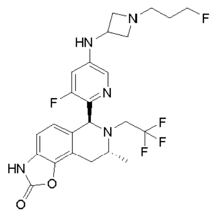 27, 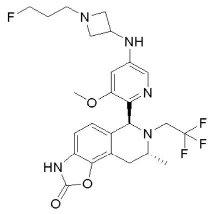 28, 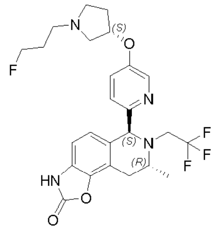 29, 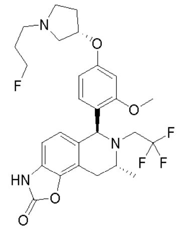 30,

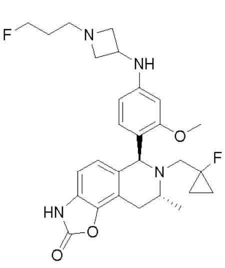 31, 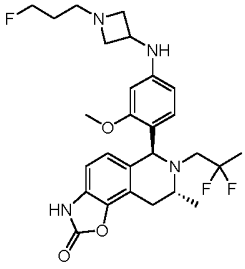 32, 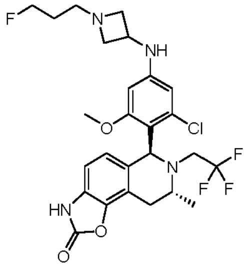 33, 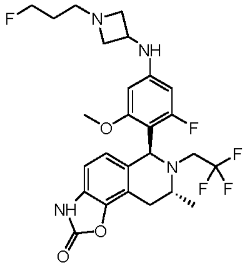 34,

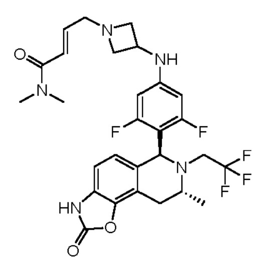 35, 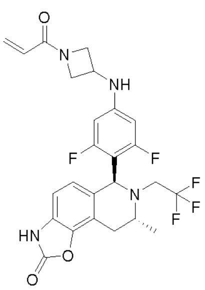 36, 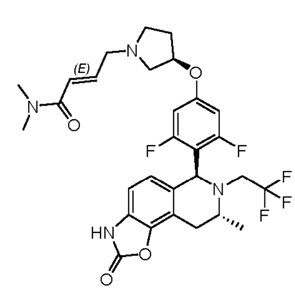 37, 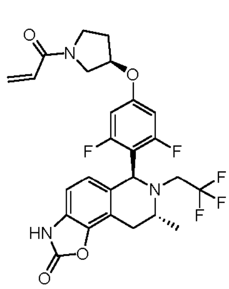 38,

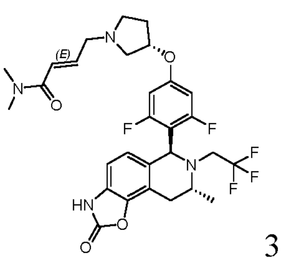 39, 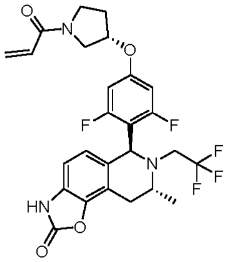 40, 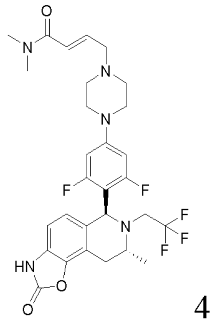 41, 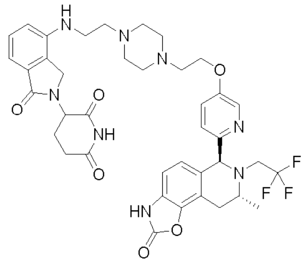 42

and

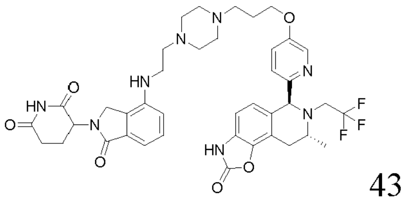 43.

9. A pharmaceutical composition comprising the compound or the stereoisomer or pharmaceutically acceptable salt thereof according to any one of claims 1-8, and a pharmaceutically acceptable carrier.

10. A compound or a stereoisomer or pharmaceutically acceptable salt thereof according to any one of claims 1-8 for use in treating an estrogen receptor-dependent or -mediated disease in a mammal.

## Patentansprüche

1. Verbindung der Formel (I) oder ein Stereoisomer oder ein pharmazeutisch annehmbares Salz davon:

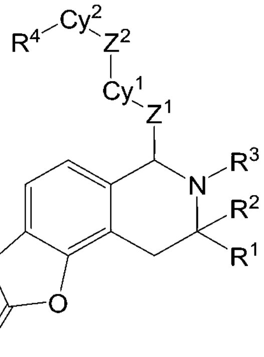 (I)

wobei:

$Z^1$ eine Bindung ist;
$Z^2$ aus $-O-CH_2-CH_2-$, $-O-CH_2-$, $-NH-CH_2-CH_2-$, $-NH-CH_2-$, -NH- oder -O-ausgewählt ist;
$Cy^1$ Phenyl oder Pyridinyl ist, jedes optional substituiert mit einem Halogenatom oder $C_{1-6}$Alkoxy;
$Cy^2$ aus einer Bindung, Azetidinyliden, Pyrrolidinyliden, Piperazinyliden,

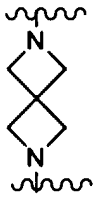 und 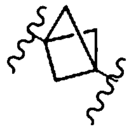

ausgewählt ist;
$R^1$ $C_{1-6}$Alkyl ist;
$R^2$ H ist;
$R^3$ $-CH_2-CR^{31}R^{32}R^{33}$ ist;
$R^{31}$, $R^{32}$ und $R^{33}$ unabhängig ausgewählt sind aus H, einem Halogenatom, Cyano und $C_{1-6}$Alkyl, optional substituiert mit Hydroxy oder einem Halogenatom, und $R^{31}$ und $R^{32}$ gemeinsam ein $C_{3-8}$Cycloalkylen bilden können, optional substituiert mit Hydroxy oder einem Halogenatom;
$R^4$ $C_{1-6}$Alkyl, $C_{1-6}$Alkylamino, Amino$C_{1-6}$alkyl, $C_{2-6}$Alkenyl oder $C_{2-6}$Alkenylamino ist, optional substituiert mit einer Gruppe ausgewählt aus einem Halogenatom, Oxo, $C_{1-6}$Alkylamino, $(C_{1-6}Alkyl)_2$amino und

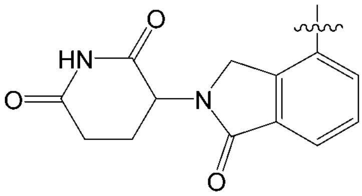 .

**2.** Verbindung oder Stereoisomer oder pharmazeutisch annehmbares Salz davon nach Anspruch 1, wobei

$Cy^1$ Phenyl oder Pyridinyl ist, jedes optional substituiert mit F oder Methoxy;
$R^1$ Methyl ist;
$R^{31}$, $R^{32}$ und $R^{33}$ unabhängig aus H, F, Cyano und Methyl, optional substituiert mit Hydroxyl oder F, ausgewählt sind, und $R^{31}$ and $R^{32}$ gemeinsam Cyclopropyliden bilden können, optional substituiert mit Hydroxy oder F;
$R^4$ $C_{1-6}$Alkyl, $C_{1-6}$Alkylamino, Amino$C_{1-6}$alkyl, $C_{2-6}$Alkenyl oder $C_{2-6}$Alkenylamino ist, optional substituiert mit einer Gruppe ausgewählt aus F, Oxo, Methylamino, Dimethylamino und

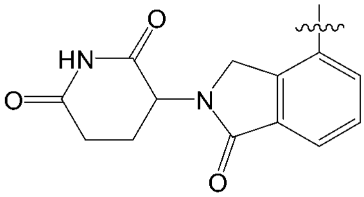 .

**3.** Verbindung oder Stereoisomer oder pharmazeutisch annehmbares Salz davon nach Anspruch 1, wobei

$Cy^2$ aus einer Bindung, Azetidinyliden und Pyrrolidinyliden ausgewählt ist, und
$R^4$ $C_{1-6}$Alkyl, $C_{1-6}$Alkylamino, $C_{2-6}$Alkenyl oder $C_{2-6}$Alkenylamino ist, optional substituiert mit einer Gruppe ausgewählt aus einem Halogenatom, Oxo, $C_{1-6}$Alkylamino und $(C_{1-6}Alkyl)_2$amino.

**4.** Verbindung oder Stereoisomer oder pharmazeutisch annehmbares Salz davon nach Anspruch 3, wobei $R^4$ $C_{1-6}$Alkyl, $C_{1-6}$Alkylamino, $C_{2-6}$Alkenyl oder $C_{2-6}$Alkenylamino ist, optional substituiert mit einer Gruppe ausgewählt aus F, Oxo, Methylamino und Dimethylamino.

**5.** Verbindung oder Stereoisomer oder pharmazeutisch annehmbares Salz davon nach Anspruch 1, wobei

$Z^1$ eine Bindung ist;
$Z^2$ aus -NH- oder -O- ausgewählt ist;
$Cy^1$ Phenyl oder Pyridinyl ist, optional substituiert mit F oder Methoxy;
$Cy^2$ aus Azetidinyliden, Pyrrolidinyliden und Piperazinyliden ausgewählt ist;

$R^1$ Methyl ist;
$R^2$ H ist;
$R^3$ $-CH_2-CR^{31}R^{32}R^{33}$ ist;
$R^{31}$ und $R^{32}$ jedes F ist, oder $R^{31}$ und $R^{32}$ gemeinsam Cyclopropyliden bilden; $R^{33}$ aus F, Hydroxymethyl und Fluoromethyl ausgewählt ist;
$R^4$ $F-(CH_2)_3-$ oder

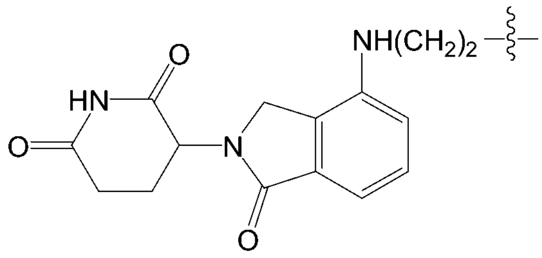

ist.

6. Verbindung oder Stereoisomer oder pharmazeutisch annehmbares Salz davon nach Anspruch 5, wobei $Cy^1$ Phenyl ist, optional substitutiert mit F oder Methoxy.

7. Verbindung oder Stereoisomer oder pharmazeutisch annehmbares Salz davon nach Anspruch 5, wobei $Cy^2$ aus Azetidinyliden und Pyrrolidinyliden ausgewählt ist, und $R^4$ $F-(CH_2)_3-$ ist.

8. Verbindung oder ein Stereoisomer oder ein pharmazeutisch annehmbares Salz davon, wobei die Verbindung aus den folgenden Verbindungen ausgewählt ist:

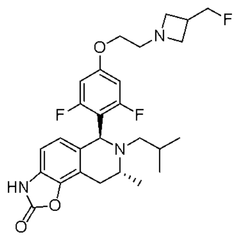 1, 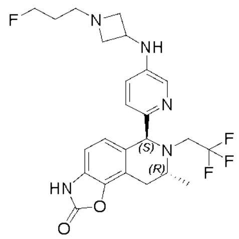 5, 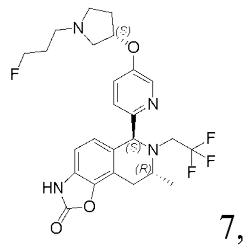 7, 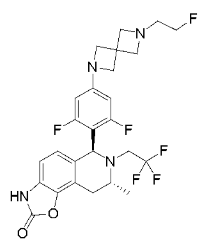 8,

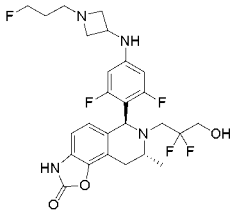 9, 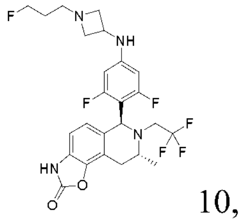 10, 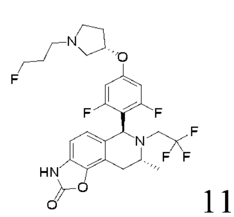 11, 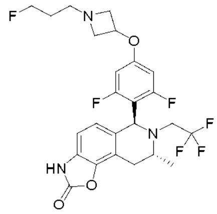 12,

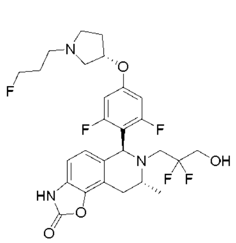 13, 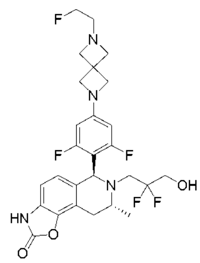 14, 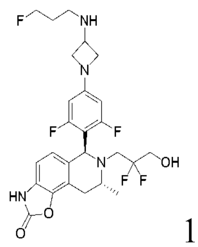 15, 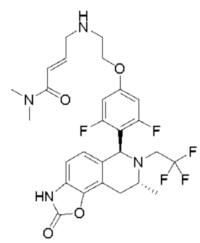 16,

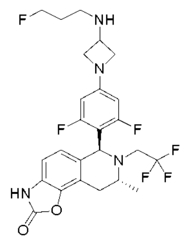 18, 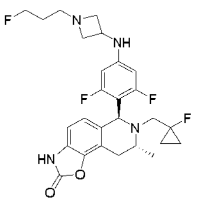 20, 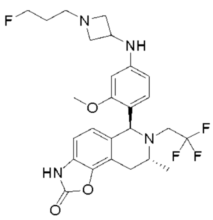 25, 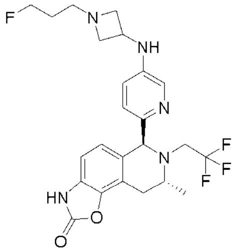 26,

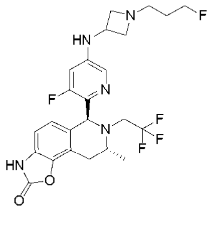 27, 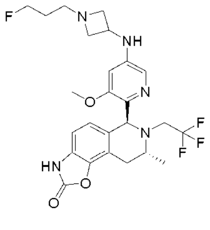 28, 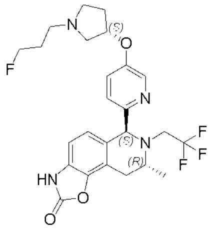 29, 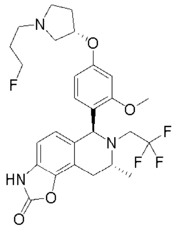 30,

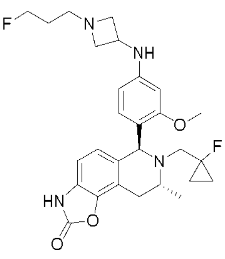 31, 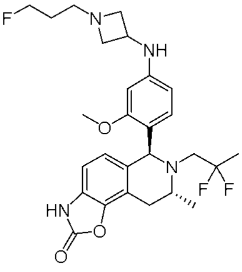 32, 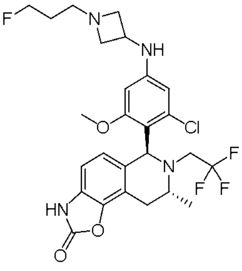 33, 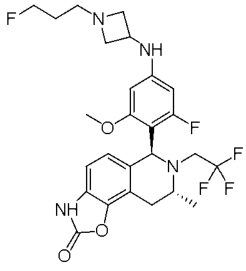 34,

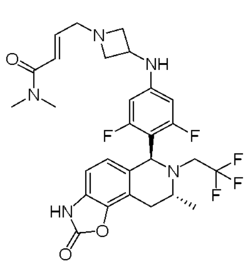 35, 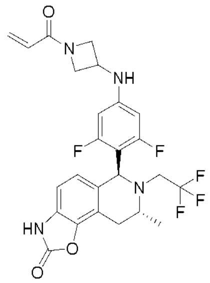 36, 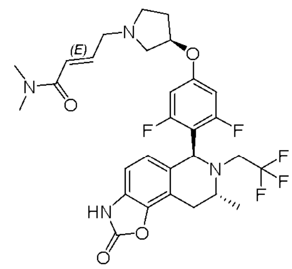 37, 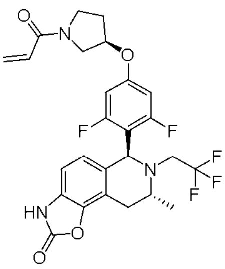 38,

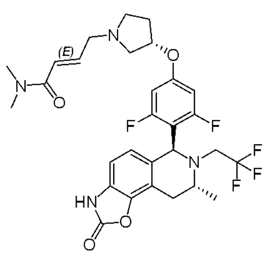 39, 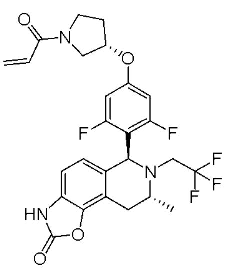 40, 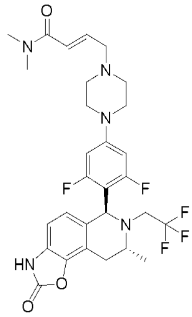 41, 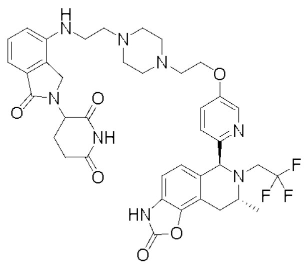 42

und

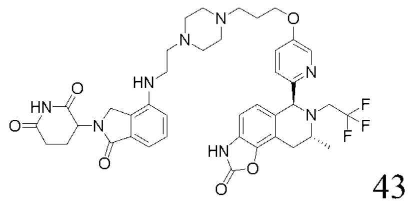

43.

**9.** Pharmazeutische Zusammensetzung umfassend die Verbindung oder das Stereoisomer oder ein pharmazeutisch annehmbares Salz davon nach einem der Ansprüch 1 bis 8 sowie einen pharmazeutisch annehmbaren Träger.

**10.** Verbindung oder ein Stereoisomer oder ein pharmazeutisch annehmbares Salz davon nach einem der Ansprüch 1 bis 8 zur Verwendung bei der Behandlung einer Östrogenrezeptor-abhängigen oder -vermittelten Erkrankung bei einem Säugetier.

**Revendications**

**1.** Composé de formule (I) ou stéréoisomère ou sel pharmaceutiquement acceptable de celui-ci :

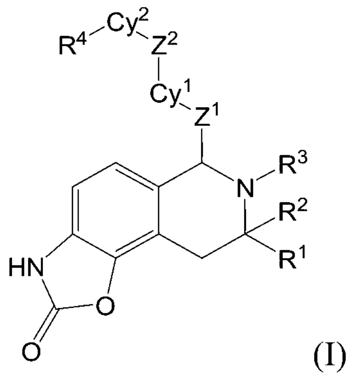

(I)

dans laquelle :

$Z^1$ est une liaison ;
$Z^2$ est choisi parmi $-O-CH_2-CH_2-$, $-O-CH_2-$, $-NH-CH_2-CH_2-$, $-NH-CH_2-$, -NH- ou - O- ;
$Cy^1$ est un groupe phényle ou pyridinyle, chacun étant éventuellement substitué par un atome d'halogène ou un groupe alcoxy en $C_{1-6}$ ;
$Cy^2$ est choisi parmi une liaison, un groupe azétidinylidène, pyrrolidinylidène, pipérazinylidène,

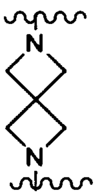

, et

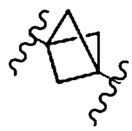

;

$R^1$ est un groupe alkyle en $C_{1-6}$ ;
$R^2$ est H ;
$R^3$ est $-CH_2-CR^{31}R^{32}R^{33}$ ;
$R^{31}$, $R^{32}$ et $R^{33}$ sont choisis indépendamment parmi H, un atome d'halogène, un groupe cyano et un groupe alkyle en $C_{1-6}$ éventuellement substitué par un groupe hydroxyle ou un atome d'halogène, et $R^{31}$ et $R^{32}$ peuvent former conjointement un groupe cycloalkylène en $C_{3-8}$ éventuellement substitué par un groupe hydroxyle ou un atome d'halogène ;
$R^4$ est un groupe alkyle en $C_{1-6}$, alkylamino en $C_{1-6}$, aminoalkyle en $C_{1-6}$, alcényle en $C_{2-6}$ ou alcénylamino en $C_{2-6}$, éventuellement substitué par un groupe choisi parmi un atome d'halogène, un groupe oxo, alkylamino en

$C_{1-6}$, (alkyle en $C_{1-6}$)$_2$amino, et

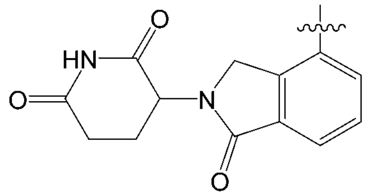

.

2. Composé, stéréoisomère ou sel pharmaceutiquement acceptable de celui-ci selon la revendication 1, dans lequel

$Cy^1$ est un groupe phényle ou pyridinyle, chacun étant éventuellement substitué par F ou un groupe méthoxy ;
$R^1$ est un groupe méthyle ;
$R^{31}$, $R^{32}$ et $R^{33}$ sont choisis indépendamment parmi H, F, un groupe cyano et un groupe méthyle éventuellement substitué par un groupe hydroxyle ou F, et $R^{31}$ et $R^{32}$ peuvent former ensemble un groupe cyclopropylidène éventuellement substitué par un groupe hydroxyle ou F ;
$R^4$ est un groupe alkyle en $C_{1-6}$, alkylamino en $C_{1-6}$, aminoalkyle en $C_{1-6}$, alcényle en $C_{2-6}$ ou alcénylamino en $C_{2-6}$, éventuellement substitué par un groupe choisi parmi F, oxo, méthylamino, diméthylamino et

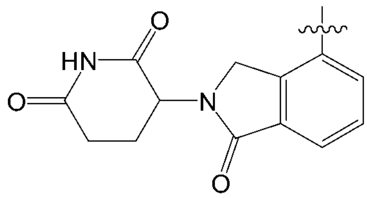

.

3. Composé, stéréoisomère ou sel pharmaceutiquement acceptable de celui-ci selon la revendication 1, dans lequel

$Cy^2$ est choisi parmi une liaison, un groupe azétidinylidène et un groupe pyrrolidinylidène, et
$R^4$ est un groupe alkyle en $C_{1-6}$, alkylamino en $C_{1-6}$, alcényle en $C_{2-6}$ ou alcénylamino en $C_{2-6}$, éventuellement substitué par un groupe choisi parmi un atome d'halogène, un groupe oxo, un groupe alkylamino en $C_{1-6}$ et un groupe (alkyle en $C_{1-6}$)$_2$amino.

4. Composé, stéréoisomère ou sel pharmaceutiquement acceptable de celui-ci selon la revendication 3, dans lequel $R^4$ est un groupe alkyle en $C_{1-6}$, alkylamino en $C_{1-6}$, alcényle en $C_{2-6}$ ou alcénylamino en $C_{2-6}$, éventuellement substitué par un groupe choisi parmi F, un groupe oxo, méthylamino et diméthylamino.

5. Composé, stéréoisomère ou sel pharmaceutiquement acceptable de celui-ci selon la revendication 1, dans lequel

$Z^1$ est une liaison ;
$Z^2$ est choisi parmi -NH- ou -O- ;
$Cy^1$ est un groupe phényle ou pyridinyle, éventuellement substitué par F ou un groupe méthoxy ;
$Cy^2$ est choisi parmi les groupes azétidinylidène, pyrrolidinylidène et pipérazinylidène ;
$R^1$ est un groupe méthyle ;
$R^2$ est H ;
$R^3$ est $-CH_2-CR^{31}R^{32}R^{33}$ ;
$R^{31}$ et $R^{32}$ sont chacun F, ou $R^{31}$ et $R^{32}$ forment ensemble un groupe cyclopropylidène ;
$R^{33}$ est choisi parmi F, un groupe hydroxyméthyle et un groupe fluorométhyle ;
$R^4$ est $F-(CH_2)_3-$ ou

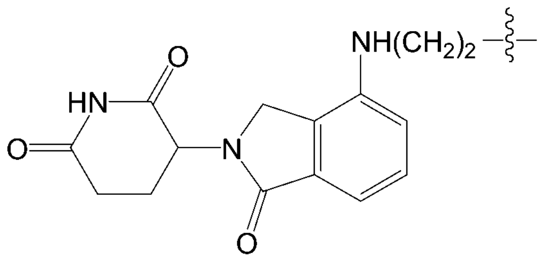
.

6. Composé, stéréoisomère ou sel pharmaceutiquement acceptable de celui-ci selon la revendication 5, dans lequel $Cy^1$ est un groupe phényle éventuellement substitué par F ou un groupe méthoxy.

7. Composé, stéréoisomère ou sel pharmaceutiquement acceptable de celui-ci selon la revendication 5, dans lequel $Cy^2$ est choisi parmi les groupes azétidinylidène et pyrrolidinylidène, et $R^4$ est $F-(CH_2)_3-$.

8. Composé, stéréoisomère ou sel pharmaceutiquement acceptable de celui-ci, dans lequel le composé est choisi parmi les composés suivants :

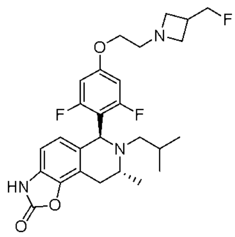
1,
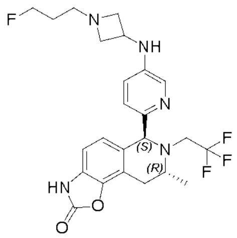
5,
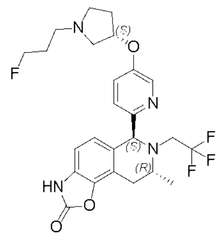
7,
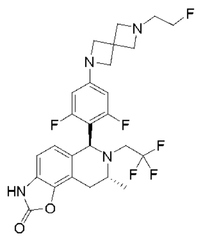
8,

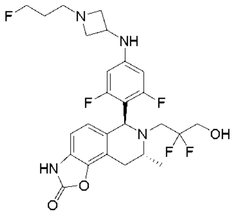
9,
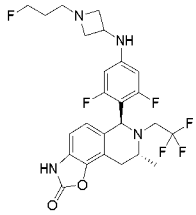
10,
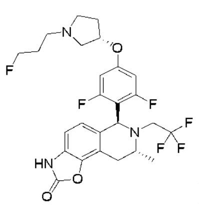
11,
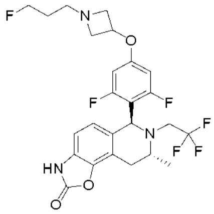
12,

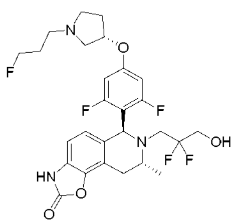
13,
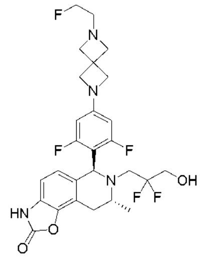
14,
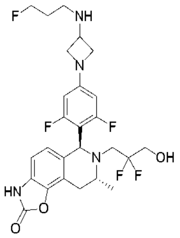
15,
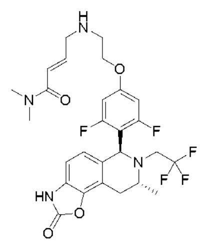
16,

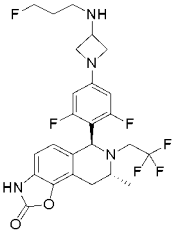
18,
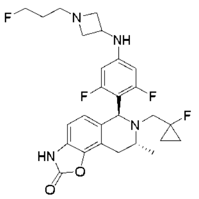
20,
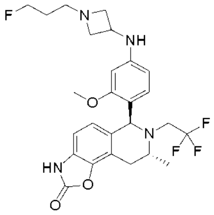
25,
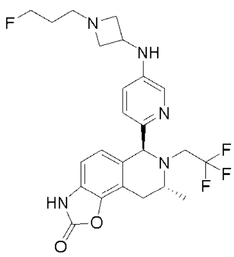
26,

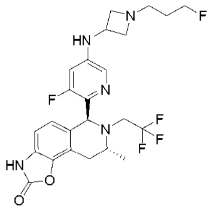 27, 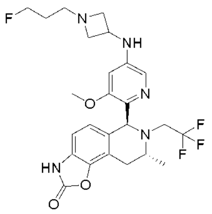 28, 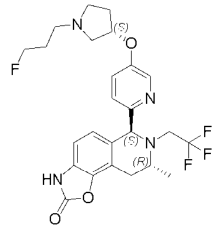 29, 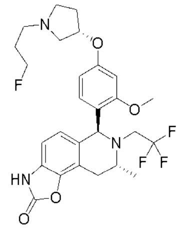 30,

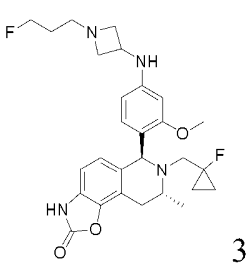 31, 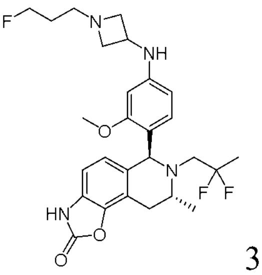 32, 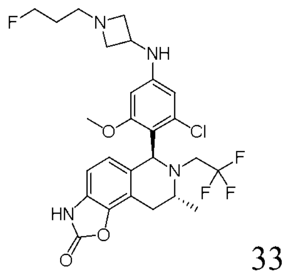 33, 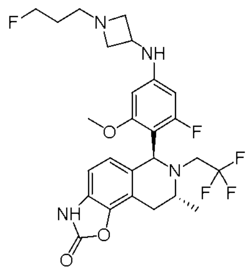 34,

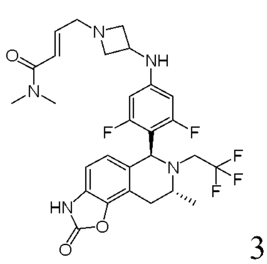 35, 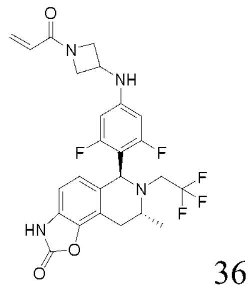 36, 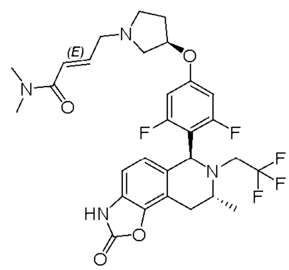 37, 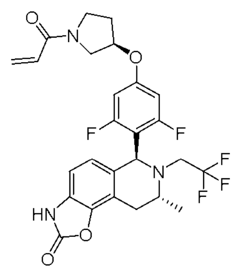 38,

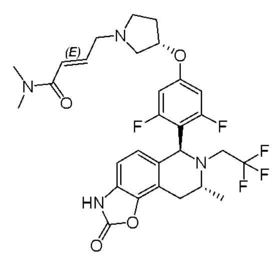 39, 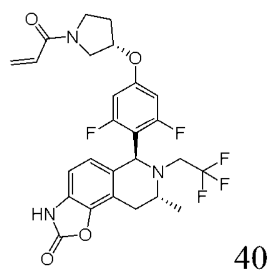 40, 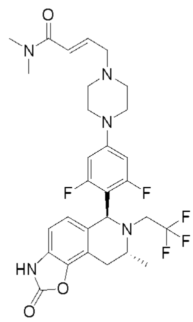 41, 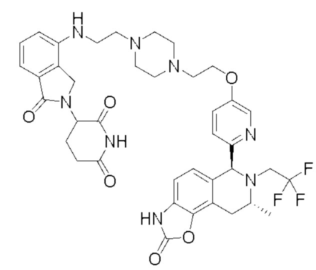 42

et

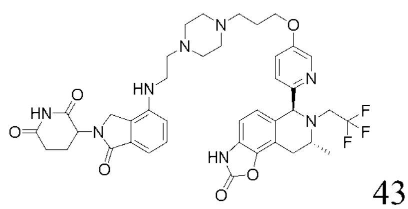 43.

9. Composition pharmaceutique comprenant le composé, stéréoisomère ou sel pharmaceutiquement acceptable de celui-ci selon l'une quelconque des revendications 1 à 8, et un excipient pharmaceutiquement acceptable.

10. Composé, stéréoisomère ou sel pharmaceutiquement acceptable de celui-ci selon l'une quelconque des revendications 1 à 8, destiné à être utilisé dans le traitement d'une maladie dépendante des récepteurs d'œstrogènes ou médiée par ceux-ci chez un mammifère.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017182493 A1
- WO 2017174757 A1
- EP 3312184 A1
- WO 2019192533 A1
- WO 2019223715 A1